(19) Europäisches Patentamt
European Patent Office
Office européen des brevets

(11) Veröffentlichungsnummer: **0 323 487 B1**

(12) **EUROPÄISCHE PATENTSCHRIFT**

(45) Veröffentlichungstag der Patentschrift: **31.03.93**

(51) Int. Cl.[5]: **C07D 239/54**, A01N 43/54

(21) Anmeldenummer: **88904904.5**

(22) Anmeldetag: **16.06.88**

(86) Internationale Anmeldenummer:
**PCT/CH88/00107**

(87) Internationale Veröffentlichungsnummer:
**WO 88/10254 (29.12.88 88/28)**

Die Akte enthält technische Angaben, die nach dem Eingang der Anmeldung eingereicht wurden und die nicht in dieser Patentschrift enthalten sind.

# (54) HETEROCYCLISCHE VERBINDUNGEN.

(30) Priorität: **19.06.87 CH 2320/87**
**17.09.87 CH 3580/87**

(43) Veröffentlichungstag der Anmeldung:
**12.07.89 Patentblatt 89/28**

(45) Bekanntmachung des Hinweises auf die Patenterteilung:
**31.03.93 Patentblatt 93/13**

(84) Benannte Vertragsstaaten:
**AT BE CH DE FR GB IT LI LU NL**

(56) Entgegenhaltungen:
**EP-A- 0 195 346**

(73) Patentinhaber: **CIBA-GEIGY AG**
**Klybeckstrasse 141**
**CH-4002 Basel(CH)**

(72) Erfinder: **WENGER, Jean**
**Brunnenwiesenstrasse 1**
**CH-8610 Uster(CH)**
Erfinder: **WINTERNITZ, Paul**
**Am Pfisterhölzli 50**
**CH-8606 Greifensee(CH)**
Erfinder: **ZELLER, Martin**
**Zwinggartenstrasse 51**
**CH-8600 Dübendorf(CH)**

EP 0 323 487 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft heterocyclische Verbindungen, und zwar 3-Aryluracile der allgemeinen Formel

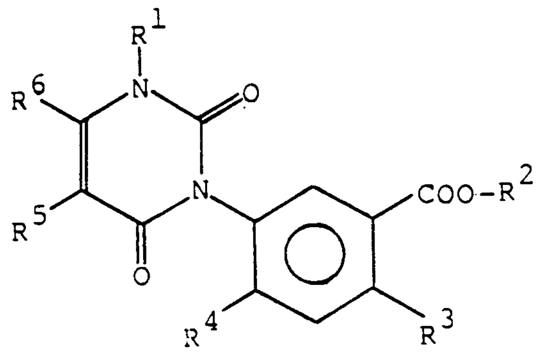

I

worin

$R^1$ Wasserstoff, $C_{1-4}$-Alkyl, $C_{3\ \text{oder}\ 4}$-Alkenyl, $C_{3\ \text{oder}\ 4}$-Alkinyl oder $C_{1-4}$-Halogenalkyl,

$R^2$ eine Gruppe

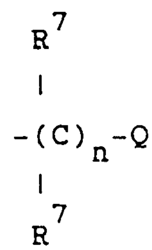

oder, im Falle, dass $R^1$ Halogenalkyl bedeutet, auch Wasserstoff, $C_{1-8}$-Alkyl, $C_{2-8}$-Alkenyl, $C_{2-8}$-Alkinyl oder $C_{2-8}$-Alkoxyalkyl,

$R^3$ Halogen oder Cyano,

$R^4$ Wasserstoff oder Halogen,

$R^5$ Wasserstoff, Fluor oder $C_{1-4}$-Alkyl,

$R^6$ $C_{1-4}$-Alkyl oder $C_{1-4}$-Halogenalkyl,

die Symbole $R^7$ unabhängig voneinander Wasserstoff oder $C_{1-3}$-Alkyl und

n 0, 1 oder 2 bedeuten,

und

Q einen gegebenenfalls mit einem oder mehreren $C_{1-4}$-Alkylresten substituierten gesättigten drei- bis siebengliedrigen carbocyclischen oder heterocyclischen Rest bedeutet, wobei letzterer 1 oder 2 aus Sauerstoff und Schwefel ausgewählte Heteroatome und gegebenenfalls eine Ketofunktion im Ring aufweist,

oder

Q einen gegebenenfalls mit Halogen, $C_{1-4}$-Alkyl, $C_{1-4}$-Halogenalkyl, $C_{1-4}$-Alkoxy, $C_{1-4}$-Alkylthio, Nitro und/oder Cyano mono- oder mehrfach substituierten Phenylrest bedeutet, der zusätzlich einen ankondensierten, gesättigten, carbocyclischen oder heterocyclischen, fünf- bis siebengliedrigen Ring enthalten kann, wobei der Heterozyklus 1 oder 2 Sauerstoffatome im Ring aufweist,

und die entsprechenden Enoläther derjenigen Verbindungen der Formel I, in denen $R^1$ von Wasserstoff oder $C_{1-4}$Halogenalkyl verschieden ist, sowie Salze derjenigen Verbindungen der Formel I, in denen $R^1$ oder $R^2$ Wasserstoff bedeutet.

Unter den obenerwähnten Enoläthern sind also die Verbindungen der Formel

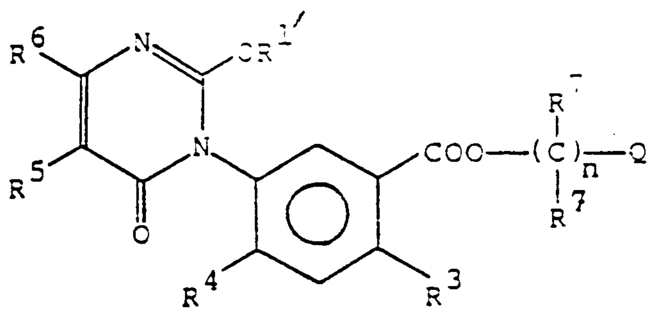

I-a

worin $R^3$, $R^4$, $R^5$, $R^6$, $R^7$, n und Q die oben angegebenen Bedeutungen besitzen und $R^{1'}$ $C_{1-4}$-Alkyl, $C_{3\ oder\ 4}$-Alkenyl oder $C_{3\ oder\ 4}$-Alkinyl bedeutet,
zu verstehen.

3-Aryluracile mit herbizider Wirkung sind aus EP-A-0 195 346 bekannt.

Die erfindungsgemässen Verbindungen sind herbizidwirksam und eignen sich als Wirkstoffe von Unkrautbekämpfungsmitteln. Somit umfasst die Erfindung auch Unkrautbekämpfungsmittel, welche erfindungsgemässe Verbindungen als Wirkstoffe enthalten, Verfahren zur Herstellung diese Verbindungen sowie die Verwendung der Verbindungen bzw. Mittel zur Bekämpfung von Unkräutern.

In der obigen Formel I umfasst "Halogen" Fluor, Chlor, Brom und Jod. Die Alkyl-, Alkenyl- und Alkinylreste können geradkettig oder verzweigt sein, wobei dies auch für den Alkylteil der Halogenalkyl-, Alkoxy- und Alkylthiogruppen gilt. Eine Halogenalkylgruppe kann ein oder mehrere (gleiche oder verschiedene) Halogenatome aufweisen.

Beispiele der Reste Q sind folgende: Cyclopropyl, Cyclobutyl, Cyclopentyl, Cyclohexyl, Cycloheptyl, 2-Oxiranyl, 2-Methyl-2-oxiranyl, 3-Oxetanyl, 3-Methyl-3-oxetanyl, 3-Aethyl-3-oxetanyl, 2-Tetrahydrofuryl, 3-Tetrahydrofuryl, 2-Tetrahydropyranyl, 3-Tetrahydropyranyl, 4-Tetrahydropyranyl, 3-Methyl-3-thietanyl, 3-Tetrahydrothienyl, 1,3-Dioxolan-5-yl, 1,3-Dioxan-5-yl, 1-Oxo-4-tetrahydrothiopyranyl, Phenyl, 1,3-Benzodioxol-5-yl und 1,3-Benzodioxan-6-yl.

Bei den Salzen der Verbindungen der Formel I handelt es sich insbesondere um Alkalimetallsalze, z.B. Natrium- und Kaliumsalze; Erdalkalimetallsalze, z.B. Calcium- und Magnesiumsalze; Ammoniumsalze, d.h. unsubstituierte Ammoniumsalze und mono- oder mehrfach-substituierte Ammoniumsalze, z.B. Triäthylammonium- und Methylammoniumsalze, sowie um Salze mit anderen organischen Basen, z.B. mit Pyridin.

Das Vorhandensein mindestens eines asymmetrischen Kohlenstoffatoms in den Verbindungen der Formel I hat zur Folge, dass die Verbindungen in optisch isomeren Formen auftreten können. Durch das Vorliegen einer allfälligen aliphatischen C=C-Doppelbindung kann auch geometrische Isomerie auftreten. Die Formel I soll all diese möglichen isomeren Formen sowie Gemische davon umfassen.

Eine interessante Gruppe erfindungsgemässer Verbindungen besteht aus denjenigen Verbindungen der Formel I, in denen $R^1$ Wasserstoff, $C_{1-4}$-Alkyl, $C_{3\ oder\ 4}$-Alkenyl oder $C_{3\ oder\ 4}$-Alkinyl bedeutet, und den Enoläthern und Salzen dieser Verbindungen. Eine weitere interessante Gruppe erfindungsgemässer Verbindungen besteht aus denjenigen Verbindungen der Formel I, in denen $R^1$ $C_{1-4}$-Halogenalkyl, $R^2$ Wasserstoff, $C_{1-8}$-Alkyl, $C_{2-8}$-Alkenyl, $C_{2-8}$-Alkinyl, $C_{2-8}$-Alkoxyalkyl oder eine Gruppe $-(CR^7R^7)_n$-Q und Q entweder einen gegebenenfalls mit einem oder mehreren $C_{1-4}$-Alkylresten substituierten gesättigten drei- bis siebengliedrigen carbocyclischen oder heterocyclischen Rest, wobei letzterer 1 oder 2 aus Sauerstoff oder Schwefel ausgewählte Heteroatome im Ring ausweist, oder einen gegebenenfalls mit Halogen, $C_{1-4}$-Alkyl, $C_{1-4}$-Halogenalkyl, $C_{1-4}$-Alkoxy, $C_{1-4}$-Alkylthio, Nitro und/oder Cyano mono- oder mehrfach substituierten Phenylrest bedeuten, und den Salzen dieser Verbindungen.

Bedeutet $R^1$ $C_{3\ oder\ 4}$-Alkenyl oder $C_{3\ oder\ 4}$-Alkinyl, ist dieser Rest vorzugsweise Allyl bzw. Propargyl. Falls $R^6$ Halogenalkyl bedeutet, ist dies vorzugsweise Trifluormethyl oder Pentafluoräthyl. Im allgemeinen ist ein allfällig vorkommendes Halogenatom vorzugsweise Fluor, Chlor oder Brom.

Unabhängig voneinander bedeuten $R^1$ vorzugsweise geradkettiges $C_{1-4}$-Alkyl, insbesondere Methyl, oder $C_{1-4}$-Fluoralkyl, insbesondere Difluormethyl; $R^3$ vorzugsweise Chlor oder Brom; $R^4$ vorzugsweise Wasserstoff oder Fluor; $R^5$ vorzugsweise Wasserstoff, Fluor oder Methyl; $R^6$ vorzugsweise $C_{1-4}$-Alkyl, insbesondere Methyl; jedes $R^7$ vorzugsweise Wasserstoff; und n vorzugsweise 0 oder 1.

Besonders bevorzugte Verbindungen der Formel I sind die Cycloheptyl-, (Tetrahydrofuran-3-yl)-, (1,3-Dioxan-5-yl)-, Cyclopropylmethyl-, Tetrahydrofurfuryl-, (1,3-Dioxolan-4-yl)methyl-, Benzyl-, (4,4-Dimethyl-2-oxo-tetrahydrofuran-3-yl)-, Cyclopentyl-, (1-Cyclopropyläthyl)- und Cyclohexylester von 2-Chlor-5-[3,6-dihydro-2,6-dioxo-3-methyl-4-trifluormethyl -1(2H)-pyrimidinyl]-4-fluorbenzoesäure.

Weitere Vertreter von Verbindungen der Formel I sind die (3-Nitrobenzyl)-, (α,α-Dimethylbenzyl)-, (Tetrahydrothiophen-3-yl)-, (4-Cyanophenyl)- und [3,5-di(trifluormethyl)-benzyl]ester von 2-Chlor-5-[3,6-dihydro-2,6-dioxo-3-methyl-4-trifluormethyl -1(2H)-pyrimidinyl]-4-fluorbenzoesäure sowie die Cyclopropylmethyl- 1-Cyclopropyläthyl-, Cyclopentyl-, Cyclohexyl-, Cycloheptyl- (Tetrahydrofuran-3-yl)-, (1,3-Dioxan-5-yl)-, (1,3-Dioxolan-4-yl)methyl-, Benzyl- und Tetrahydrofurfurylester von 2-Chlor-5-[3,6-dihydro-3,4-dimethyl-2,6-dioxo-1(2H)-pyrimidinyl]-4-fluorbenzoesäure.

Besonders bevorzugte Verbindungen der Formel I, in denen $R^1$ $C_{1-4}$-Halogenalkyl bedeutet, sind:

2-Chlor-5-[3-difluormethyl-3,6-dihydro-4-methyl-2,6-dioxo-1(2H)-pyrimidinyl]-4-fluorbenzoesäure-isopropylester,

2-Chlor-5-[3-difluormethyl-3,6-dihydro-4-methyl-2,6-dioxo-1(2H)-pyrimidinyl]-4-fluorbenzoesäure,

5-[4-Aethyl-3-difluormethyl-3,6-dihydro-2,6-dioxo-1(2H)-pyrimidinyl]-2-chlor-4-fluorbenzoesäure-isopropylester,

5-[4-Aethyl-3-difluormethyl-3,6-dihydro-2,6-dioxo-1(2H)-pyrimidinyl]-2-brom-4-fluorbenzoesäure-isopropylester,

2-Brom-5-[3-difluormethyl-3,6-dihydro-4-methyl-2,6-dioxo-1(2H)-pyrimidinyl]-4-fluorbenzoesäure-isopropylester,

2-Chlor-5-[3-difluormethyl-3,6-dihydro-4-methyl-2,6-dioxo-1(2H)-pyrimidinyl]-4-fluorbenzoesäure-2-propinylester und

2-Chlor-5-[3-difluormethyl-3,6-dihydro-4-methyl-2,6-dioxo-1(2H)-pyrimidinyl]-4-fluorbenzoesäure-methylester.

Weitere Vertreter derartigen Verbindungen der Formel I sind die Aethyl-, n-Propyl-, n-Butyl-, n-Hexyl-, n-Octyl-, sek.-Butyl-, 1-Methylbutyl-, Neopentyl-, Isobutyl-, Isopentyl-, 2-Methylbutyl-, 1,2-Dimethylpropyl-, tert.Butyl-, 1-Aethylpropyl-, Allyl-, 2-Butenyl-, 3-Methyl-2-butenyl-, 3-Methyl-3-butenyl-, 1-Aethyl-2-propenyl-, 4-Pentenyl-, 1-Methyl-2-butenyl-, 1-Methyl-3-butenyl-, 3-Butenyl-, 2-Methyl-2-propenyl-, 3-Butinyl-, 2-Butinyl-, Methoxymethyl-, Aethoxymethyl-, n-Propoxymethyl-, Isopropoxymethyl-, n-Pentyloxymethyl-, 1-Methoxyäthyl-, 2-Methoxyäthyl-, 2-Aethoxyäthyl-, 2-Isopropoxyäthyl-, 2-Methoxy-1-methyläthyl-, 2-n-Butyloxyäthyl-, Cyclopentyl-, Cyclohexyl-, Cycloheptyl-, Cyclopropylmethyl-, 1-Cyclopropyläthyl-, 3-Tetrahydrofuryl-, Tetrahydrofurfuryl-, 1,3-Dioxan-5-yl-, 1,3-Dioxolan-5-ylmethyl-, Benzyl- und 4-Methoxyphenylester von 2-Chlor-5-[3-difluormethyl-3,6-dihydro-4-methyl-2,6 -dioxo-1(2H)-pyrimidinyl]-4-fluorbenzoesäure, der 2-Chlor-5-[3-difluormethyl-3,6-dihydro-4,5-dimethyl-2,6-dioxo-1(2H)-pyrimidinyl]-4-fluorbenzoesäureisopropylester, der 2-Chlor-5-[3-difluormethyl-3,6-dihydro-5-fluor-4-methyl-2,6-dioxo-1(2H)-pyrimidinyl]-4-fluorbenzoesäure-isopropylester, der 2-Chlor-5-[3-difluormethyl-3,6-dihydro-4-methyl-2,6 -dioxo-1(2H)-pyrimidinyl]-benzoesäure-isopropylester und der 2-Chlor-5-[3,6-dihydro-4-methyl-3-(1,1,2,2-tetrafluoräthyl) -2,6-dioxo-1(2H)-pyrimidinyl] -4-fluorbenzoesäure-isopropylester.

Das erfindungsgemässe Verfahren zur Herstellung der Verbindungen der Formel I und ihrer Enoläther sowie Salze ist dadurch gekennzeichnet, dass man

a) zwecks Herstellung derjenigen Verbindungen der Formel I, in denen $R^1$ Wasserstoff bedeutet, sowie gewünschtenfalls von Metallsalzen dieser Verbindungen, eine Verbindung der allgemeinen Formel

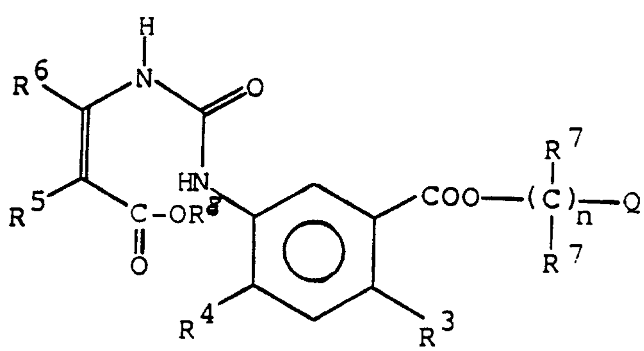

II

worin $R^3$, $R^4$, $R^5$, $R^6$, $R^7$, n und Q die oben angegebenen Bedeutungen besitzen und $R^8$ nieder Alkyl, vorzugsweise $C_{1-4}$-Alkyl, bedeutet,

einer Cyclisierung unter basischen Bedingungen unterwirft und gewünschtenfalls ein allfällig erhaltenes Metallsalz des Uracilderivats der Formel I durch Behandlung mit einer Säure in die saure Form ($R^1$ = Wasserstoff) überführt,

b) zwecks Herstellung derjenigen Verbindungen der Formel I, in denen $R^1$ $C_{1-4}$-Alkyl, $C_{3\ oder\ 4}$-Alkenyl, $C_{3\ oder\ 4}$-Alkinyl oder $C_{1-4}$-Halogenalkyl bedeutet, wobei, im Fall, dass $R^1$ $C_{1-4}$-Halogenalkyl bedeutet, $R^2$ verschieden von Wasserstoff ist, ein Uracilderivat der allgemeinen Formel

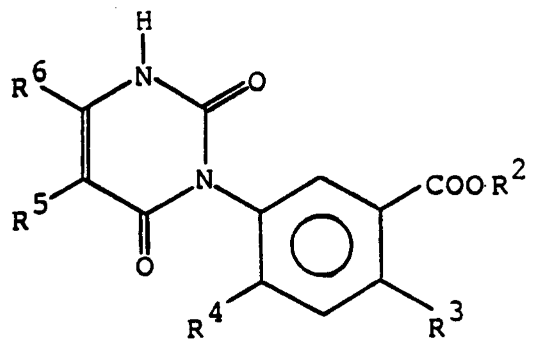

I'

worin $R^2$, $R^3$, $R^4$, $R^5$ und $R^6$ die oben angegebenen Bedeutungen besitzen,

einer Alkylierung mit einem entsprechenden, eine $C_{1-4}$-Alkyl-, $C_{3\ oder\ 4}$-Alkenyl- oder $C_{3\ oder\ 4}$-Alkinylgruppe enthaltenden Alkylierungsmittel unterwirft, mit der Massgabe, dass im Falle der Herstellung einer Verbindung der Formel I, in der $R^1$ $C_{1-4}$-Halogenalkyl bedeutet, $R^2$ der Formel I' verschieden von Wasserstoff ist,

c) zwecks Herstellung derjenigen Verbindungen der Formel I, in denen $R^1$ verschieden von Wasserstoff ist, und der entsprechenden Enoläther eine Benzoesäure der allgemeinen Formel

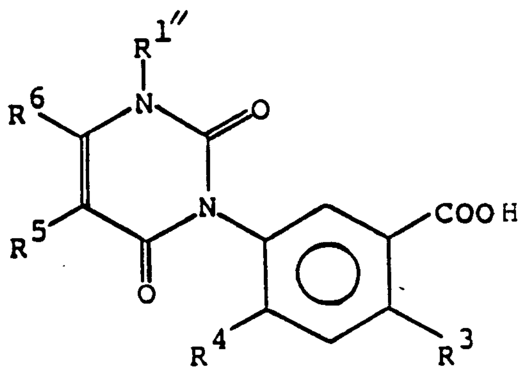

III

worin $R^3$, $R^4$, $R^5$ und $R^6$ die oben angegebenen Bedeutungen besitzen, und $R^{1''}$ $C_{1-4}$-Alkyl, $C_{3\ oder\ 4}$-Alkenyl, $C_{3\ oder\ 4}$-Alkinyl oder $C_{1-4}$-Halogenalkyl bedeutet.

oder den entsprechenden Enoläther, wobei die Benzoesäure bzw. deren Enoläther in Form eines reaktionsfähigen Derivats vorliegen kann, mit einer Hydroxyverbindung der allgemeinen Formel

$R^2OH$ IV

worin $R^2$ die oben angegebene Bedeutung besitzt , mit der Massgabe, dass im Falle der Herstellung einer Verbindung der Formel I, in der $R^1$ $C_{1-4}$-Halogenalkyl bedeutet, $R^2$ der Formel IV verschieden von Wasserstoff ist,

oder mit einem reaktionsfähigen Derivat dieser Hydroxyverbindung verestert,

d) zwecks Herstellung derjenigen Verbindungen der Formel I, in denen $R^1$ verschieden von Wasserstoff ist, einen Benzoesäureester der allgemeinen Formel

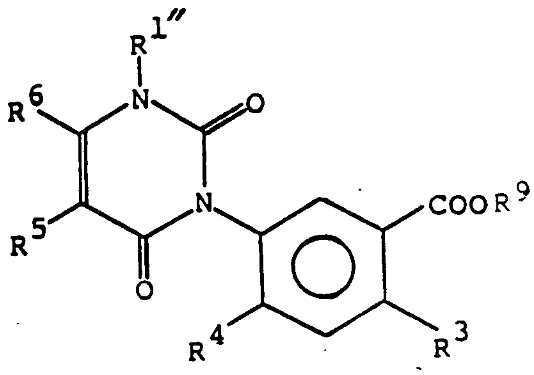

V

worin $R^{1''}$, $R^3$, $R^4$, $R^5$ und $R^6$ die oben angegebenen Bedeutungen besitzen und $R^9$ $C_{1-6}$-Alkyl, $C_{2-4}$-Alkenyl, $C_{2-4}$-Alkinyl oder $C_{2-6}$-Alkoxyalkyl bedeutet,
einer Umesterungsreaktion mit einer Hydroxyverbindung der oben angegebenen Formel IV unterwirft, wobei das Reagens IV höher siedend ist als das Alkanol, Alkenol bzw. Alkinol $R^9OH$,
e) zwecks Herstellung derjenigen Verbindungen der Formel I, in denen $R^1$ $C_{1-4}$-Halogenalkyl und $R^2$ Wasserstoff bedeuten, einen Benzoesäureester der allgemeinen Formel

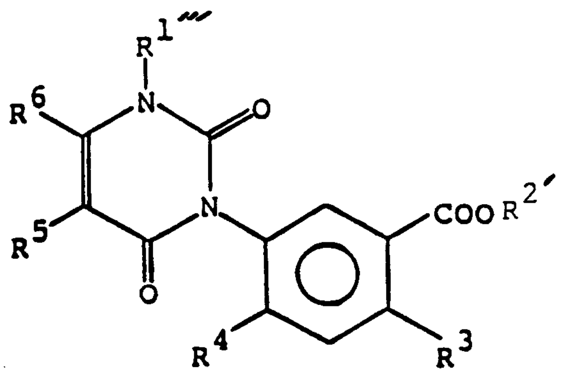

I''''

worin $R^3$, $R^4$, $R^5$ und $R^6$ die oben angegebenen Bedeutungen besitzen und $R^{1'''}$ $C_{1-4}$-Halogenalkyl bedeutet, und $R^{2'}$ die Bedeutung von $R^2$ mit Ausnahme von Wasserstoff besitzt,
zur entsprechenden Benzoesäure hydrolysiert,
f) zwecks Herstellung der Enoläther der Verbindungen der Formel I, ein Uracilderivat der allgemeinen Formel

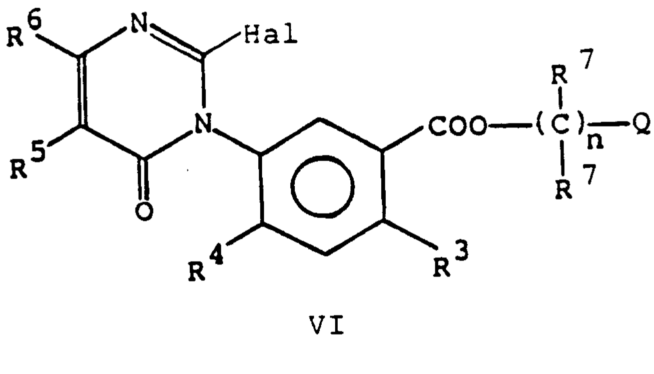

VI

worin $R^3$, $R^4$, $R^5$, $R^6$, $R^7$, n und Q die oben angegebenen Bedeutungen besitzen und Hal Chlor oder Brom bedeutet,
mit einem Alkanol, Alkenol oder Alkinol $R^{1'}OH$ in Gegenwart einer organischen Base oder mit dem entsprechenden Alkoholat, Alkenolat bzw. Alkinolat der allgemeinen Formel

$R^{1'}O^{\ominus} M^{\oplus}$ VII

worin $R^{1'}$ die oben angegebene Bedeutung besitzt und $M^{\oplus}$ ein Aequivalent eines Metallions bedeutet,

behandelt, und gewünschtenfalls eine so erhaltene Verbindung der Formel I, in der $R^1$ oder $R^2$ Wasserstoff bedeutet, in ein Salz überführt.

Die Cyclisierung nach Verfahrensvariante a) kann zweckmässigerweise durchgeführt werden, indem man die Verbindung der Formel II in einem inerten protischen organischen Lösungsmittel, wie einem Alkohol, z.B. Methanol, Aethanol oder Isopropanol; einem inerten aprotischen organischen Lösungsmittel, wie einem aliphatischen oder cyclischen Aether, z.B. 1,2-Dimethoxyäthan, Tetrahydrofuran oder Dioxan, oder einem Aromaten, z.B. Benzol oder Toluol; einem inerten aprotischen, polaren organischen Lösungsmittel, z.B. Dimethylformamid oder Dimethylsulfoxid, wobei solche Lösungsmittel gegebenenfalls im Zweiphasen-Gemisch mit einem Kohlenwasserstoff, z.B. n-Hexan oder Toluol, verwendet werden können; oder Wasser mit einer Base bei Temperaturen zwischen -78°C und der Rückflusstemperatur des Reaktionsgemisches behandelt. Als Basen kommen vorzugsweise Natriumalkoholate, Alkalimetallhydroxide, insbesondere Natriumhydroxid und Kaliumhydroxid, Alkalimetallcarbonate, insbesondere Natriumcarbonat und Kaliumcarbonat, und Natriumhydrid in Betracht. Falls als Lösungsmittel ein Alkanol verwendet wird, entspricht dieses Lösungsmittel zweckmässigerweise der jeweiligen Hydroxyverbindung $Q\text{-}(CR^7R^7)_n\text{-}OH$; dadurch werden unerwünschte konkurrierende Umesterungsreaktionen vermieden. Bei der Verwendung von Natriumhydrid als Base ist das Lösungsmittel vorzugsweise ein aliphatischer oder cyclischer Aether, Dimethylformamid oder Dimethylsulfoxid, wobei jedes dieser Lösungsmittel im Gemisch mit Toluol verwendet werden kann.

Nach Beendigung der Cyclisierung liegt das Produkt im Falle der Verwendung einer der obenerwähnten Basen oder dergleichen in Form des entsprechenden Alkalimetallsalzes vor. Dieses kann in an sich bekannter Weise isoliert und gereinigt werden, oder man kann das Gemisch ansäuern, um die jeweilige Verbindung der Formel I an sich zu isolieren. Zu diesem Zwecke verwendet man vorzugsweise eine Mineralsäure, wie Salzsäure, oder eine starke organische Säure, wie Essigsäure oder p-Toluolsulfonsäure.

Bei der Verfahrensvariante b) steht der Ausdruck "Alkylierung" für die Substitution des Wasserstoffatoms des $N^1$-Atoms des Uracilkerns mit einer $C_{1-4}$-Alkyl-, $C_{3\ \text{oder}\ 4}$-Alkenyl-, $C_{3\ \text{oder}\ 4}$-Alkinyl- oder $C_{1-4}$-Halogenalkylgruppe. Als Alkylierungsmittel wird zweckmässigerweise ein $C_{1-4}$-Alkyl-, $C_{3\ \text{oder}\ 4}$-Alkenyl- oder $C_{3\ \text{oder}\ 4}$-Alkinylhalogenid, insbesondere das diesbezügliche Chlorid oder Bromid, oder -sulfat, bzw. ein mehrfach halogeniertes $C_{1-4}$-Alkan, wie beispielsweise Chlordifluormethan, oder ein mono- oder mehrfach halogeniertes Alken, wie beispielsweise Tetrafluoräthen, verwendet.

Die Alkylierung wird zweckmässigerweise in Gegenwart eines inerten, protischen organischen Lösungsmittels, wie eines niederen Alkanols, z.B. Aethanol, gegebenenfalls im Gemisch mit Wasser; eines inerten, aprotischen organischen Lösungsmittels, wie eines aliphatischen oder cyclischen Aethers, z.B. 1,2-Dimethoxyäthan, Tetrahydrofuran oder Dioxan; eines Ketons, z.B. Aceton oder Butan-2-on; oder eines inerten, aprotischen, polaren organischen Lösungsmittels, z.B. Dimethylformamid, Dimethylsulfoxid oder Acetonitril, sowie in Gegenwart einer Base, wie Natriumhydrid, eines Alkalimetallhydroxids, insbesondere Natrium- oder Kaliumhydroxid, eines Alkalimetallalkoholats, insbesondere Natriumalkoholat, oder eines Alkalimetallcarbonats oder -hydrogencarbonats, insbesondere Natriumcarbonat, Kaliumcarbonat, Natriumhydrogencarbonat oder Kaliumhydrogencarbonat, bei Temperaturen zwischen 0°C und der Rückflusstemperatur des Reaktionsgemisches, vorzugsweise bei Raumtemperatur oder, im Falle der Substitution des Wasserstoffatoms des $N^1$-Atoms mit einer $C_{1-4}$-Halogenalkylgruppe, vorzugsweise bei Temperaturen zwischen 50°C und 100°C, durchgeführt. In einer bevorzugten Ausführungsform wird das Uracilderivat der Formel I' zunächst mit der Base, wie Natriumhydrid, -äthanolat oder -carbonat, im Lösungsmittel behandelt und nach einer kurzen Reaktionszeit mit dem Halogenid im gleichen Lösungsmittel versetzt. In einer weiteren Ausführungsform wird das Uracilderivat I' zusammen mit einem Dialkylsulfat in Gegenwart eines Alkalimetallhydrogencarbonats, insbesondere Natrium- oder Kaliumhydrogencarbonat, im Lösungsmittel, z.B. Aceton, bei Rückflusstemperatur zur Reaktion gebracht. Die Reaktion ist in der Regel je nach verwendetem Lösungsmittel innert relativ kurzer Zeit oder nach wenigen Stunden beendet.

Bei der Verfahrensvariante c) handelt es sich um eine Veresterung der Benzoesäure oder des Enoläthers bzw. eines reaktionsfähigen Derivats davon, die nach an sich bekannten Methoden durchgeführt werden kann. So wird beispielsweise ein Salz einer Benzoesäure der Formel III oder des entsprechenden Enoläthers mit einem Halogenid, insbesondere Chlorid, Bromid oder Jodid, oder dem Sulfat, Mesylat oder Tosylat der Hydroxyverbindung IV in einem inerten Verdünnungsmittel bei Temperaturen zwischen dem Raumtemperatur und 100°C, z.B. bei der Rückflusstemperatur des Reaktionsgemisches, vorzugsweise im Temperaturbereich von 40°C bis 70°C, umgesetzt. Es kommen als Salze der Benzoesäure der Formel III

oder des entsprechenden Enoläthers insbesondere Alkalimetallsalze, z.B. das Natrium-, Kalium- oder Lithiumsalz, Erdalkalimetallsalze, z.B. das Magnesium-, Calcium- oder Bariumsalz, und Salze mit organischen Basen, wie tertiäre Amine, z.B. Triäthylamin, 1,5-Diaza-bicyclo[4,3,0]-non-5-en, 1,8-Diaza-bicyclo-[5,4,0]undec-7-en und 1,4-Diaza-bicyclo[2,2,2]octan, in Frage, wobei die Alkalimetallsalze, insbesondere das Natriumsalz und das Kaliumsalz, bevorzugt sind. Die verwendbaren Verdünnungsmittel sind vorzugsweise inerte organische Lösungsmittel, wie niedere Alkanole, z.B. Aethanol, aliphatische und cyclische Aether, z.B. Diäthyläther, 1,2-Dimethoxyäthan, Tetrahydrofuran und Dioxan, Ketone, z.B. Aceton und 2-Butanon, Dimethylformamid, Dimethylsulfoxid, Acetonitril und Hexamethylphosphorsäuretriamid. Das Salz kann in situ herge- stellt werden, indem man die Säure mit einer geeigneten anorganischen Base, z.B. einem Alkalimetall- oder Erdalkalimetallcarbonat, -hydrogencarbonat, -hydroxid oder -hydrid bzw. organischen Base, zum Salz umsetzt und dies anschliessend im gleichen Reaktionsmedium mit dem zweiten Reaktionspartner reagieren lässt.

Im Falle der Verwendung eines Säurehalogenids der Benzoesäure der Formel III oder des entsprechenden Enoläthers als reaktionsfähiges Derivat wird dies zweckmässigerweise mit der Hydroxyverbindung der Formel IV in einem inerten organischen Lösungsmittel, wie einem aliphatischen oder cyclischen Aether, z.B. Diäthyläther, 1,2-Dimethoxyethan, Tetrahydrofuran oder Dioxan, einem aliphatischen oder aromatischen Kohlenwasserstoff, z.B. n-Hexan, Benzol oder Toluol, oder einem halogenierten, insbesondere chlorierten, Kohlenwasserstoff, z.B. Methylenchlorid, Chloroform oder Tetrachlorkohlenstoff, bei Temperaturen von etwa -20°C bis 100°C, vorzugsweise von 0°C bis 50°C, umgesetzt. Zudem wird zweckmässigerweise in Gegenwart eines säurebindenden Mittels gearbeitet, wie einer organischen Base, z.B. Triäthylamin, Pyridin, 1,5-Diaza-bicyclo[4,3,0]non-5-en, 1,8-Diaza-bicyclo[5,4,0]undec-7-en oder 1,4-Diaza-bicyclo[2,2,2]octan. Das Säurehalogenid ist vorzugsweise das Säurechlorid.

Als weitere in Frage kommende reaktionsfähige Derivate der Benzoesäure der Formel III oder des entsprechenden Enoläthers seien der entsprechende O-Acyl-1,3-dicyclohexylisoharnstoff und das entsprechende N-Acylimidazol oder Säureanhydrid genannt. Solche Derivate können wie das Säurehalogenid mit den Hydroxyverbindungen der Formel IV umgesetzt werden, um zu den gewünschten Benzoesäureestern zu gelangen. In diesen Fällen erübrigt sich jedoch die Verwendung eines säurebindenden Mittels.

Die Umsetzung nach Verfahrensvariante d) kann zweckmässigerweise durchgeführt werden, indem man den Benzoesäureester der Formel V in überschüssiger Hydroxyverbindung der Formel IV in Gegenwart eines basischen Katalysators, wie Natriumcyanid, erhitzt, und zwar vorzugsweise bei der Rückflusstemperatur des Reaktionsgemisches. Im Laufe der Reaktion wird der Rest $R^9$ des Benzoesäureesters V durch die Gruppe $R^2$ der Hydroxyverbindung IV ersetzt, wobei das niedriger siedende Alkanol, Alkenol bzw. Alkinol $R^9OH$ freigesetzt wird.

Die Hydrolyse des Benzoesäureesters I" nach Verfahrensvariante e) kann nach an sich bekannten Methoden durchgeführt werden, insbesondere unter Verwendung einer starken Mineralsäure, wie Schwefelsäure, oder einer Base, wie eines Alkalimetall- oder Erdalkalimetallhydroxids, z.B. Natriumhydroxid oder Kaliumhydroxid, und gegebenenfalls in Gegenwart eines organischen Lösungsmittels, wie eines Alkohols, z.B. Methanol oder Aethanol, oder eines chlorierten Kohlenwasserstoffes, z.B. Methylenchlorid, gewünschtenfalls auch im Gemisch mit Wasser. Die Hydrolyse erfolgt zweckmässigerweise bei Temperaturen zwischen -20°C und 100°C, vorzugsweise bei Raumtemperatur. Falls das Produkt nach alkalischer Hydrolyse als Salz vorliegt, kann dieses beispielsweise durch einfaches Abdampfen des Lösungsmittels isoliert werden. Die freie Säure kann man dann erhalten, indem man das Salz mit einer Säure, vorzugsweise einer Mineralsäure, z.B. Salzsäure oder Schwefelsäure, ansäuert.

Bei der Verfahrensvariante f) steht der Ausdruck "Metallion" insbesondere für ein Alkalimetallion, z.B. das Natrium- oder Kaliumion, oder ein Erdalkalimetallion, z.B. das Calcium- oder Magnesiumion. Das Natriumion ist das bevorzugte Metallion. Im Falle der Verwendung des Alkanols, Alkenols oder Alkinols $R^{1'}OH$ ist insbesondere Pyridin die geeignete organische Base.

Die Umsetzung erfolgt zweckmässigerweise in einem Ueberschuss an dem entsprechenden Alkohol $R^{1'}OH$ als Verdünnungsmittel und bei Temperaturen zwischen 0°C und 50°C, vorzugsweise bei Raumtemperatur.

Sofern sie nicht unmittelbar durch die oben beschriebene unter basischen Bedingungen durchgeführte Cyclisierung herstellbar sind, können die gewünschten Salze der Verbindungen der Formel I, in denen $R^1$ oder $R^2$ Wasserstoff bedeutet, auch aus diesen Verbindungen I in an sich bekannter Weise hergestellt werden, wie beispielsweise durch Auflösen der Verbindung der Formel I in einer Lösung einer diesbezüglichen anorganischen oder organischen Base. Die Salzbildung erfolgt in der Regel innert kurzer Zeit bei Raumtemperatur. In einer Ausführungsform wird das Natriumsalz durch Auflösen des Uracilderivats I in wässriger Natriumhydroxidlösung bei Raumtemperatur hergestellt, wobei äquivalente Mengen des Uracilderivats und des Natriumhydroxids verwendet werden. Das feste Salz kann dann durch Fällen mit einem

geeigneten inerten Lösungsmittel oder durch Abdampfen des Lösungsmittels isoliert werden. Eine weitere Ausführungsform besteht darin, eine wässrige Lösung eines Alkalimetallsalzes des Uracilderivats I in eine wässrige Lösung eines Salzes, das ein anderes Metallion als ein Alkalimetallion aufweist, einzuführen, wobei das zweite Metallsalz des Uracilderivats hergestellt wird. Diese Ausführungsform dient im allgemeinen zur Herstellung von Uracil-Metallsalzen, die in Wasser unlöslich sind.

Die erhaltenen Verbindungen der Formel I, Enoläther sowie Salze können nach an sich bekannten Methoden isoliert und gereinigt werden. Ferner ist dem Fachmann geläufig, in welcher Reihenfolge allfällige Kombinationen der Verfahrensvarianten b) bis f) zweckmässig durchzuführen sind, um mögliche, unerwünschte konkurrierende Reaktionen zu vermeiden.

Sofern keine gezielte Synthese zur Isolierung reiner Isomerer durchgeführt wird, kann das Produkt als Gemisch zweier oder mehrerer Isomerer anfallen. Die Isomeren können nach an sich bekannten Methoden aufgetrennt werden. Gewünschtenfalls können beispielsweise reine optisch aktive Isomere auch durch Synthese aus entsprechenden optisch aktiven Ausgangsmaterialien hergestellt werden.

Die Ausgangsmaterialien der Formel II, die neu sind, können in an sich bekannter Weise hergestellt werden, z.B. gemäss den nachfolgenden Reaktionsschemata [Methoden aa), bb) und cc)]:

## Reaktionsschemata

aa)

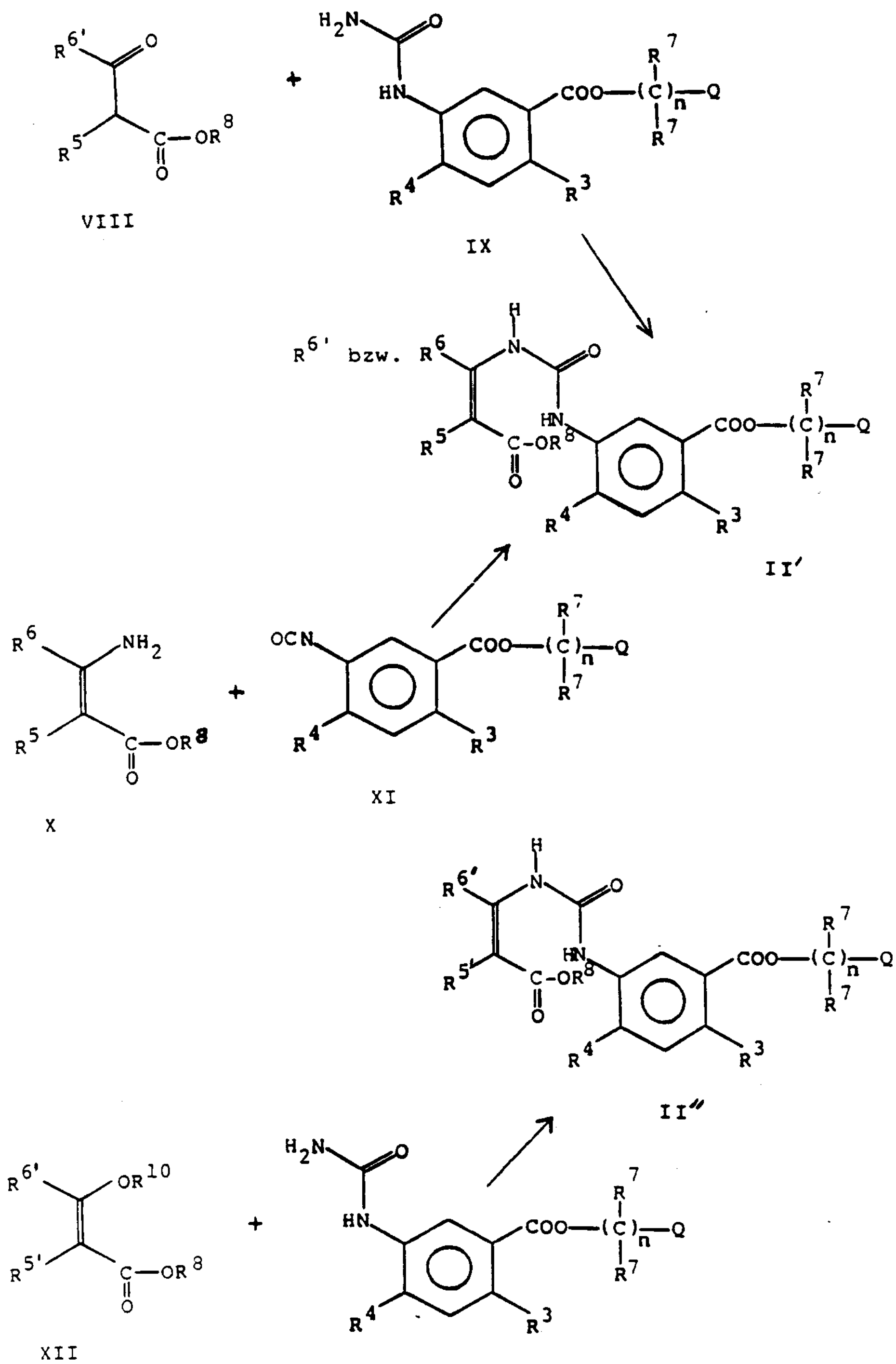

VIII

IX

II'

bb)

X

XI

II''

cc)

XII

IX

In den obigen Reaktionsschemata besitzen $R^3$, $R^4$, $R^5$, $R^6$, $R^7$, $R^8$, n und Q die oben angegebenen Bedeutungen; $R^{5'}$ bedeutet Wasserstoff oder $C_{1-4}$-Alkyl; $R^{6'}$ bedeutet $C_{1-4}$-Alkyl; und $R^{10}$ bedeutet nieder Alkyl, vorzugsweise $C_{1-4}$-Alkyl.

Die Methode aa) wird zweckmässigerweise dadurch durchgeführt, dass man die Verbindungen der Formeln VIII und IX in einem im wesentlichen wasserfreien Verdünnungsmittel und in Gegenwart eines

sauren Katalysators bei erhöhter Temperatur miteinander reagieren lässt. Als Verdünnungsmittel kommen insbesondere mit Wasser Azeotrope bildende organische Lösungsmittel, wie Aromaten, z.B. Benzol, Toluol und Xylole; halogenierte Kohlenwasserstoffe, wie Methylenchlorid, Chloroform, Tetrachlorkohlenstoff und Chlorbenzol; und aliphatische und cyclische Aether, wie 1,2-Dimethoxyäthan, Tetrahydrofuran und Dioxan, und als saure Katalysatoren insbesondere starke Mineralsäuren, wie Schwefelsäure und Salzsäure; organische Säuren, wie p-Toluolsulfonsäure; Phosphor enthaltende Säuren, wie Orthophosphorsäure und Polyphosphorsäure; und saure Kationenaustauscher, wie "Amberlyst 15" (Fluka), in Frage. Man arbeitet im allgemeinen in einem Temperaturbereich von etwa 70 °C bis 120 °C, vorzugsweise bei der Rückflusstemperatur des Reaktionsgemisches. Unter diesen Reaktionsbedingungen wird die erwünschte rasche Entfernung des in der Reaktion gebildeten Wassers erzielt.

Die Umsetzung nach der Methode bb) erfolgt zweckmässigerweise in Gegenwart eines im wesentlichen wasserfreien aprotischen organischen Lösungsmittels, wie eines aliphatischen oder cyclischen Aethers, z.B. Diäthyläther, 1,2-Dimethoxyäthan, Tetrahydrofuran oder Dioxan, eines aliphatischen oder aromatischen Kohlenwasserstoffs, z.B. n-Hexan, Benzol, Toluol oder eines Xylols; oder eines halogenierten, aliphatischen Kohlenwasserstoffs, z.B. Methylenchlorid, Chloroform, Tetrachlorkohlenstoff oder 1,2-Dichloräthan, sowie gegebenenfalls in Gegenwart einer Base, insbesondere einer organischen tertiären Base, wie Triäthylamin oder Pyridin, wobei letzteres sowohl als Lösungsmittel wie auch als Base dienen kann, oder eines Metallhydrids, wie Natrium- oder Kaliumhydrid. Die Reaktionstemperaturen sind vorzugsweise im Bereich von etwa -80 °C bis 50 °C, wobei besonders bevorzugt bei Temperaturen zwischen -30 °C und der Raumtemperatur gearbeitet wird.

Die Umsetzung nach der Methode cc) wird zweckmässigerweise in einem inerten, mit Wasser mischbaren, organischen Lösungsmittel, wie einem aliphatischen oder cyclischen Aether, z.B. 1,2-Dimethoxyäthan, Tetrahydrofuran oder Dioxan, oder einem niederen Alkanol, wie Aethanol, bei Temperaturen zwischen 50 °C und 100 °C, vorzugsweise bei der Rückflusstemperatur des Reaktionsgemisches, oder in einem aromatischen Lösungsmittel, wie Benzol, Toluol oder einem Xylol in Gegenwart eines sauren Katalysators, wie Salzsäure oder p-Toluolsulfonsäure, bei Temperaturen zwischen 50 °C und 100 °C, vorzugsweise 60 °C bis 80 °C, durchgeführt.

Die Ausgangsmaterialien der Formeln III und V und deren Herstellung sind grösstenteils in der europäischen Patent-publikation Nr. 195.346 beschrieben. Diejenigen Ausgangsmaterialien III und V, deren Herstellung nicht beschrieben ist, können analog den bekannten Ausgangsmaterialien hergestellt werden. Die ebenfalls als Ausgangsmaterialien verwendbaren reaktionsfähigen Derivate der Benzoesäuren der Formel III können aus dieser Benzoesäuren nach an sich bekannten Methoden hergestellt werden. Neu sind hingegen sämtliche, ebenfalls bei der Verfahrensvariante c) als Ausgangsmaterialien verwendbare Enoläther der Benzoesäuren III, d.h. die Verbindungen der allgemeinen Formel

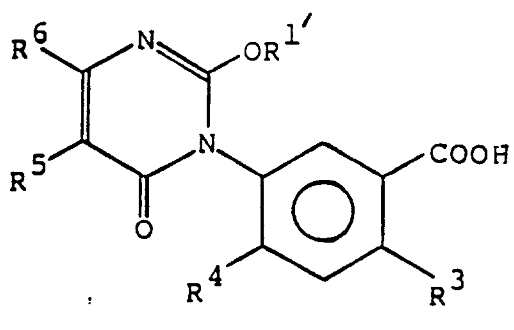

IIIa

Diese können beispielsweise gemäss dem nachfolgenden Reaktionsschema, in dem $R^3$, $R^4$, $R^5$, $R^6$, $R^{1'}$, $R^9$, Hal und $M^⊕$ die oben angegebenen Bedeutungen besitzen, hergestellt werden:

Reaktionsschema

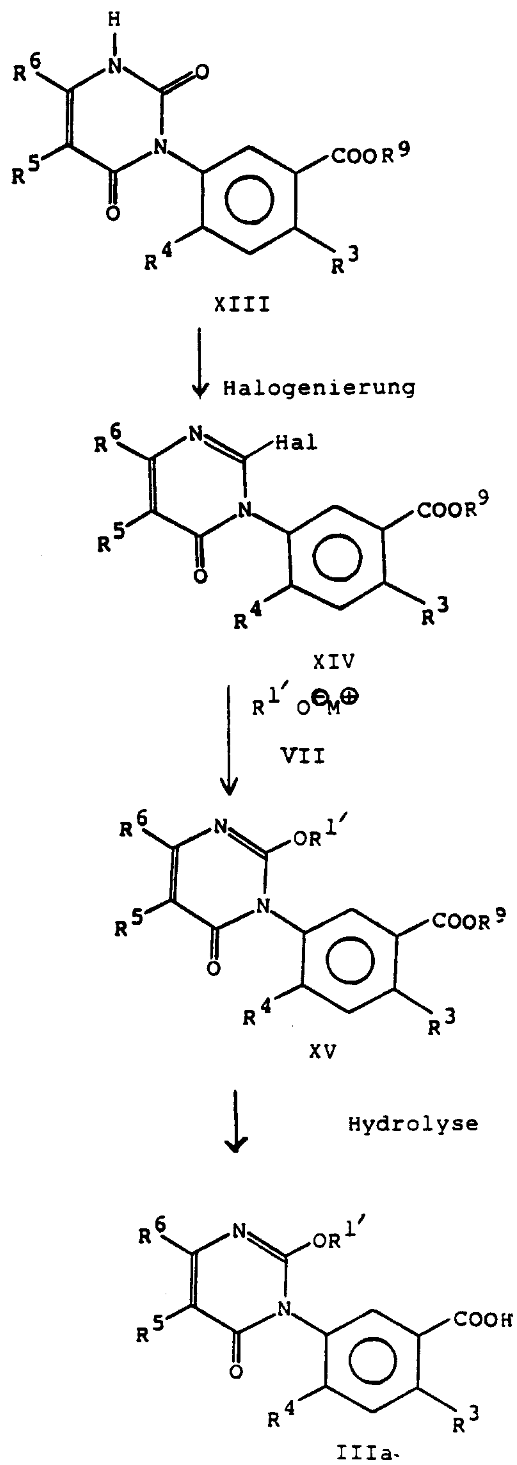


Bei der Halogenierung des Benzoesäureesters der Formel XIII wird als Halogenierungsmittel insbesondere Thionylchlorid, Phosphorpentachlorid oder Phosphoroxychlorid bzw. Phosphorpentabromid oder Phosphorylbromid verwendet. Gegebenenfalls wird ein Gemisch von Phosphorpentachlorid und Phosphoroxychlorid bzw. von Phosphorpentabromid und Phosphorylbromid eingesetzt, wobei ein Ueberschuss an Phosphoroxychlorid bzw. Phosphorylbromid als Verdünnungsmittel dienen kann. Die Chlorierung bzw.

Bromierung kann in Gegenwart eines inerten Verdünnungsmittels, insbesondere eines aprotischen organischen Lösungsmittels, wie eines aliphatischen oder aromatischen Kohlenwasserstoffes, z.B. n-Hexan, Benzol, Toluol oder eines Xylols; eines halogenierten aliphatischen Kohlenwasserstoffes, z.B. Methylenchlorid, Chloroform oder 1,2-Dichloräthan; eines halogenierten aromatischen Kohlenwasserstoffes, z.B. Chlorbenzol, oder eines tertiären Amins, z.B. N,N-Dimethylanilin, durchgeführt werden, jedoch ist dies im Falle der Verwendung von Phosphoroxychlorid bzw. Phosphorylbromid als Halogenierungsmittel nicht notwendig. Bei Verwendung von Thionylchlorid als Halogenierungsmittel erweist es sich als zweckmässig, eine katalytische Menge Dimethylformamid zuzusetzen. Die Reaktionstemperaturen liegen im allgemeinen zwischen 0°C und der Rückflusstemperatur des Reaktionsgemisches, vorzugsweise zwischen 80°C und 120°C.
Die Umsetzung der Verbindung XIV mit der Verbindung VII kann analog der oben beschriebenen Verfahrensvariante f) erfolgen.

Die anschliessende Hydrolyse der Verbindung XV kann nach an sich bekannten Methoden durchgeführt werden, insbesondere unter Verwendung einer anorganischen Säure und gegebenenfalls in Gegenwart eines organischen Lösungsmittels und/oder von Wasser. Als Säuren kommen vorzugsweise Salzsäure, Schwefelsäure und Phosphorsäure, als organische Lösungsmittel Alkohole, z.B. Aethanol; aliphatische oder cyclische Aether, z.B. 1,2-Dimethoxyäthan, Tetrahydrofuran und Dioxan, und gegebenenfalls chlorierte aliphatische oder alicyclische Kohlenwasserstoffe, z.B. Methylenchlorid, Tetrachlormethan, n-Hexan und Cyclohexan, in Frage. Die Reaktionstemperaturen liegen im allgemeinen zwischen -20°C und 120°C, vorzugsweise zwischen 0°C und 30°C, insbesondere zwischen 15°C und 20°C.

Die ebenfalls als Ausgangsmaterialien verwendbaren reaktionsfähigen Derivate der Enoläther der Benzoesäuren III können aus diesen Enoläthern nach an sich bekannten Methoden hergestellt werden.

Die in der Verfahrensvariante f) verwendeten Ausgangsmaterialien der Formel VI können durch Halogenierung der entsprechenden Uracilderivate der oben angegebenen Formel I' hergestellt werden, und zwar analog dem oben beschriebenen Verfahren XIII→XIV (siehe das Reaktionsschema und die darauf folgende Beschreibung der Reaktionsbedingungen).

Bei den als Ausgangsmaterialien in der Verfahrensvarianten b) und e) dienenden Uracilderivaten der Formel I' bzw. I'' handelt es sich um Untergruppen von Verbindungen der Formel I. Die restlichen in den Verfahrensvarianten c), d) und f) sowie in den Reaktionsschemata involvierten Ausgangsmaterialien bzw. Reagenzien sind entweder bekannt oder können nach an sich bekannten Methoden hergestellt werden.

Die Verbindungen der Formel I sowie ihre Enoläther oder Salze besitzen herbizide Eigenschaften und eignen sich zur Bekämpfung von Unkräutern, einschliesslich Ungräsern, insbesondere Setaria faberii, Digitaria sanguinalis, Poa annua, Chenopodium album, Amaranthus retroflexus, Abutilon theophrasti, Sinapsis alba und Datura stramonium, in diversen Nutzpflanzenkulturen, insbesondere in Getreide-, Soja-, Mais-, Reis- und Baumwollekulturen. Zudem sind die Verbindungen sowohl Vorauflauf- als auch Nachauflauf-Herbizide.

Auch die Verbindungen der Formel IIIa sowie ihre Salze besitzen herbizide Eigenschaften und können auf ähnliche Weise wie die Verbindungen I zur Bekämpfung von Ungräsern und Unkräutern, insbesondere den obenerwähnten, eingesetzt werden. Die neuen Verbindungen IIIa sowie ihre Salze, Unkrautbekämpfungsmittel, welche diese Verbindungen als Wirkstoffe enthalten, das oben definierte Verfahren zur Herstellung dieser Verbindungen und die Verwendung dieser Verbindungen bzw. Mittel zur Bekämpfung von Unkräutern bilden einen weiteren Gegenstand der vorliegenden Erfindung. Bei den Salzen der Verbindungen IIIa handelt es sich insbesondere um Alkalimetallsalze, Erdalkalimetallsalze, Ammoniumsalze und Salze mit organischen Basen, wie diese oben im Zusammenhang mit den Verbindungen I näher beschrieben sind. Die Verbindungen IIIa können wie die Verbindungen I in ihre Salze übergeführt werden. Im folgenden werden die Verbindungen der Formel I und ihre Enoläther oder Salze sowie die Verbindungen der Formel IIIa und ihre Salze insgesamt als erfindungsgemässe Verbindungen bezeichnet.

In der Praxis genügt üblicherweise eine Konzentration von 1 g bis 3 kg erfindungsgemässe Verbindung/ha, vorzugsweise 10 bis 500 g erfindungsgemässe Verbindung/ha, um den gewünschten herbiziden Effekt zu erzielen. Besonders bevorzugt ist die Konzentrationsreihe 15 bis 100 g erfindungsgemässe Verbindung/ha.

Das erfindungsgemässe Unkrautbekämpfungsmittel ist dadurch gekennzeichnet, dass es eine wirksame Menge mindestens einer Verbindung der Formel I, wie oben definiert, oder eines Enoläthers oder Salzes davon, bzw. einer Verbindung der Formel IIIa oder eines Salzes davon sowie Formulierungshilfsstoffe enthält. Das Mittel enthält zweckmässigerweise zumindest einen der folgenden Formulierungshilfsstoffe: feste Trägerstoffe; Lösungs- bzw. Dispersionsmittel; Tenside (Netz- und Emulgiermittel); Dispergatoren (ohne Tensidwirkung); und Stabilisatoren. Unter Verwendung solcher und anderer Hilfsstoffe können diese Verbindungen, also die herbiziden Wirkstoffe, in die üblichen Formulierungen übergeführt werden, wie

Stäube, Pulver, Granulate, Lösungen, Emulsionen, Suspensionen, emulgierbare Konzentrate, Pasten und dergleichen.

Die Verbindungen der Formel I, ihre Enoläther und die Verbindungen der Formel IIIa sind im allgemeinen wasserunlöslich, die Salze hingegen, insbesondere die Alkalimetallsalze und Ammoniumsalze, im allgemeinen wasserlöslich, und können nach den für wasserunlösliche bzw. wasserlösliche Verbindungen üblichen Methoden unter Verwendung der diesbezüglichen Formulierungshilfsstoffe konfektioniert werden. Die Herstellung der Mittel kann in an sich bekannter Weise durchgeführt werden, z.B. durch Vermischen des jeweiligen Wirkstoffes mit festen Trägerstoffen, durch Auflösen oder Suspendieren in geeigneten Lösungs- bzw. Dispersionsmitteln, eventuell unter Verwendung von Tensiden als Netz- oder Emulgiermitteln und/oder von Dispergatoren, durch Verdünnen bereits vorbereiteter emulgierbarer Konzentrate mit Lösungs- bzw. Dispersionsmitteln usw.

Als feste Trägerstoffe kommen im wesentlichen in Frage: natürliche Mineralstoffe, wie Kreide, Dolomit, Kalkstein, Tonerden und Kieselsäure und deren Salze (beispielsweise Kieselgur, Kaolin, Bentonit, Talkum, Attapulgit und Montmorrillonit); synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Pulver oder als Granulate vorliegen können.

Als Lösungs- bzw. Dispersionsmittel kommen im wesentlichen in Frage: Aromaten, wie Benzol, Toluol, Xylole und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungs- bzw. Dispersionsmittel, wie Dimethylformamid, N-Methylpyrrolidon und Dimethylsulfoxid, wobei solche Lösungsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Liegt das erfindungsgemässe Unkrautbekämpfungsmittel in Form einer Druckgaspackung vor, so wird zweckmässigerweise zusätzlich zum Treibgas ein Lösungsmittel verwendet.

Die Tenside (Netz- und Emulgiermittel) können nicht-ionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen sein, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzolsulfonat, und Butylnaphthalinsulfonatee; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium- und Ammoniumsalze von Ligninsulfonsäuren, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide; Antioxidantien, z.B. Gallussäureester und Butylhydroxytoluol; UV-Absorber, z.B. substituierte Benzophenone, Diphenylacrylonitrilsäureester und Zimtsäureester: und Deaktivatoren, z.B. Salze der Aethylendiaminotetraessigsäure und Polyglykole.

Die erfindungsgemässen Unkrautbekämpfungsmittel können zusätzlich zu den erfindungsgemässen Wirkstoffen Synergisten und andere Wirkstoffe, z.B. Insektizide, Akarizide, Fungizide, Pflanzenwachstumsregulatoren und Düngemittel, enthalten. Solche Kombinationsmittel eignen sich zur Verstärkung der Aktivität bzw. zur Verbreiterung des Wirkungsspektrums.

Die erfindungsgemässen Unkrautbekämpfungsmittel enthalten im allgemeinen zwischen 0,001 und 95 Gewichtsprozent, vorzugsweise zwischen 0,5 und 75 Gewichtsprozent einer bzw. mehrerer erfindungsgemässer Verbindungen als Wirkstoff(e). Sie können z.B. in einer Form vorliegen, die sich für die Lagerung und den Transport eignet. In solchen Formulierungen, z.B. emulgierbaren Konzentraten, ist die Wirkstoffkonzentration normalerweise im höheren Bereich, vorzugsweise zwischen 1 und 50 Gewichtsprozent, insbesondere zwischen 10 und 30 Gewichtsprozent. Diese Formulierungen können dann, z.B. mit gleichen oder verschiedenen inerten Stoffen, bis zu Wirkstoffkonzentrationen verdünnt werden, die sich für den praktischen Gebrauch eignen, also vorzugsweise ca. 0,001 bis 10 Gewichtsprozent, insbesondere ca. 0,005 bis 5 Gewichtsprozent. Die Wirkstoffkonzentrationen können jedoch auch kleiner oder grösser sein.

Wie oben erwähnt, kann die Herstellung der erfindungsgemässen Unkrautbekämpfungsmittel in an sich bekannter Weise durchgeführt werden.

Zur Herstellung pulverförmiger Präparate kann der Wirkstoff, d.h. mindestens eine erfindungsgemässe Verbindung, mit festem Trägerstoff vermischt werden, z.B. durch Zusammenmahlen; oder man kann den festen Trägerstoff mit einer Lösung oder Suspension des Wirkstoffes imprägnieren und dann das Lösungs- bzw. Dispersionsmittel durch Abdunsten, Erhitzen oder Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Der Wirkstoff kann auch mit einem Tensid und einem festen Trägerstoff zur Bildung eines netzbaren Pulvers vermischt werden, welches in Wasser dispergierbar ist, oder er kann mit einem festen vorgranulierten Trägerstoff zur Bildung eines granulatförmigen Produktes vermischt werden.

Wenn gewünscht, kann der Wirkstoff in einem mit Wasser nicht mischbaren Lösungsmittel, wie beispielsweise einem hochsiedenden Kohlenwasserstoff, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Unkrautbekämpfungsmittel, die einen weiteren Gegenstand der vorliegenden Erfindung bildet, kann nach üblichen Applikationsmethoden, wie Spritzen, Sprühen Stäuben, Giessen oder Streuen, erfolgen. Das erfindungsgemässe Verfahren zur Bekämpfung von Unkräutern ist dadurch gekennzeichnet, dass man das gegen Unkräuter zu schützende Gut und/oder die Unkräuter mit einer erfindungsgemässen Verbindung bzw. mit einem erfindungsgemässen Unkrautbekämpfungsmittel behandelt.

Die nachfolgenden Beispiele dienen zur näheren Erläuterung der Erfindung.

I. Herstellung der Verbindungen der Formel I:

Beispiel 1

3,0 g 2-Chlor-5-[3,6-dihydro-2,6-dioxo-3-methyl-4-trifluormethyl -1(2H)-pyrimidinyl]-4-fluorbenzoesäure werden in 20 ml Benzol und 2,9 ml Thionylchlorid zusammen mit 2 Tropfen Dimethylformamid 3 Stunden auf Rückflusstemperatur erhitzt. Anschliessend wird das Reaktionsgemisch zur Trockene eingedampft und in 20 ml Dioxan gelöst. Diese Lösung, die hauptsächlich aus dem Säurechlorid der obenerwähnten Benzoesäure und dem Lösungsmittel besteht, wird zu einer Lösung von 0,86 g 3-Thietanol und 1,0 g Pyridin in 10 ml Dioxan getropft. Dann wird das Reaktionsgemisch 2,5 Stunden bei Raumtemperatur gerührt, mit 400 ml Wasser versetzt und zweimal mit je 400 ml Aethylacetat extrahiert. Man wäscht die vereinigten organischen Phasen zweimal mit je 200 ml 1n Salzsäure und einmal mit 200 ml gesättigter Natriumchloridlösung, trocknet sie über wasserfreiem Natriumsulfat und dampft sie ein. Der Rückstand wird an Kieselgel mit Aethylacetat/n-Hexan (2:3) chromatographisch gereinigt. Auf diese Weise erhält man den 2-Chlor-5-[3,6-dihydro-2,6-dioxo-3-methyl-4-trifluormethyl -1(2H)-pyrimidinyl]-4-fluorbenzoesäure-(thietan-3-yl)-ester, der sich aus Aethylacetat/n-Hexan umkristallisieren lässt: Smp. 121-123 ° C.

In analoger Weise erhält man ausgehend von 2-Chlor-5-[3,6-dihydro-2,6-dioxo-3-methyl-4-trifluormethyl -1(2H)-pyrimidinyl]-4-fluorbenzoesäure über deren Säurechlorid und:

- Cycloheptanol den 2-Chlor-5-[3,6-dihydro-2,6-dioxo-3-methyl-4-trifluormethyl -1(2H)-pyrimidinyl]-4-fluorbenzoesäure-cycloheptylester als Oel, $^1$H-NMR($D_6$-DMSO, 400 MHz): 1,40-1,82 (m,10H), 1,94-2,04 (m,2H), 3,41 (s,3H), 5,07-5,16 (m,1H), 6,61 (s,1H), 7,87 (d,1H), 8,00 (d,1H);

- 3-Hydroxytetrahydrofuran den 2-Chlor-5-[3,6-dihydro-2,6-dioxo-3-methyl-4-trifluormethyl -1(2H)-pyrimidinyl]-4-fluorbenzoesäure -(tetrahydrofuran-3-yl)ester als Oel, $^1$H-NMR($D_6$-DMSO, 400 MHz): 2,01-2,10 (m,1H), 2,20-2,31 (m,1H), 3,42 (s,3H), 3,73-3,80 (m,1H), 3,82-3,91 (m,3H), 5,48-5,53 (m,1H), 6,62 (s,1H), 7,89 (d,1H), 8,07 (d,1H);
- 5-Hydroxy-1,3-dioxan den 2-Chlor-5-[3,6-dihydro-2,6-dioxo-3-methyl-4-trifluormethyl -1(2H)-pyrimidinyl]-4-fluorbenzoesäure-(1,3-dioxan-5-yl)ester als Oel, $^1$H-NMR($D_6$-DMSO, 400 MHz): 3,42 (s,3H), 3,97-4,02 (m,2H), 4,05-4,11 (m,2H), 4,78 (d,1H), 4,88-4,93 (m,2H), 6,62 (s,1H), 7,92 (d,1H), 8,08 (d,1H);
- Cyclopropylmethanol den 2-Chlor-5-[3,6-dihydro-2,6-dioxo-3-methyl-4-trifluormethyl -1(2H)-pyrimidinyl]-4-fluorbenzoesäure-cyclopropylmethylester als Oel, $^1$H-NMR($D_6$-DMSO, 400 MHz): 0,34-0,39 (m,2H), 0,54-0,60 (m,2H), 1,16-1,24 (m,1H), 3,42 (s,3H), 4,15 (d,2H), 6,61 (s,1H), 7,90 (d,1H), 8,05 (d,1H);
- 3-Hydroxymethyl-3-methyl-oxetan den 2-Chlor-5-[3,6-dihydro-2,6-dioxo-3-methyl-4-trifluormethyl -1-(2H)-pyrimidinyl]-4-fluorbenzoesäure-(3-methyl -3-oxetanyl)methylester als Oel, $^1$H-NMR($D_6$-DMSO, 400 MHz): 1,35 (s,3H), 3,42 (s,3H), 4,30 (d,2H), 4,44 (s,2H), 4,48 (d,2H), 6,62 (s,1H), 7,90 (d,1H), 8,07 (d,1H);
- 2-Tetrahydrofurylmethanol den 2-Chlor-5-[3,6-dihydro-2,6-dioxo-3-methyl-4-trifluormethyl 1(2H)-pyrimidinyl]-4-fluorbenzoesäure-tetrahydrofurfurylester als Oel, $^1$H-NMR($D_6$-DMSO, 400 MHz): 1,59-1,70 (m,1H), 1,76-1,90 (m,2H), 1,93-2,04 (m,1H), 3,42 (s,3H), 3,62-3,69 (m,1H), 3,72-3,80 (m,1H), 4,11-4,17 (m,1H), 4,21-4,33 (m,2H), 6,61 (s,1H), 7,90 (d,1H), 8,05 (d,1H);
- 4-Hydroxymethyl-1,3-dioxolan den 2-Chlor-5-[3,6-dihydro-2,6-dioxo-3-methyl-4-trifluormethyl -1(2H)-pyrimidinyl]-4-fluorbenzoesäure-(1,3-dioxolan -4-yl)methylester als Oel, $^1$H-NMR($D_6$-DMSO, 400 MHz): 3,42 (s,3H), 3,67-3,72 (m,1H), 3,96-4,04 (m,1H), 4,32-4,42 (m,3H), 4,83 (s,1H), 4,94 (s,1H), 6,62 (s,1H), 7,91 (d,1H), 8,08 (d,1H);
- Benzylalkohol den 2-Chlor-5-[3,6-dihydro-2,6-dioxo-3-methyl-4-trifluormethyl -1(2H)-pyrimidinyl]-4-fluorbenzoesäure-benzylester als Oel, $^1$H-NMR($D_6$-DMSO, 400 MHz): 3,40 (s,3H), 5,37 (s,2H), 6,60 (s,1H), 7,36-7,43 (m,3H), 7,47-7,50 (m,2H), 7,90 (d,1H), 8,08 (d,1H);
- R-4,4-Dimethyl-3-hydroxy-2-oxo-tetrahydrofuran den R-2-Chlor-5-[3,6-dihydro-2,6-dioxo-3-methyl-4-trifluormethyl -1(2H)-pyrimidinyl]-4-fluorbenzoesäure-(4,4-dimethyl -2-oxo-tetrahydrofuran-3-yl)ester als Oel, $[\alpha]_D^{20}$ = -10,6°; $^1$H-NMR($D_6$-DMSO, 400 MHz): 1,11 (s,3H), 1,22 (s,3H), 3,43 (s,3H), 4,13 (d,1H), 4,24 (d,1H), 5,89 (s,1H), 6,62 (d,1H), 7,96 (d,1H), 8,15 (d,1H);
- 4-Hydroxy-2,2,6,6-tetramethyl-tetrahydrothiopyran den 2-Chlor-5-[3,6-dihydro-2,6-dioxo-3-methyl-4-trifluormethyl -1(2H)-pyrimidinyl]-4-fluorbenzoesäure-(2,2,6,6-tetramethyl -tetrahydrothiopyran-4-yl)ester als Oel, $^1$H-NMR($D_6$-DMSO, 400 MHz): 1,25 (s,6H), 1,49 (s,6H), 1,61 (t,2H), 2,19 (dd,2H), 3,42 (s,3H), 5,31-5,38 (m,1H), 6,62 (s,1H), 7,89 (d,1H), 8,06 (d,1H);
- Cyclopentanol den 2-Chlor-5-[3,6-dihydro-2,6-dioxo-3-methyl-4-trifluormethyl -1(2H)-pyrimidinyl]-4-fluorbenzoesäure-cyclopentylester als Oel, $^1$H-NMR($D_6$-DMSO, 400 MHz): 1,50-2,00 (m,8H), 3,42 (s,3H), 5,31-5,38 (m,1H), 6,61 (s,1H), 7,87 (d,1H), 8,02 (d,1H);
- Phenol den 2-Chlor-5-[3,6-dihydro-2,6-dioxo-3-methyl-4-trifluormethyl -1(2H)-pyrimidinyl]-4-fluorbenzoesäure-phenylester als Oel, $^1$H-NMR($D_6$-DMSO, 400 MHz): 3,43 (s,3H), 6,63 (s,1H), 7,30-7,37 (m,3H), 7,45-7,53 (m,2H), 7,99 (d,1H), 8,39 (d,1H);
- 2-Cyclohexyläthanol den 2-Chlor-5-[3,6-dihydro-2,6-dioxo-3-methyl-4-trifluormethyl -1(2H)-pyrimidinyl]-4--fluorbenzoesäure -(2-cyclohexyläthyl)ester als Oel, $^1$H-NMR($D_6$-DMSO, 400 MHz): 0,87-0,98 (m,2H), 1,10-1,25 (m,3H), 1,32-1,45 (m,1H), 1,56-1,75 (m,7H), 3,42 (s,3H), 4,34 (t,2H), 6,61 (s,1H), 7,89 (d,1H), 8,03 (d,1H);
- 1-Cyclopropyläthanol den 2-Chlor-5-[3,6-dihydro-2,6-dioxo-3-methyl-4-trifluormethyl -1(2H)-pyrimidinyl]-4-fluorbenzoesäure -(1-cyclopropyläthyl)ester als Oel, $^1$H-NMR($D_6$-DMSO, 400 MHz): 0,34-0,45 (m,2H), 0,47-0,60 (m,2H), 1,08-1,18 (m,1H), 1,38 (d,3H), 3,43 (s.3H), 4,55 (m,1H), 6,61 (s,1H), 7,88 (d,1H), 8,01 (d,1H), Massenspektrym (m/e): $M^+$ 434(4);
- p-Methoxyphenol den 2-Chlor-5-[3,6-dihydro-2,6-dioxo-3-methyl-4-trifluormethyl -1(2H)-pyrimidinyl]-4-fluorbenzoesäure -(p-methoxyphenyl)ester, Smp. 128-129°C, Massenspektrum (m/e): $M^+$ 472(24);
- 5-Hydroxy-1,3-benzodioxol den 2-Chlor-5-[3,6-dihydro-2,6-dioxo-3-methyl-4-trifluormethyl -1(2H)-pyrimidinyl]-4-fluorbenzoesäure -(1,3-benzodioxol-5-yl)ester, Smp. 134-135°C, Massenspektrum (m/e): $M^+$ 486(15);
- 2,4-Dichlorbenzylalkohol den 2-Chlor-5-[3,6-dihydro-2,6-dioxo-3-methyl-4-trifluormethyl-1(2H)-pyrimidinyl]-4-fluorbenzoesäure-2,4-dichlorbenzylester, Smp. 88-90°C;
- 3-Nitrobenzylalkohol den 2-Chlor-5-[3,6-dihydro-2,6-dioxo-3-methyl-4-trifluormethyl-1(2H)-pyrimidinyl]-4-fluorbenzoesäure-(3-nitrophenyl)ester, $^1$H-NMR ($D_6$-DMSO, 400 MHz): 3,41 (s, 3H), 5,52 (s, 2H),

6,60 (s,1H), 7,70 - 7,74 (m,1H), 7,92 (d,1H), 7,95 - 7,98 (m,1H), 8,12 (d,1H), 8,20 - 8,26 (m,1H), 8,37 - 8,39 (m, 1H) Massenspektrum (m/e): $M^+$ 501 (3);

- Cyclohexanol den 2-Chlor-5-[3,6-dihydro-2,6-dioxo-3-methyl-4-trifluormethyl-1(2H)-pyrimidinyl]-4-fluorbenzoesäure-cyclohexylester, $^1$H-NMR ($D_6$-DMSO, 400 MHz): 1,27 - 1,59 (m,6H), 1,66 - 1,76 (m,2H), 1,86 - 1,96 (m,2H), 3,39 (s, 3H), 4,93 - 5,00 (m, 1H), 6,61 (s, 1H), 7,87 (d, 1H), 8,01 (d, 1H); Massenspektrum: (m/e): $M^+$ 448 (1);
- 3,5-Bis-(trifluormethyl)benzylalkohol den 2-Chlor-5- [3,6-dihydro-2,6-dioxo-3-methyl-4-trifluormethyl-1-(2H)-pyrimidinyl]-4-fluorbenzoesäure-[3,5 - bis (trifluormethyl)phenyl]ester, Smp. 129 - 130 °C;
- p-Cyanophenol den 2-Chlor-5-[3,6-dihydro-2,6-dioxo-3-methyl-4-trifluormethyl-1(2H)-pyrimidinyl]-4-fluorbenzoesäure-(p-cyanophenyl)ester, $^1$H-NMR ($D_6$-DMSO, 400 MHz): 3,43 (s,3H), 6,63 (s,1H), 7,57 - 7,61 (m,2H), 7,98 - 8,02 (m,3H), 8,42 (d,1H);
- Tetrahydrothiophen-3-ol den 2-Chlor-5-[3,6-dihydro-2,6-dioxo-3-methyl-4-trifluormethyl-1(2H)-pyrimidinyl]-4-fluorbenzoesäure-(tetrahydrothien-3-yl)ester, $^1$H-NMR ($D_6$-DMSO, 400 MHz): 1,98 - 2,09 (m,1H), 2,28 - 2,38 (m,1H), 2,91 - 3,00 (m,3H), 3,18 - 3,24 (m,1H), 3,42 (s,3H), 5,66 - 5.73 (m,1H), 6,62 (s,1H), 7,89 (d,1H), 8,03 (d,1H);
- Tetrahydropyran-4-ol den 2-Chlor-5-[3,6-dihydro-2,6-dioxo-3-methyl-4-trifluormethyl-1(2H)-pyrimidinyl]-4-fluorbenzoesäure-(tetrahydropyran-3-yl)ester, Smp. 173-174°C.
- 2,2-Dimethyl-4-hydroxymethyl-1,3-dioxolan den 2-Chlor-5-[3,6-dihydro-2,6-dioxo-3-methyl-4-trifluormethyl-1(2H)-pyrimidinyl]-4-fluorbenzoesäure-(2,2-dimethyl-1,3-dioxolan-4-yl)methylester $^1$H-NMR ($D_6$-DMSO, 400 MHz): 1,28 (s,3H), 1,33 (s,3H), 3,42 (s,3H), 3,75 - 3,80 (m,1H), 4,02 - 4,10 (m,1H), 4,25 - 4,43 (m,3H), 6,61 (s,1H), 7,90 (d,1H), 8,07 (d,1H);

In analoger Weise erhält man ausgehend von 2-Chlor-5-[3,6-dihydro-2,6-dioxo-3-methyl-4-trifluormethyl-1(2H)-pyrimidinyl]-benzoesäure über deren Säurechlorid und 1-Cyclopropyläthanol den 2-Chlor-5-[3,6-dihydro-2,6-dioxo-3-methyl-4-trifluormethyl-1(2H)-pyrimidinyl]-benzoesäure[1-cyclopropyläthyl]ester, Smp. 118 - 119 °C;

In analoger Weise erhält man ausgehend von 2-Chlor-5-[3,6-dihydro-2,6-dioxo-3-methyl-4-pentafluoräthyl-1 (2H)-pyrimidinyl]-4-fluorbenzoesäure über deren Säurechlorid und Tetrahydrofuran-3-ol den 2-Chlor-5-[3,6-dihydro-2,6-dioxo-3-methyl-4-pentafluoräthyl-1(2H)-pyrimidinyl]-4-fluorbenzoesäure-(tetrahydrofuran-3-yl)ester, $^1$H-NMR ($D_6$-DMSO, 400 MHz): 2,00 - 2,09 (m, 1H), 2,20 - 2,32 (m,1H), 3,43 (s,3H), 3,74 - 3,92 (m,4H), 5,48 - 5,56 (m,1H), 6,50 (s,1H), 7,89 (d,1H), 8,08 (d,1H);

In analoger Weise erhält man ausgehend von 2-Chlor-5-[3,6-dihydro-3,4-dimethyl-2,6-dioxo-1(2H)-pyrimidinyl]-4-fluorbenzoesäure über deren Säurechlorid und

- Hydroxymethylcyclopropan den 2-Chlor-5-[3,6-dihydro-3,4- dimethyl-2,6-dioxo-1(2H)-pyrimidinyl]-4-fluorbenzoesäure-(cyclopropylmethyl)ester, $^1$H-NMR ($CDCl_3$, 400 MHz): 0.31 - 0,38 (m,2H), 0,57 - 0,64 (m,2H), 1,18 - 1,27 (m,1H), 2,32 (s,3H), 3,45 (s,3H), 4,11 - 4,15 (m,2H), 5,74 (s,1H), 7,35 (d,1H), 7,89 (d,1H);
  Massenspektrum (m/e): $M^+$ 366 (13);
- 3-Hydroxytetrahydrofuran den 2-Chlor-5-[3,6-dihydro-3,4-dimethyl-2,6-dioxo-1(2H)-pyrimidinyl]-4-fluorbenzoesäure-(tetrahydrofuran-3-yl)ester, Smp. 149 - 151 °C;
- 1-Cyclopropyläthanol den 2-Chlor-5-[3,6-dihydro-3,4-dimethyl-2,6-dioxo-1(2H)-pyrimidinyl]-4-fluorbenzoesäure-(1-cyclopropyläthyl)ester als Oel, $^1$H-NMR ($D_6$-DMSO, 400 MHz): 0,33 - 0,43 (m,2H), 0,47 - 0,58 (m,2H), 1,07 - 1,17 (m,1H), 1,37 (d,3H), 2,33 (s,3H), 3,35 (s,3H), 4,49 - 4,57 (m,,1H), 5,81 (s,1H), 7,82 (d,1H), 7,91 (d,1H);
  Massenspektrum (m/e): $M^+$ 380 (8);
- Cyclopentanol den 2-Chlor-5-[3,6-dihydro-3,4-dimethyl-2,6-dioxo-1(2H)-pyrimidinyl]-4-fluorbenzoesäure-cyclopentylester als Oel, $^1$H-NMR ($D_6$-DMSO, 400 MHz): 1,58 - 1,97 (m,8H), 2,33 (s,3H), 3,35 (s,3H), 5,31 - 5,36 (m,1H), 5,80 (s,1H), 7,81 (d,1H), 7,91 (d,1H);
  Massenspektrum (m/e): $M^+$ 380 (19);

## Beispiel 2

3,4 g 2-Chlor-4-fluor-5-[2-methoxy-6-oxo-4-trifluormethyl -1(6H)-pyrimidinyl]-benzoesäure werden in 25 ml Benzol und 3,4 ml Thionylchlorid zusammen mit 2 Tropfen Dimethylformamid 3 Stunden auf Rückflusstemperatur erhitzt. Anschliessend wird das Reaktionsgemisch zur Trockene eingedampft und in 20 ml Dioxan gelöst. Diese Lösung, die hauptsächlich aus dem Säurechlorid der obenerwähnten Benzoesäure und dem Lösungsmittel besteht, wird zu einer Lösung von 1,0 g 3-Thietanol und 1,3 g Pyridin in 20 ml Dioxan getropft. Dann wird das Reaktionsgemisch 3,5 Stunden bei Raumtemperatur gerührt, mit 400 ml Wasser versetzt und zweimal mit je 400 ml Aethylacetat extrahiert. Man wäscht die vereinigten organischen

Phasen zweimal mit je 200 ml 1n Salzsäure und einmal mit 200 ml gesättigter Natriumchloridlösung, trocknet sie über wasserfreiem Magnesiumsulfat und dampft sie ein. Der Rückstand wird an Kieselgel mit Diäthyläther/n-Hexan (1:2) chromatographisch gereinigt. Auf diese Weise erhält man den 2-Chlor-4-fluor-5-[2-methoxy-6-oxo-4-trifluormethyl -1(6H)-pyrimidinyl]-benzoesäure-(thietan-3-yl)ester als farbloses Oel, $^1$H-NMR($CDCl_3$, 400 MHz): 3,37-3,44 (m,2H), 3,57-3,66 (m,2H), 4,03 (s,3H), 5,85 (Quintett, 1H), 6,63 (s,1H), 7,42 (d,1H), 7,88 (d,1H).

In analoger Weise erhält man aus 2-Chlor-4-fluor-5-[2-methoxy-6-oxo-4-trifluormethyl-1(6H)-pyrimidinyl]-benzoesäure über deren Säurechlorid und Tetrahydrofuran-3-ol den 2-Chlor-4-fluor-5-[2-methoxy-6-oxo-4-trifluormethyl-1(6H)-pyrimidinyl]-benzoesäure-(tetrahydrofuran-3-yl)ester, $^1$H-NMR ($D_6$-DMSO, 400 MHz): 2,02 - 2,10 (m,1H), 2,20 - 2,30 (m,1H), 3,75 - 3,92 (m,4H), 3,96 (s,3H), 5,48 - 5,55 (m,1H), 6,86 (s,1H), 7,95 (d,1H), 8,25 (d,1H), Massenspektrum (m/e): $M^+$ 436 (2).

Beispiel 3

3,0 g 2-Chlor-4-fluor-5-[3,6-dihydro-2,6-dioxo-3-methyl -4-trifluormethyl-1(2H)-pyrimidiny]-4-fluorbenzoesäure -Kaliumsalz und 0,6 ml Epichlorhydrin werden in 25 ml Dimethylformamid 3 Stunden auf 70 °C erwärmt. Danach wird die Reaktionslösung auf 500 ml Wasser gegeben und mit wenig gesättigter Kaliumcarbonatlösung auf pH 10 bis 11 eingestellt. Es wird zweimal mit 300 ml Aethylacetat extrahiert. Die organischen Phasen werden zweimal mit 200 ml Wasser gewaschen, über wasserfreiem Magnesiumsulfat getrocknet und eingeengt. Der Rückstand wird an Kieselgel mit Aethylacetat/n-Hexan chromatographisch gereinigt. Man erhält den 2-Chlor-5-[3,6-dihydro-2,6-dioxo-3-methyl-4-trifluormethyl -1(2H)-pyrimidinyl]-4-fluorbenzoesäure -(2,3-epoxypropyl)ester als hellgelbes Oel, $^1$H-NMR($D_6$-DMSO, 400 MHz): 2,73 (m,1H), 2,85 (m,1H), 3,32 (m,1H), 3,42 (s,3H), 4,10 (m,1H), 4,69 (m,1H), 6,62 (s,1H), 7,92 (d,1H), 8,11 (d,1H), Massenspektrum (m/e): $M^+$ 422(16).

Beispiel 4

In eine Suspension von 145,0 g 2-Chlor-4-fluor-5-[3,6-dihydro-2,6-dioxo-4-methyl-1(2H) - pyrimidinyl]-benzoesäure-isopropylester und 59,0 g wasserfreiem, fein pulverisiertem Kaliumcarbonat in 1 l Dimethylformamid werden unter Rühren bei 80 °C während 6 Stunden 103 g Chlordifluormethan eingeleitet. Nach Abkühlen wird der feste Anteil abgenutscht und mit 100 ml Dimethylformamid nachgewaschen. Das Filtrat wird unter vermindertem Druck bei 55 °C weitgehend eingeengt, der Rückstand auf 2 l Wasser gegossen und das wässrige Gemisch mit konzentrierter Salzsäure auf pH 3 eingestellt. Das Gemisch wird mit 1,5 l Essigsäure-äthylester ausgeschüttelt und die organische Phase zweimal mit je 1 l Wasser gewaschen, über wasserfreiem Natriumsulfat getrocknet und unter vermindertem Druck zur Trockene eingedampft. Der harzige Rückstand wird an einer Kieselgel-Säule unter Verwendung von Essigsäure-äthylester/n-Hexan (1:1) als Laufmittel chromatographisch gereinigt. Das Produkt wird in 1500 ml heissem n-Hexan gelöst und die Lösung mit Kohle behandelt, filtriert und bei erhöhter Temperatur auf 700 ml eingeengt. Anschliessend wird geimpft und unter Rühren auf 5 °C abgekühlt. Die resultierenden Kristalle werden abgenutscht, mit n-Hexan gewaschen und getrocknet. Man erhält den 2-Chlor-5-[3-difluormethyl-3,6-dihydro-2,6-dioxo-4-methyl-1(2H)-pyrimidinyl]-4-fluorbenzoesäure-isopropylester, Smp. 90-93 °C.

Beispiele 5-8

Analog dem in Beispiel 4 beschriebenen Verfahren wird das entsprechende Uracilderivat der Formel II mit Chlordifluormethan alkyliert, um die in der nachstehenden Tabelle 1 aufgeführten Verbindungen der Formel I herzustellen.

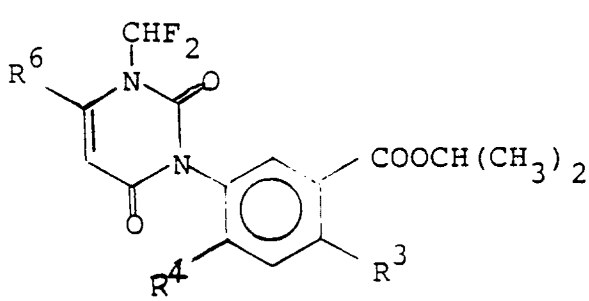

Tabelle 1

| Beispiel | $R^3$ | $R^4$ | $R^6$ | Physikalische Daten |
|---|---|---|---|---|
| 5 | Cl | F | $C_2H_5$ | $^1$H-NMR ($CDCl_3$, 400 MHz): 7,83 ppm (d,1H), 7,74 ppm (t,1H), 7,36 ppm (d,1H), 5,90 ppm (s,1H), 5,25 ppm (m,1H), 2,87 ppm (q,2H), 1,36 ppm (d,6H), 1,31 ppm (t,3H). |
| 6 | Br | F | $C_2H_5$ | $^1$H-NMR ($CDCl_3$, 400 MHz): 7,81 ppm (d,1H), 7,74 ppm (t,1H), 7,57 ppm (d,1H), 5,90 ppm (s,1H), 5,24 ppm (m,1H), 2,87 ppm (q,2H), 1,37 ppm (d,6H), 1,32 ppm (t,3H). |
| 7 | Br | F | $CH_3$ | $^1$H-NMR ($CDCl_3$, 400 MHz): 7,80 ppm (d,1H), 7,72 ppm (t,1H), 7,57 ppm (d,1H), 5,83 ppm (s,1H), 5,24 ppm (m,1H), 2,49 ppm (d,3H), 1,37 ppm (d,6H). |
| 8 | Cl | H | $CH_3$ | Smp. 115-117°C |

Beispiel 9

Eine Suspension von 2,00 g 2-Chlor-5-[3-difluormethyl-3,6-dihydro-2,6 -dioxo-4-methyl-1(2H)-pyrimidinyl]-4-fluorbenzoesäure, 1,63 g Methyljodid und 0,61 g Natriumcarbonat in 20 ml Aceton wird 4 Stunden beim Siedepunkt erhitzt. Anschliessend werden 1,63 g Methyljodid zugefügt und nach 2 Stunden weitere 1,63 g. Das Reaktionsgemisch wird weitere 16 Stunden bei Rückflusstemperatur erhitzt. Nach Abkühlen werden unlösliche Anteile abgenutscht und mit Aceton nachgewaschen, und das Filtrat wird unter vermindertem Druck zur Trockene eingedampft. Der Rückstand wird in 100 ml Essigsäure-äthylester gelöst und die Lösung zweimal mit je 100 ml Wasser gewaschen. Die organische Phase wird über wasserfreiem Natriumsulfat getrocknet und zur Trockene eingedampft. Der harzige Rückstand wird an einer Kieselgel-Säule unter Verwendung von Diäthyläther/n-Hexan (1:2) als Laufmittel chromatographisch gereinigt. Das Produkt wird aus Diäthyläther/n-Hexan umkristallisiert. Man erhält den 2-Chlor-5-[3-difluormethyl-3,6-dihydro-2,6-dioxo-4-methyl-1(2H)-pyrimidinyl]-4-fluorbenzoesäure-methylester, Smp. 121-123 ° C.

Beispiele 10-19

Analog dem in Beispiel 9 beschriebenen Verfahren wird 2-Chlor-5-[3-difluormethyl-3,6-dihydro-2,6-dioxo-4-methyl-1-(2H)-pyrimidinyl]-4-fluorbenzoesäure entsprechend verestert, um die in der nachstehenden Tabelle 2 aufgeführten Verbindungen der Formel I herzustellen.

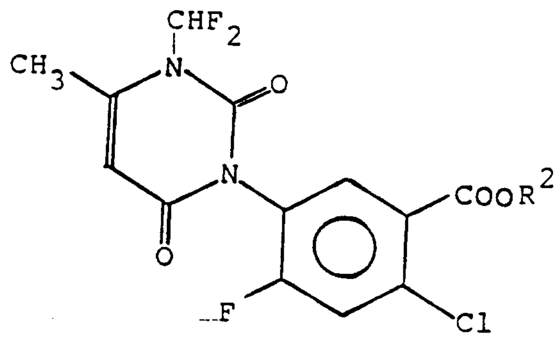

Tabelle 2

| Beispiel | $R^2$ | Physikalische Daten |
|---|---|---|
| 10 | $C_2H_5$ | Smp. 90-92° C |
| 11 | $nC_3H_7$ | $^1H$-NMR($CDCl_3$, 400 MHz): 1,01 (t,3H), 1,77 (m,2H), 2,49 (s,3H), 4,28 (t,2H), 5,83 (s,1H), 7,37 (d,1H), 7,72 (t,1H), 7,87 (d,1H). |
| 12 | $nC_4H_9$ | $^1H$-NMR ($CDCl_3$, 400 MHz): 0,96 (t,3H), 1,44 (m,2H), 1,74, (m,2H), 2,49 (s,3H), 4,32 (t,2H), 5,83 (s,1H), 7,36 (d,1H), 7,72 (t,1H), 7,86 (d,1H). |
| 13 | sek.$C_4H_9$ | $^1H$-NMR ($CDCl_3$, 400 MHz): 0,97 (t,3H), 1,34 (d,3H), 1,68 (m,2H), 2,50 (s,3H), 5,10 (m,1H), 5,83 (s,1H), 7,36 (d,1H), 7,72 (t,1H), 7,85 (d,1H). |
| 14 | iso$C_5H_{11}$ | $^1H$-NMR ($CDCl_3$, 400 MHz): 0,95 (d,6H), 1,64 (q,2H), 1,75 (m,1H), 2,45 (s,3H), 4,35 (t,2H), 5,83 (s,1H), 7,36 (d,1H), 7,72 (t,1H), 7,85 (d,1H). |
| 15 | $nC_6H_{13}$ | $^1H$-NMR ($CDCl_3$, 400 MHz): 0,89 (t,3H), 1,35 (m,6H), 1,75 (m,2H), 2,50 (s,3H), 4,31 (t,2H), 5,83 (s,1H), 7,36 (d,1H), 7,72 (t,1H), 7,86 (d,1H). |
| 16 | $nC_8H_{17}$ | $^1H$-NMR ($CDCl_3$, 400 MHz): 0,88 (t,3H), 1,35 (m,10H), 1,74 (m,2H), 2,50 (s,3H), 4,31 (t,2H), 5,83 (s,1H), 7,36 (d,1H), 7,72 (t,1H), 7,86 (d,1H). |
| 17 | iso$C_4H_9$ | $^1H$-NMR ($CDCl_3$, 400 MHz): 1,00 (d,6H), 2,07 (m,1H), 2,49 (s,3H), 4,11 (d,2H), 5,83 (s,1H), 7,37 (d,1H), 7,72 (t,1H), 7,87 (d,1H). |

| | | |
|---|---|---|
| 18 | $CH_2CH{=}CH_2$ | $^1$H-NMR ($CDCl_3$, 400 MHz): 2,49 (s,3H), 4,81 (m,2H), 5,30 (m,1H), 5,39 (m,1H), 5,83 (s,1H), 6,00 (m,1H), 7,38 (d,1H), 7,71 (t,1H), 7,90 (d,1H). |
| 19 | $CH_2OCH_3$ | $^1$H-NMR ($CDCl_3$, 400 MHz): 2,50 (s,3H), 3,54 (s,3H), 5,47 (s,2H), 5,83 (s,1H), 7,39 (d,1H), 7,72 (t,1H), 7,94 (d,1H). |

Beispiel 20

Eine Suspension von 2,00 g 2-Chlor-5-[3-difluormethyl-3,6-dihydro-2,6 -dioxo-4-methyl-1(2H)-pyrimidinyl]-4-fluorbenzoesäure und 2,05 g Thionylchlorid in 25 ml Benzol wird 20 Stunden bei Rückflusstemperatur erhitzt. Die klare Lösung wird unter vermindertem Druck bei 50°C zur Trockene eingedampft, der Rückstand in 20 ml Methylenchlorid gelöst und anschliessend mit 5 ml Propargylalkohol und 0,55 g Pyridin 3,5 Stunden bei Raumtemperatur gerührt. Das Reaktionsgemisch wird unter vermindertem Druck zur Trockene eingedampft, der Rückstand in 100 ml Essigsäure-äthylester gelöst und die Lösung dreimal mit je 100 ml Wasser ausgeschüttelt. Die organische Phase wird über wasserfreiem Natriumsulfat getrocknet und zur Trockene eingedampft. Der harzige Rückstand wird an einer Kieselgel-Säule unter Verwendung von Diäthyläther/n-Hexan (1:1) als Laufmittel chromatographisch gereinigt. Man erhält den 2-Chlor-5-[3-difluormethyl-3,6-dihydro-2,6 -dioxo-4-methyl-1(2H)-pyrimidinyl]-4-fluorbenzoesäure-propargylester. $^1$H-NMR ($CDCl_3$, 400 MHz): 7,94 ppm (d,1H), 7,71 ppm (t,1H), 7,39 ppm (d,1H), 5,83 ppm (s,1H), 4,91 ppm (d,2H), 2,52 ppm (t,1H), 2,50 ppm (m,3H).

Beispiele 21 - 25

Analog dem in Beispiel 20 beschriebenen Verfahren wird 2-Chlor-5-[3-difluormethyl-3,6-dihydro-2,6-dioxo-4-methyl-1(2H)-pyrimidinyl]-4-fluorbenzoesäure entsprechend verestert, um die in der nachstehenden Tabelle 3 aufgeführten Verbindungen der Formel I herzustellen.

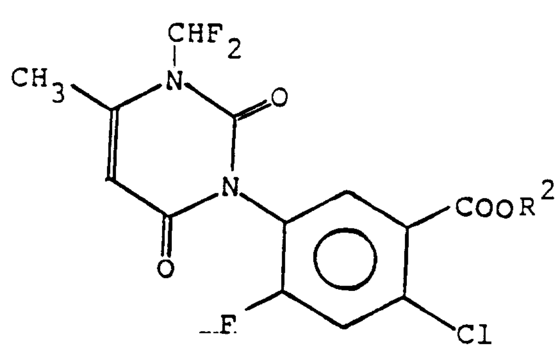

Tabelle 3

| Beispiel | $R^2$ | Physikalische Daten |
|---|---|---|
| 21 | $C(CH_3)_2CH=CH_2$ | $^1H$-NMR($CDCl_3$, 400 MHz): 1,65 (s,6H); 2,49 (s,3H), 5,15 (d,1H), 5,26 (d,1H), 5,83 (s,1H), 6,18 (m,1H), 7,34 (d,1H), 7,72 (t,1H), 7,78 (d,1H). |
| 22 | neo $C_5H_{11}$ | $^1H$-NMR($CDCl_3$, 400 MHz): 1,02 (s,9H), 2,50 (s,3H), 4,03 (s,2H), 5,83 (s,1H), 7,37 (d,1H), 7,72 (t,1H), 7,87 (d,1H). |
| 23 | $(CH_2)_2C(CH_3)(=CH_2)$ | $^1H$-NMR($CDCl_3$, 400 MHz): 1,79 (s,3H), 2,46 (m,5H), 4,43 (t,2H), 4,78 (s,1H), 4,83 (s,1H), 5,83 (s,1H), 7,36 (d,1H), 7,72 (t,1H) 7,85 (d,1H). |
| 24 | tert. $C_4H_9$ | $^1H$-NMR($CDCl_3$, 400 MHz): 1,58 (s,9H), 2,49 (s,3H), 5,83 (s,1H), 7,33 (d,1H), 7,72 (t,1H), 7,76 (d,1H). |

| | | |
|---|---|---|
| 25 | Cyclopentyl | $^1$H-NMR($D_6$-DMSO, 400 MHz): 1,55-1,82 (m,6H), 1,90-2,00 (m,2H), 2,44 (s,3H), 5,35 (m,1H), 6,08 (s,1H), 7,84 (d,1H), 7,90 (t,1H), 8,08 (d,1H). |

Beispiel 26

Eine 55-60%-ige Suspension von 2,9g Natriumhydrid in Weissöl wird in 30ml Dimethylformamid und 2 ml Toluol vorgelegt, und das Gemisch wird unter Rühren auf 8-0 ° C abgekühlt. Es wird eine Lösung von 12 g 3-Amino-4,4,4-trifluorcarbonsäure-äthylester in 30ml Dimethylformamid zugetropft. Nach beendeter Zugabe rührt man das Gemisch 1 Stunde bei 5 ° C. Anschliessend kühlt man das Gemisch auf eine Innentemperatur von -65 ° C ab und tropft eine Lösung von 10g 2-Chlor-4-fluor-5-isocyanatobenzoesäure-cyclopentylest in 30ml Toluol zu. Das Reaktionsgemisch wird noch 2,5 Stunden bei -65 ° C nachgerührt. Danach tropft man 30 ml Cyclopentanol zu und lässt das Reaktionsgemisch sich auf Raumtemperatur aufwärmen. Es wird mit 10ml Eisessig angesäuert und auf 500 ml Wasser gegeben und die resultierende wässrige Lösung zweimal mit je 250 ml Aethylacetat extrahiert. Die organischen Phasen werden vereinigt, je einmal mit 200 ml Wasser und 200ml gesättigter Natriumchloridlösung gewaschen, über wasserfreiem Magnesiumsulfat getrocknet und unter vermindertem Druck eingeengt.Man reinigt den Rückstand chromatisch an einer Kieselgel-Säule unter Verwendung von Aethylacetat/n-Hexan (1:3) als Laufmittel und erhält auf diese Weise den 2-Chlor-5-[3,6-dihydro-2,6-dioxo-4-trifluormethyl-1(2H)pyrimidinyl]-4-fluorbenzoesäure-cyclopentylester als farbloses Oel. $^1$H-NMR($CDCl_3$, 400MHz): 1,50-2,05 (m,8H), 5,34-5,48 (m,1H), 6,23 (s,1H), 7,36 (d,1H), 7,85 (d,1H); Massenspektrum (m/e): $M^+$ 420 (1).

Beispiel 27

4,5g 2-Chlor-5-[3,6-dihydro-2,6-dioxo-4-trifluormethyl -1(2H)-pyrimidinyl]-4-fluorbenzoesäure-cyclopentylester, 2,1g Kaliumhydrogencarbonat und 1,5 ml Diäthylsulfat werden in 50ml Aceton 4 Stunden bei Rückflusstemperatur erhitzt. Anschliessend wird das Lösungsmittel abdestilliert und der Rückstand auf 150 ml Diäthyläther und 150 ml Wasser verteilt. Man extrahiert die wässrige Phase mit weiteren 150 ml Diäthyläther und wäscht die vereinigten organischen Phasen zweimal mit je 150 ml Wasser, trocknet sie über wasserfreiem Magnesiumsulfat und engt sie ein. Der Rückstand wird an einer Kieselgel-Säule unter Verwendung von Aethylacetat/n-Hexan (1:4) als Laufmittel chromatographisch gereinigt. Man erhält auf diese Weise den 2-Chlor-5-[3-äthyl-3,6-dihydro-2,6-dioxo-4-trifluormethyl-1(2H)-pyrimidinyl]-4-fluorbenzoesäure-cyclopentylester als farbloses Oel, $^1$H-NMR ($CDCl_3$, 400 MHz): 1,36 (t,3H), 1,56-2,04 (m,8H), 4,02 (q,2H), 5,36-5,45 (m,1H), 6,35 (s,1H), 7,36 (d,1H), 7,82 (d,1H); Massenspektrum (m/e): $M^+$ 448(1), sowie den 2-Chlor-5-[2-äthoxy-6-oxo-4-trifluormethyl-1(6H)-pyrimidinyl]-4-fluorbenzoesäure-cyclopentylester, Smp. 90-91 ° C.

Beispiel 28

Eine Lösung von 18,0 g 2-Chlor-5-[3-difluormethyl-3,6-dihydro-4-methyl-2,6-dioxo -1(2H)-pyrimidinyl]-4-fluorbenzoesäure-isopropylester in 60 ml Methylenchlorid wird mit 100 ml konzentrierter Schwefelsäure 5 Minuten intensiv gerührt und anschliessend auf 1 kg Eis gegossen. Die resultierenden Kristalle werden abgenutscht, zweimal mit je 50 ml Wasser gewaschen und in 300 ml Methanol gelöst. Die Mutterlauge wird zweimal mit je 100 ml Methylenchlorid ausgeschüttelt und die organische Phase mit Wasser neutral gewaschen und mit der methanolischen Lösung vereinigt. Diese Lösung wird über wasserfreiem Natriumsulfat getrocknet und zur Trockene eingedampft. Der kristalline Rückstand wird mit 100 ml Essigsäureäthylester bei 70 ° C aufgeschlämmt und auf Raumtemperatur abgekühlt, und die Kristalle werden abgenutscht und mit Diäthyläther gewaschen. Man erhält die 2-Chlor-5-[3-difluormethyl-3,6-dihydro-4-methyl-2,6 -dioxo-1(2H)-pyrimidinyl]-4-fluorobenzoesäure, Smp. 247-248 ° C.

II. Herstellung der Benzoesäuren III und von deren Enoläthern:

Beispiel 29

Eine Lösung von 5 g 2-Chlor-4-fluor-5-[2-methoxy-6-oxo-4-trifluormethyl-1(6H)-pyrimidinyl]-benzoesäure-isopropylester in 20 ml Methylenchlorid wird unter Rühren und Kühlen bei 20-25°C mit 25 ml konzentrierter Schwefelsäure versetzt. Das Reaktionsgemisch wird 20 Minuten bei Raumtemperatur nachgerührt und auf 100 g Eis gegossen. Die organischen Phase wird abgetrennt, die wässrige Phase zweimal mit je 15 ml Aethylacetat ausgeschüttelt und die vereinigten organischen Phasen mit Wasser neutral gewaschen. Anschliessend wird die Lösung über wasserfreiem Natriumsulfat getrocknet und unter vermindertem Druck zur Trockne eingedampft. Der harzige Rückstand wird aus Diäthyläther/n-Hexan umkristallisiert. Man erhält die 2-Chlor-4-fluor-5-[2-methoxy-6-oxo-4-trifluormethyl-1(6H)-pyrimidinyl]-benzoesäure, Smp. 205-210°C.

In analoger Weise erhält man beim Einsatz von:

- 2-Chlor-4-fluor-5-[2-methoxy-6-oxo-4-pentafluoräthyl -1(6H)-pyrimidinyl]-benzoesäure-isopropylester die 2-Chlor-4-fluor-5-[2-methoxy-6-oxo-4-pentafluoräthyl -1(6H)-pyrimidinyl]-benzoesäure, Smp. 197-199°C;
- 2-Chlor-4-fluor-5-[5-fluor-2-methoxy-6-oxo-4-trifluormethyl-1(6H)-pyrimidinyl]-benzoesäure-isopropylester die 2-Chlor-4-fluor-5-[5-fluor-2-methoxy-6-oxo-4-trifluormethyl -1(6H)-pyrimidinyl]-benzoesäure, Smp. 199-201°C;
- 2-Chlor-4-fluor-5-[3,6-dihydro-2,6-dioxo-3-methyl-4-trifluormethyl-1(2H)-pyrimidinyl]-benzoesäure-isopropylester die 2-Chlor-5-[3,6-dihydro-2,6-dioxo-3-methyl-4-trifluormethyl-1(2H)-pyrimidinyl]-4-fluorbenzoesäure, Smp. 239-242° C;
- 2-Chlor-5-[3,6-dihydro-2,6-dioxo-3-methyl-4-trifluormethyl-1(2H)-pyrimidinyl]-benzoesäure-isopropylester die 2-Chlor-5-[3,6-dihydro-2,6-dioxo-3-methyl-4-trifluormethyl -1(2H)-pyrimidinyl]-benzoesäure` Smp. 235-236° C;
- 2-Chlor-5-[3,6-dihydro-2,6-dioxo-3-methyl-4-pentafluoräthyl-1(2H)-pyrimidinyl]-4-fluorbenzoesäure-isopropylester die 2-Chlor-5-[3,6-dihydro-2,6-dioxo-3-methyl-4-pentafluor-äthyl-1(2H)-pyrimidinyl]-4-fluorbenzoesäure, Smp. 229-231°C;
- 2-Chlor-5-[3,6-dihydro-3,4-dimethyl-2,6-dioxo-1(2H) -pyrimidinyl]-4-fluorbenzoesäure-isopropylester die 2-Chlor-5-[3,6-dihydro-3,4-dimethyl-2,6-dioxi-1(2H)-pyrimidinyl]-4-fluorbenzoesäure,Smp. 236-239°C;
- 2-Chlor-5-[2-methoxy-6-oxo-4-trifluormethyl-1(6H)-pyrimidinyl]-benzoesäure-isopropylester die 2-Chlor-5-[2-methoxy-6-oxo-4-trifluormethyl-1(6H)pyrimidinyl] -benzoesäure, Smp. 265-268°C.

III. Herstellung der 3-Isocyanatobenzoesäure-ester der Formel XI:

Beispiel 30

Der als Ausgangsmaterial in Beispiel 26 benötigte 2-Chlor-4-fluor-5-isocyanatobenzoesäure-cyclopentylester kann wie folgt hergestellt werden:

Ein Gemisch von 100 g 2-Chlor-4-fluor-5-nitrobenzoesäure, 50 ml Thionylchlorid und 100 ml Benzol wird unter Zusatz von 0,5 ml Dimethylformamid 4 Stunden bei Rückflusstemperatur erhitzt. Anschliessend destilliert man das Lösungsmittel ab; es bleibt rohes 2-Chlor-4-fluor-5-nitrobenzoylchlorid zurück.

Das 2-Chlor-4-fluor-5-nitrobenzoylchlorid wird in 400 ml Dioxan gelöst und die Lösung unter Rühren bei Raumtemperatur zu einer Lösung von 36 ml Cyclopentanol und 42 ml Pyridin in 300 ml Dioxan zugetropft. Nach beendeter Zugabe wird noch 4 Stunden bei Raumtemperatur gerührt, auf 300 g Eis und 300 ml 2N-Salzsäure gegeben und zweimal mit je 300 ml Aethylacetat extrahiert.

Die vereinigten organischen Phasen werden einmal mit 300 ml 2N-Salzsäure und dreimal mit je 150 ml gesättigter Natriumchloridlösung gewaschen, über wasserfreiem Magnesiumsulfat getrocknet und eingeengt. Nach Umkristallisieren aus Diisopropyläthyl/n-Hexan erhält man den 2-Chlor-4-fluor-5-nitrobenzoesäure-cyclopentylester, Smp. 44-45°C.

71 g Eisenpulver werden in 190 ml Aethanol, 50 ml Wasser und 5 ml 32%iger Salzsäure unter Rühren vorgelegt, und das Ganze wird auf 75°C (Innentemperatur) erwärmt. Zu diesem Gemisch wird eine warme Lösung von 100 g 2-Chlor-4-fluor-5-nitrobenzoesäure-cyclopentylester in 50 ml Aethanol zugetropft. Man rührt das Reaktionsgemisch weitere 2 Stunden bei 70-75°C (Innentemperatur), kühlt es ab, nutscht es nach Erhalten über Celite® ab und spült den Nutschrückstand mit 500 ml Wasser, gefolgt von 200 ml Aethylacetat, nach. Das Filtrat wird mit gesättigter Kaliumhydrogencarbonatlösung auf einen pH-Wert von 8

gebracht und der resultierende braune Niederschlag über Celite abfiltriert. Man extrahiert das Filtrat zweimal mit je 400 ml Aethylacetat, wäscht die vereinigten organischen Phasen zweimal mit je 200 ml gesättigter Natriumchloridlösung, trocknet sie über wasserfreiem Magnesiumsulfat und engt sie ein. Dann wird der Rückstand an einer Kieselgel-Säule unter Verwendung von Aethylacetat/n-Hexan (1:4) als Laufmittel chromatographisch gereinigt und aus Diisopropyläther/n-Hexan kristallisiert. Man erhält auf diese Weise den 5-Amino-2-chlor-4-fluorbenzoesäure-cyclopentylester, Smp. 61-62° C.

Zu einer auf 70° (Innentemperatur) erwärmten Lösung von 9 ml Diphosgen in 40 ml Aethylacetat wird unter Rühren eine Lösung von 19 g 5-Amino-2-chlor-4-fluorbenzoesäurecyclopentylester in 100 ml Aethylacetat zugetropft. Das Reaktionsgemisch wird dann 2 Stunden bei Rückflusstemperatur erhitzt. Anschliessend wird das Lösungsmittel abdestilliert und der Rückstand im Kugelrohr unter stark vermindertem Druck destilliert. Man erhält dann den als Ausgangsmaterial in Beispiel 26 benötigten 2-Chlor-4-fluor-5-isocyanatobenzoesäure-cyclopentylester, Sdp. 160°C/7,98 Pa (0,06mm Hg).

IV. Formulierungsbeispiele:

Beispiel 31

Ein emulgierbares Konzentrat enthält folgende Bestandteile:

| | |
|---|---|
| Erfindungsgemässe Verbindung (Wirkstoff) | 50 g/l |
| N-Methylpyrrolidon (Lösungsvermittler) | 200 g/l |
| Nonylphenol-(10)äthoxylat (nichtionogener Emulgator) | 50 g/l |
| Calcium-dodecylbenzosulfonat (anionischer Emulgator) | 25 g/l |
| Gemisch von Alkylbenzolen (Lösungsmittel) | ad 1000 ml |

Der Wirkstoff und die Emulgatoren werden unter Rühren im Lösungsvermittler gelöst, und die Lösung wird mit dem Lösungsmittel auf 1 Liter ergänzt.

Das resultierende emulgierbare Konzentrat lässt sich in Wasser emulgieren und ergibt so eine gebrauchsfertige Spritzbrühe mit der gewünschten Konzentration.

Beispiel 32

Zur Herstellung eines 25% Spritzpulvers werden die nachstehend aufgeführten Bestandteile miteinander vermischt:

| | |
|---|---|
| Erfindungsgemässe Verbindung (Wirkstoff) | 25 g |
| Kieselsäure, hydratisiert (Trägerstoff, Mahlhilfsmittel) | 5 - 25 g |
| Natrium-laurylsulfat (Netzmittel) | 1 g |
| Natrium-lignosulfonat (Dispergator) | 2 g |
| Kaolin (Trägerstoff) | 67 - 47 g |
| | 100 g |

Anschliessend wird das Gemisch unter Verwendung einer Stiftmühle oder vergleichbaren Mahleinrichtung fein gemahlen.

Das resultierende Spritzpulver ergibt beim Einrühren in Wasser eine feine Suspension, die sich als gebrauchsfertige Spritzbrühe eignet.

## Patentansprüche

1. Verbindungen der allgemeinen Formel

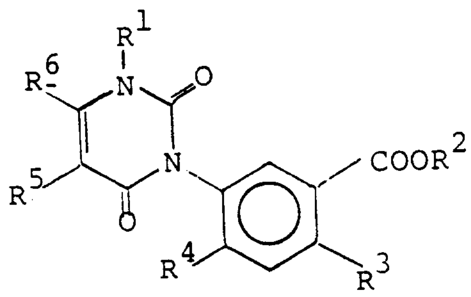

I

worin

$R^1$ Wasserstoff, $C_{1-4}$-Alkyl, $C_{3\ \text{oder}\ 4}$-Alkenyl, $C_{3\ \text{oder}\ 4}$-Alkinyl oder $C_{1-4}$-Halogenalkyl,

$R^2$ eine Gruppe

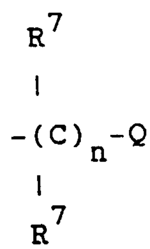

oder, im Falle, dass $R^1$ Halogenalkyl bedeutet, auch Wasserstoff, $C_{1-8}$-Alkyl, $C_{2-8}$-Alkenyl, $C_{2-8}$-Alkinyl oder $C_{2-8}$-Alkoxyalkyl,

$R^3$ Halogen oder Cyano,

$R^4$ Wasserstoff oder Halogen,

$R^5$ Wasserstoff, Fluor oder $C_{1-4}$-Alkyl,

$R^6$ $C_{1-4}$-Alkyl oder $C_{1-4}$-Halogenalkyl,

die Symbole $R^7$ unabhängig voneinander Wasserstoff oder $C_{1-3}$-Alkyl und

n 0, 1 oder 2 bedeuten,

und

Q einen gegebenenfalls mit einem oder mehreren $C_{1-4}$-Alkylresten substituierten gesättigten drei- bis siebengliedrigen carbocyclischen oder heterocyclischen Rest bedeutet, wobei letzterer 1 oder 2 aus Sauerstoff und Schwefel ausgewählte Heteroatome und gegebenenfalls eine Ketofunktion im Ring aufweist,

oder

Q einen gegebenenfalls mit Halogen, $C_{1-4}$-Alkyl, $C_{1-4}$-Halogenalkyl, $C_{1-4}$-Alkoxy, $C_{1-4}$-Alkylthio, Nitro und/oder Cyano mono- oder mehrfach substituierten Phenylrest bedeutet, der zusätzlich einen ankondensierten, gesättigten, carbocyclischen oder heterocyclischen, fünf- bis siebengliedrigen Ring enthalten kann, wobei der Heterozyklus 1 oder 2 Sauerstoffatome im Ring aufweist,

und die entsprechenden Enoläther derjenigen Verbindungen der Formel I, in denen $R^1$ von Wasserstoff oder $C_{1-4}$-Halogenalkyl verschieden ist, sowie Salze derjenigen Verbindungen der Formel I, in denen $R^1$ oder $R^2$ Wasserstoff bedeutet.

2. Verbindungen nach Anspruch 1, worin $R^1$ Wasserstoff, $C_{1-4}$-Alkyl, $C_{3\ \text{oder}\ 4}$-Alkenyl oder $C_{3\ \text{oder}\ 4}$-Alkinyl bedeutet, und deren Enoläther und Salze.

3. Verbindungen nach Anspruch 1, worin $R^1$ $C_{1-4}$-Halogenalkyl, $R^2$ Wasserstoff, $C_{1-8}$-Alkyl, $C_{2-8}$-Alkenyl, $C_{2-8}$-Alkinyl, $C_{2-8}$-Alkoxyalkyl oder eine Gruppe $-(CR^7R^7)_n$-Q und Q entweder einen gegebenenfalls

mit einem oder mehreren $C_{1-4}$-Alkylresten substituierten, gesättigten 3- bis 7-gliedrigen carbocyclischen oder heterocyclischen Rest, wobei letzterer 1 oder 2 aus Sauerstoff oder Schwefel ausgewählte Heteroatome im Ring ausweist, oder einen gegebenenfalls mit Halogen, $C_{1-4}$-Alkyl, $C_{1-4}$-Halogenalkyl, $C_{1-4}$-Alkoxy, $C_{1-4}$-Alkylthio, Nitro und/oder Cyano mono- oder mehrfach substituierten Phenylrest bedeuten, und die Salze dieser Verbindungen.

**4.** Verbindungen nach Anspruch 2, worin $R^1$ Methyl bedeutet.

**5.** Verbindungen nach Anspruch 3, worin $R^1$ Difluormethyl bedeutet.

**6.** Verbindungen nach einem der Ansprüche 1 bis 5, worin $R^3$ Chlor oder Brom und $R^4$ Fluor bedeuten.

**7.** Verbindungen nach einem der Ansprüche 1 bis 6, worin $R^3$ Chlor oder Brom und $R^4$ Wasserstoff bedeuten.

**8.** Verbindungen nach einem der Ansprüche 1 bis 7, worin $R^5$ Wasserstoff, Fluor oder Methyl bedeutet.

**9.** Verbindungen nach einem der Ansprüche 1 bis 8, worin $R^6$ $C_{1-4}$-Alkyl bedeutet.

**10.** Verbindungen nach einem der Ansprüche 1 bis 9, worin $R^2$ eine Gruppe $-(CR^7R^7)_n-Q$ bedeutet, in der jedes $R^7$ Wasserstoff und n O oder 1 bedeuten.

**11.** Eine Verbindung nach Anspruch 2, ausgewählt aus
2-Chlor-5-[3,6-dihydro-2,6-dioxo-3-methyl-4-trifluormethyl-1(2H)-pyrimidinyl]-4-fluorbenzoesäure-cycloheptylester,
2-Chlor-5-[3,6-dihydro-2,6-dioxo-3-methyl-4-trifluormethyl-1(2H)-pyrimidinyl]-4-fluorbenzoesäure-(tetrahydrofuran-3-yl)ester,
2-Chlor-5-[3,6-dihydro-2,6-dioxo-3-methyl-4-trifluormethyl-1(2H)-pyrimidinyl]-4-fluorbenzoesäure-(1,3-dioxan-5-yl)ester,
2-Chlor-5-[3,6-dihydro-2,6-dioxo-3-methyl-4-trifluormethyl-1(2H)-pyrimidinyl]-4-fluorbenzoesäure-cyclopropylmethylester,
2-Chlor-5-[3,6-dihydro-2,6-dioxo-3-methyl-4-trifluormethyl-1(2H)-pyrimidinyl]-4-fluorbenzoesäure-tetrahydrofurfurylester,
2-Chlor-5-[3,6-dihydro-2,6-dioxo-3-methyl-4-trifluormethyl-1(2H)-pyrimidinyl]-4-fluorbenzoesäure-(1,3-dioxolan-4-ylmethyl)ester,
2-Chlor-5-[3,6-dihydro-2,6-dioxo-3-methyl-4-trifluormethyl-1(2H)-pyrimidinyl]-4-fluorbenzoesäure-benzylester,
2-Chlor-5-[3,6-dihydro-2,6-dioxo-3-methyl-4-trifluormethyl-1(2H)-pyrimidinyl]-4-fluorbenzoesäure-(4,4-dimethyl-2-oxo-tetrahydrofuran-3-yl)ester,
2-Chlor-5-[3,6-dihydro-2,6-dioxo-3-methyl-4-trifluormethyl-1(2H)-pyrimidinyl]-4-fluorbenzoesäure-cyclopentylester,
2-Chlor-5-[3,6-dihydro-2,6-dioxo-3-methyl-4-trifluormethyl-1(2H)-pyrimidinyl]-4-fluorbenzoesäure-(1-cyclopropyläthyl)ester und
2-Chlor-5-[3,6-dihydro-2,6-dioxo-3-methyl-4-trifluormethyl-1(2H)-pyrimidinyl]-4-fluorbenzoesäure-cyclohexylester.

**12.** Eine Verbindung nach Anspruch 3, ausgewählt aus
2-Chlor-5-[3-difluormethyl-3,6-dihydro-4-methyl-2,6-dioxo-1(2H)-pyrimidinyl]-4-fluorbenzoesäure-isopropylester,
2-Chlor-5-[3-difluormethyl-3,6-dihydro-4-methyl-2,6-dioxo-1(2H)-pyrimidinyl]-4-fluorbenzoesäure,
5-[4-Aethyl-3-difluormethyl-3,6-dihydro-2,6-dioxo-1(2H)-pyrimidinyl]-2-chlor-4-fluorbenzoesäure-isopropylester,
5-[4-Aethyl-3-difluormethyl-3,6-dihydro-2,6-dioxo-1(2H)-pyrimidinyl]-2-brom-4-fluorbenzoesäure-isopropylester,
2-Brom-5-[3-difluormethyl-3,6-dihydro-4-methyl-2,6-dioxo-1(2H)-pyrimidinyl]-4-fluorbenzoesäure-isopropylester,
2-Chlor-5-[3-difluormethyl-3,6-dihydro-4-methyl-2,6-dioxo-1(2H)-pyrimidinyl]-4-fluorbenzoesäure-2-propinylester und

2-Chlor-5-[3-difluormethyl-3,6-dihydro-4-methyl-2,6-dioxo-1(2H)-pyrimidinyl]-4-fluorbenzoesäure-methylester.

**13.** Eine Verbindung nach Anspruch 1, ausgewählt aus
2-Chlor-5-[3,6-dihydro-3,4-dimethyl-2,6-dioxo-1(2H)-pyrimidinyl]-4-fluorbenzoesäure-cyclopropylmethylester,
2-Chlor-5-[3,6-dihydro-3,4-dimethyl-2,6-dioxo-1(2H)-pyrimidinyl-4-fluorbenzoesäure-(1-cyclopropyläthyl)ester,
2-Chlor-5-[3,6-dihydro-3,4-dimethyl-2,6-dioxo-1(2H)-pyrimidinyl]-4-fluorbenzoesäure-cyclopentylester,
2-Chlor-5-[3,6-dihydro-3,4-dimethyl-2,6-dioxo-1(2H)-pyrimidinyl]-4-fluorbenzoesäure-cyclohexylester,
2-Chlor-5-[3,6-dihydro-3,4-dimethyl-2,6-dioxo-1(2H)-pyrimidinyl]-4-fluorbenzoesäure-cycloheptylester,
2-Chlor-5-[3,6-dihydro-3,4-dimethyl-2,6-dioxo-1(2H)-pyrimidinyl]-4-fluorbenzoesäure-(tetrahydrofuran-3-yl)ester,
2-Chlor-5-[3,6-dihydro-3,4-dimethyl-2,6-dioxo-1(2H)-pyrimidinyl]-4-fluorbenzoesäure-(1,3-dioxan-5-yl)ester,
2-Chlor-5-[3,6-dihydro-3,4-dimethyl-2,6-dioxo-1(2H)-pyrimidinyl]-4-fluorbenzoesäure-(1,3-dioxolan-4-yl)methylester,
2-Chlor-5-[3,6-dihydro-3,4-dimethyl-2,6-dioxo-1(2H)-pyrimidinyl]-4-fluorbenzoesäure-benzylester und
2-Chlor-5-[3,6-dihydro-3,4-dimethyl-2,6-dioxo-1(2H)-pyrimidinyl]-4-fluorbenzoesäure-tetrahydrofurfurylester.

**14.** Verbindungen der allgemeinen Formel

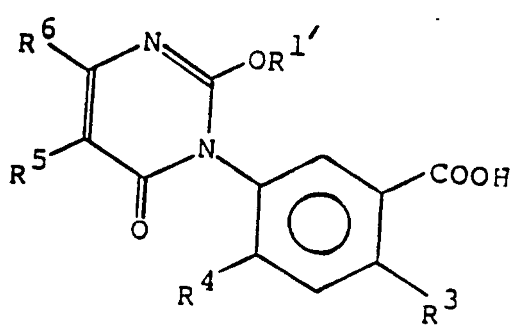

IIIa

worin
$R^{1'}$ $C_{1-4}$-Alkyl, $C_{3 \text{ oder } 4}$-Alkenyl oder $C_{3 \text{ oder } 4}$-Alkinyl,
$R^3$ Halogen oder Cyano,
$R^4$ Wasserstoff oder Halogen,
$R^5$ Wasserstoff, Fluor oder $C_{1-4}$-Alkyl und
$R^6$ $C_{1-4}$-Alkyl oder $C_{1-4}$-Halogenalkyl bedeuten,
sowie die Salze davon.

**15.** Unkrautbekämpfungsmittel, dadurch gekennzeichnet, dass es eine wirksame Menge mindestens einer Verbindung der allgemeinen Formel

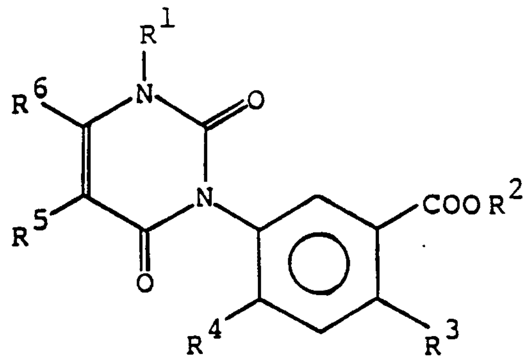

I

worin

$R^1$ Wasserstoff, $C_{1-4}$-Alkyl, $C_{3\ \mathrm{oder}\ 4}$-Alkenyl, $C_{3\ \mathrm{oder}\ 4}$-Alkinyl oder $C_{1-4}$-Halogenalkyl,

$R^2$ eine Gruppe

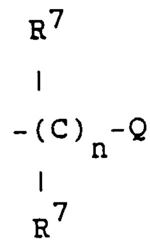

oder, im Falle, dass $R^1$ Halogenalkyl bedeutet, auch Wasserstoff, $C_{1-8}$-Alkyl, $C_{2-8}$-Alkenyl, $C_{2-8}$-Alkinyl oder $C_{2-8}$-Alkoxyalkyl,

$R^3$ Halogen oder Cyano,

$R^4$ Wasserstoff oder Halogen,

$R^5$ Wasserstoff, Fluor oder $C_{1-4}$-Alkyl,

$R^6$ $C_{1-4}$-Alkyl oder $C_{1-4}$-Halogenalkyl,

die Symbole $R^7$ unabhängig voneinander Wasserstoff oder $C_{1-3}$-Alkyl und

n 0, 1 oder 2 bedeuten,

und

Q einen gegebenenfalls mit einem oder mehreren $C_{1-4}$-Alkylresten substituierten gesättigten drei- bis siebengliedrigen carbocyclischen oder heterocyclischen Rest bedeutet, wobei letzterer 1 oder 2 aus Sauerstoff und Schwefel ausgewählte Heteroatome und gegebenenfalls eine Ketofunktion im Ring aufweist,

oder

Q einen gegebenenfalls mit Halogen, $C_{1-4}$-Alkyl, $C_{1-4}$-Halogenalkyl, $C_{1-4}$-Alkoxy, $C_{1-4}$-Alkylthio, Nitro und/oder Cyano mono- oder mehrfach substituierten Phenylrest bedeutet, der zusätzlich einen ankondensierten, gesättigten, carbocyclischen oder heterocyclischen, fünf- bis siebengliedrigen Ring enthalten kann, wobei der Heterozyklus 1 oder 2 Sauerstoffatome im Ring aufweist,

oder eines Enoläthers einer solchen Verbindung I, in der $R^1$ von Wasserstoff oder $C_{1-4}$-Halogenalkyl verschieden ist, oder eines Salzes einer solchen Verbindung I, in der $R^1$ oder $R^2$ Wasserstoff bedeutet, sowie Formulierungshilfsstoffe enthält.

**16.** Unkrautbekämpfungsmittel nach Anspruch 15, dadurch gekennzeichnet, dass es eine wirksame Menge mindestens einer aus der Gruppe

2-Chlor-5-[3,6-dihydro-2,6-dioxo-3-methyl-4-trifluormethyl-1(2H)-pyrimidinyl]-4-fluorbenzoesäure-cycloheptylester,

2-Chlor-5-[3,6-dihydro-2,6-dioxo-3-methyl-4-trifluormethyl-1(2H)-pyrimidinyl]-4-fluorbenzoesäure-(tetrahydrofuran-3-yl)ester,

2-Chlor-5-[3,6-dihydro-2,6-dioxo-3-methyl-4-trifluormethyl-1(2H)-pyrimidinyl]-4-fluorbenzoesäure-(1,3-dioxan-5-yl)ester,
2-Chlor-5-[3,6-dihydro-2,6-dioxo-3-methyl-4-trifluormethyl-1(2H)-pyrimidinyl]-4-fluorbenzoesäure-cyclopropylmethylester,
2-Chlor-5-[3,6-dihydro-2,6-dioxo-3-methyl-4-trifluormethyl-1(2H)-pyrimidinyl]-4-fluorbenzoesäure-tetrahydrofurfurylester,
2-Chlor-5-[3,6-dihydro-2,6-dioxo-3-methyl-4-trifluormethyl-1(2H)-pyrimidinyl]-4-fluorbenzoesäure-(1,3-dioxolan-4-ylmethyl)ester,
2-Chlor-5-[3,6-dihydro-2,6-dioxo-3-methyl-4-trifluormethyl-1(2H)-pyrimidinyl]-4-fluorbenzoesäure-benzylester,
2-Chlor-5-[3,6-dihydro-2,6-dioxo-3-methyl-4-trifluormethyl-1(2H)-pyrimidinyl]-4-fluorbenzoesäure-(4,4-dimethyl-2-oxo-tetrahydrofuran-3-yl)ester,
2-Chlor-5-[3,6-dihydro-2,6-dioxo-3-methyl-4-trifluormethyl-1(2H)-pyrimidinyl]-4-fluorbenzoesäure-cyclopentylester,
2-Chlor-5-[3,6-dihydro-2,6-dioxo-3-methyl-4-trifluormethyl-1(2H)-pyrimidinyl]-4-fluorbenzoesäure-(1-cyclopropyläthyl)ester und
2-Chlor-5-[3,6-dihydro-2,6-dioxo-3-methyl-4-trifluormethyl-1(2H)-pyrimidinyl]-4-fluorbenzoesäure-cyclohexylester ausgewählten Verbindung sowie Formulierungshilfsstoffe enthält.

**17.** Unkrautbekämpfungsmittel nach Anspruch 15, dadurch gekennzeichnet, dass es eine wirksame Menge mindestens einer aus der Gruppe
2-Chlor-5-[3-difluormethyl-3,6-dihydro-4-methyl-2,6-dioxo-1(2H)-pyrimidinyl]-4-fluorbenzoesäure-isopropylester,
2-Chlor-5-[3-difluormethyl-3,6-dihydro-4-methyl-2,6-dioxo-1(2H)-pyrimidinyl]-4-fluorbenzoesäure,
5-[4-Aethyl-3-difluormethyl-3,6-dihydro-2,6-dioxo-1(2H)-pyrimidinyl]-2-chlor-4-fluorbenzoesäure-isopropylester,
5-[4-Aethyl-3-difluormethyl-3,6-dihydro-2,6-dioxo-1(2H)-pyrimidinyl]-2-brom-4-fluorbenzoesäure-isopropylester,
2-Brom-5-[3-difluormethyl-3,6-dihydro-4-methyl-2,6-dioxo-1(2H)-pyrimidinyl]-4-fluorbenzoesäure-isopropylester,
2-Chlor-5-[3-difluormethyl-3,6-dihydro-4-methyl-2,6-dioxo-1(2H)-pyrimidinyl]-4-fluorbenzoesäure-2-propinylester und
2-Chlor-5-[3-difluormethyl-3,6-dihydro-4-methyl-2,6-dioxo-1(2H)-pyrimidinyl]-4-fluorbenzoesäure-methylester
ausgewählten Verbindung sowie Formulierungshilfsstoffe enthält.

**18.** Unkrautbekämpfungsmittel nach Anspruch 15, dadurch gekennzeichnet, dass es eine wirksame Menge mindestens einer aus der Gruppe
2-Chlor-5-[3,6-dihydro-3,4-dimethyl-2,6-dioxo-1-(2H)-pyrimidinyl]-4-fluorbenzoesäure-cyclopropylmethylester,
2-Chlor-5-[3,6-dihydro-3,4-dimethyl-2,6-dioxo-1(2H)-pyrimidinyl]-4-fluorbenzoesäure-(1-cyclopropyläthyl)ester,
2-Chlor-5-[3,6-dihydro-3,4-dimethyl-2,6-dioxo-1(2H)-pyrimidinyl]-4-fluorbenzoesäure-cyclopentylester,
2-Chlor-5-[3,6-dihydro-3,4-dimethyl-2,6-dioxo-1(2H)-pyrimidinyl]-4-fluorbenzoesäure-cyclohexylester,
2-Chlor-5-[3,6-dihydro-3,4-dimethyl-2,6-dioxo-1(2H)-pyrimidinyl]-4-fluorbenzoesäure-cycloheptylester,
2-Chlor-5-[3,6-dihydro-3,4-dimethyl-2,6-dioxo-1(2H)-pyrimidinyl]-4-fluorbenzoesäure-(tetrahydrofuran-3-yl)ester,
2-Chlor-5-[3,6-dihydro-3,4-dimethyl-2,6-dioxo-1(2H)-pyrimidinyl]-4-fluorbenzoesäure-(1,3-dioxan-5-yl)ester,
2-Chlor-5-[3,6-dihydro-3,4-dimethyl-2,6-dioxo-1(2H)-pyrimidinyl]-4-fluorbenzoesäure-(1,3-dioxolan-4-yl)methylester,
2-Chlor-5-[3,6-dihydro-3,4-dimethyl-2,6-dioxo-1(2H)-pyrimidinyl]-4-fluorbenzoesäure-benzylester und
2-Chlor-5-[3,6-dihydro-3,4-dimethyl-2,6-dioxo-1(2H)-pyrimidinyl]-4-fluorbenzoesäure-tetrahydrofurfurylester
ausgewählten Verbindung sowie Formulierungshilfsstoffe enthält.

19. Unkrautbekämpfungsmittel, dadurch gekennzeichnet, dass es eine wirksame Menge mindestens einer Verbindung der allgemeinen Formel

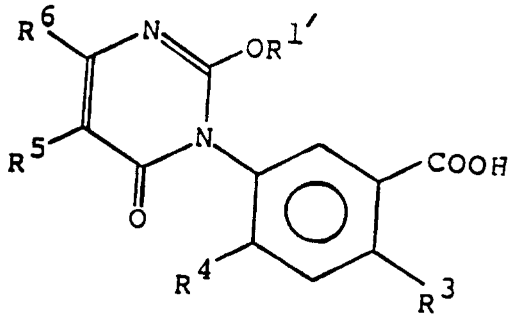

IIIa

worin

$R^{1'}$ $C_{1-4}$-Alkyl, $C_{1-4}$-Alkyl, $C_{3\text{ oder }4}$-Alkenyl oder $C_{3\text{ oder }4}$-Alkinyl,
$R^3$ Halogen oder Cyano,
$R^4$ Wasserstoff oder Halogen,
$R^5$ Wasserstoff, Fluor oder $C_{1-4}$-Alkyl und
$R^6$ $C_{1-4}$-Alkyl oder $C_{1-4}$-Halogenalkyl bedeuten,

oder eines Salzes davon, sowie Formulierungshilfsstoffe enthält.

20. Verfahren zur Herstellung von Verbindungen der allgemeinen Formel

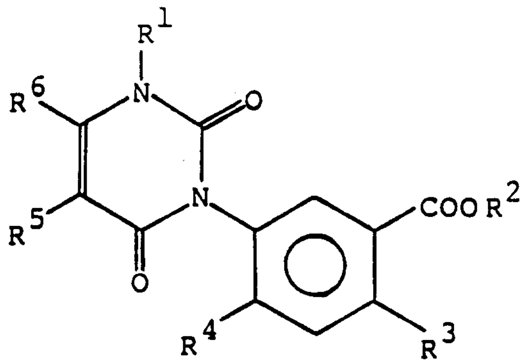

I

worin

$R^1$ Wasserstoff, $C_{1-4}$-Alkyl, $C_{3\text{ oder }4}$-Alkenyl, $C_{3\text{ oder }4}$-Alkinyl oder $C_{1-4}$-Halogenalkyl,
$R^2$ eine Gruppe

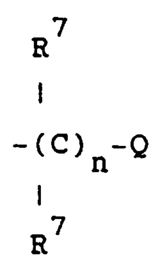

oder, im Falle, dass $R^1$ Halogenalkyl bedeutet, auch Wasserstoff, $C_{1-8}$-Alkyl, $C_{2-8}$-Alkenyl, $C_{2-8}$-Alkinyl oder $C_{2-8}$-Alkoxyalkyl,
$R^3$ Halogen oder Cyano,
$R^4$ Wasserstoff oder Halogen,
$R^5$ Wasserstoff, Fluor oder $C_{1-4}$-Alkyl,
$R^6$ $C_{1-4}$-Alkyl oder $C_{1-4}$-Halogenalkyl,

die Symbole $R^7$ unabhängig voneinander Wasserstoff oder $C_{1-3}$-Alkyl und

n 0, 1 oder 2 bedeuten,

und

Q einen gegebenenfalls mit einem oder mehreren $C_{1-4}$-Alkylresten substituierten gesättigten drei- bis siebengliedrigen carbocyclischen oder heterocyclischen Rest bedeutet, wobei letzterer 1 oder 2 aus Sauerstoff und Schwefel ausgewählte Heteroatome und gegebenenfalls eine Ketofunktion im Ring aufweist,

oder

Q einen gegebenenfalls mit Halogen, $C_{1-4}$-Alkyl, $C_{1-4}$-Halogenalkyl, $C_{1-4}$-Alkoxy, $C_{1-4}$-Alkylthio, Nitro und/oder Cyano mono- oder mehrfach substituierten Phenylrest bedeutet, der zusätzlich einen ankondensierten, gesättigten, carbocyclischen oder heterocyclischen, fünf- bis siebengliedrigen Ring enthalten kann, wobei der Heterozyklus 1 oder 2 Sauerstoffatome im Ring aufweist,

und von den entsprechenden Enoläthern derjenigen Verbindungen der Formel I, in denen $R^1$ von Wasserstoff oder $C_{1-4}$-Halogenalkyl verschieden ist, sowie von Salzen derjenigen Verbindungen der Formel I, in denen $R^1$ oder $R^2$ Wasserstoff bedeutet, dadurch gekennzeichnet, dass man

a) zwecks Herstellung derjenigen Verbindungen der Formel I, in denen $R^1$ Wasserstoff bedeutet, sowie gewünschtenfalls von Metallsalzen dieser Verbindungen, eine Verbindung der allgemeinen Formel

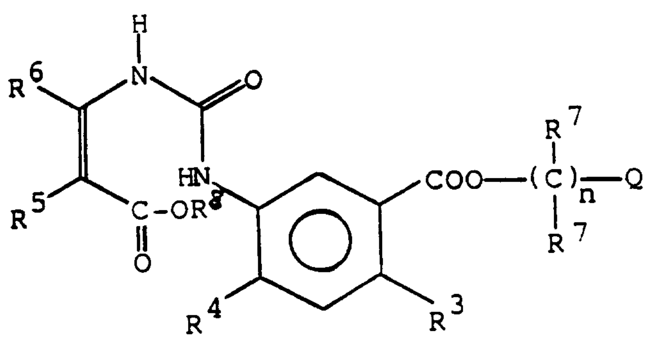

II

worin $R^3$, $R^4$, $R^5$, $R^6$, $R^7$, n und Q die oben angegebenen Bedeutungen besitzen und $R^8$ nieder Alkyl bedeutet,

einer Cyclisierung unter basischen Bedingungen unterwirft und gewünschtenfalls ein allfällig erhaltenes Metallsalz des Uracilderivats der Formel I durch Behandlung mit einer Säure in die saure Form ($R^1$ = Wasserstoff) überführt,

b) zwecks Herstellung derjenigen Verbindungen der Formel I, in denen $R^1$ $C_{1-4}$-Alkyl, $C_{3\text{ oder }4}$-Alkenyl, $C_{3\text{ oder }4}$-Alkinyl oder $C_{1-4}$-Halogenalkyl bedeutet, wobei, im Fall, dass $R^1$ $C_{1-4}$-Halogenalkyl bedeutet, $R^2$ verschieden von Wasserstoff ist, ein Uracilderivat der allgemeinen Formel

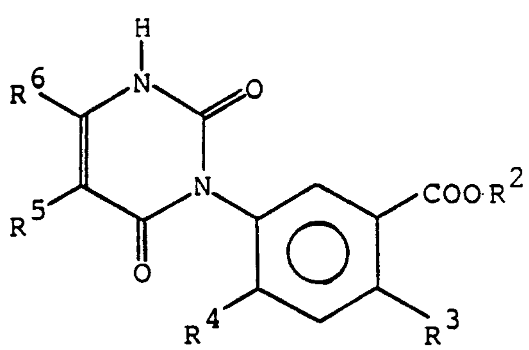

I'

worin $R^2$, $R^3$, $R^4$, $R^5$ und $R^6$ die oben angegebenen Bedeutungen besitzen,

einer Alkylierung mit einem entsprechenden, eine $C_{1-4}$-Alkyl-, $C_{3\text{ oder }4}$-Alkenyl- oder $C_{3\text{ oder }4}$-Alkinylgruppe enthaltenden Alkylierungsmittel unterwirft, mit der Massgabe, dass im Falle der Herstellung einer Verbindung der Formel I, in der $R^1$ $C_{1-4}$-Halogenalkyl bedeutet, $R^2$ der Formel I' verschieden

von Wasserstoff ist,
c) zwecks Herstellung derjenigen Verbindungen der Formel I, in denen $R^1$ verschieden von Wasserstoff ist, und der entsprechenden Enoläther eine Benzoesäure der allgemeinen Formel

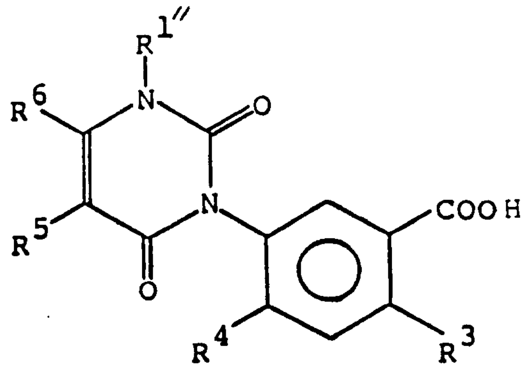

III

worin $R^3$, $R^4$, $R^5$ und $R^6$ die oben angegebenen Bedeutungen besitzen, und $R^{1''}$ $C_{1-4}$-Alkyl, $C_{3\ oder\ 4}$-Alkenyl, $C_{3\ oder\ 4}$-Alkinyl oder $C_{1-4}$-Halogenalkyl bedeutet,
oder den entsprechenden Enoläther, wobei die Benzoesäure bzw. deren Enoläther in Form eines reaktionsfähigen Derivats vorliegen kann, mit einer Hydroxyverbindung der allgemeinen Formel

$R^2OH$ IV

worin $R^2$ die oben angegebene Bedeutung besitzt, mit der Massgabe, dass im Falle der Herstellung einer Verbindung der Formel I, in der $R^1$ $C_{1-4}$-Halogenalkyl bedeutet, $R^2$ der Formel IV verschieden von Wasserstoff ist,
oder mit einem reaktionsfähigen Derivat dieser Hydroxyverbindung verestert,
d) zwecks Herstellung derjenigen Verbindungen der Formel I, in denen $R^1$ verschieden von Wasserstoff ist, einen Benzoesäureester der allgemeinen Formel

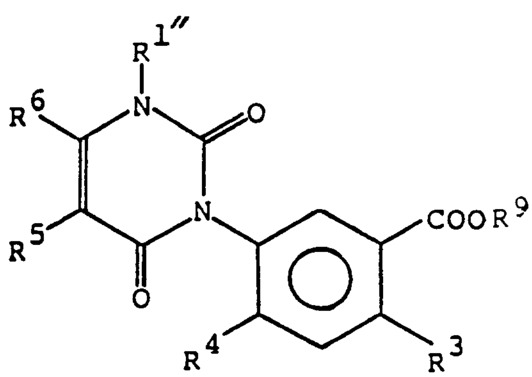

V

worin $R^{1''}$, $R^3$, $R^4$, $R^5$ und $R^6$ die oben angegebenen Bedeutungen besitzen und $R^9$ $C_{1-6}$-Alkyl, $C_{2-4}$-Alkenyl, $C_{2-4}$-Alkinyl oder $C_{2-6}$-Alkoxyalkyl bedeutet,
einer Umesterungsreaktion mit einer Hydroxyverbindung der oben angegebenen Formel IV unterwirft, wobei das Reagens IV höher siedend ist als das Alkanol, Alkenol bzw. Alkinol $R^9OH$,
e) zwecks Herstellung derjenigen Verbindungen der Formel I, in denen $R^1$ $C_{1-4}$-Halogenalkyl und $R^2$ Wasserstoff bedeuten, einen Benzoesäureester der allgemeinen Formel

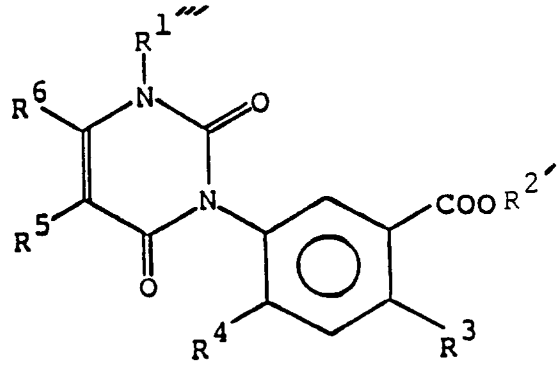

I'''

worin $R^3$, $R^4$, $R^5$ und $R^6$ die oben angegebenen Bedeutungen besitzen und $R^{1'''}$ $C_{1-4}$-Halogenalkyl bedeutet, und $R^{2'}$ die Bedeutung von $R^{2'}$ mit Ausnahme von Wasserstoff besitzt, zur entsprechenden Benzoesäure hydrolysiert,

f) zwecks Herstellung der Enoläther der Verbindungen der Formel I, ein Uracilderivat der allgemeinen Formel

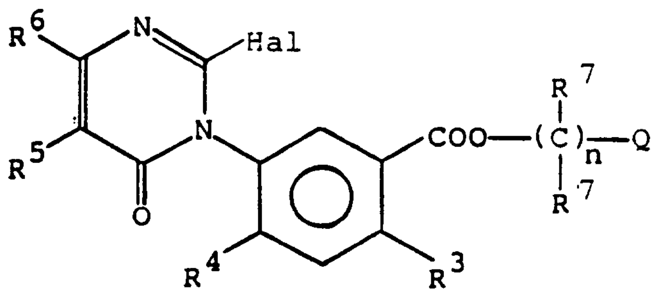

VI

worin $R^3$, $R^4$, $R^5$, $R^6$, $R^7$, n und Q die oben angegebenen Bedeutungen besitzen und Hal Chlor oder Brom bedeutet,

mit einem Alkanol, Alkenol oder Alkinol $R^{1'}$ OH in Gegenwart einer organischen Base oder mit dem entsprechenden Alkoholat, Alkenolat bzw. Alkinolat der allgemeinen Formel

$R^{1'}O^{\ominus}M^{\oplus}$ VII

worin $R^{1'}$ die oben angegebene Bedeutung besitzt und $M^{\oplus}$ ein Aequivalent eines Metallions bedeutet, behandelt,

und gewünschtenfalls eine so erhaltene Verbindung der Formel I, in der $R^1$ oder $R^2$ wasserstoff bedeutet, in ein Salz überführt.

**21.** Verfahren zur Herstellung von Verbindungen der allgemeinen Formel

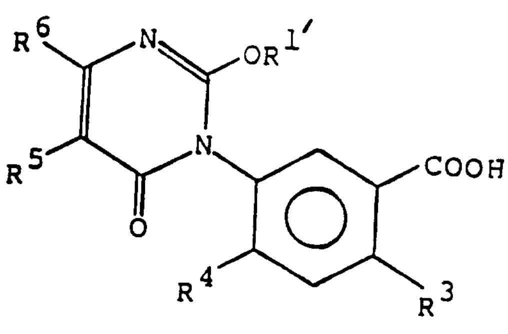

IIIa

worin

$R^{1'}$ $C_{1-4}$-Alkyl, $C_{3\ oder\ 4}$-Alkenyl oder $C_{3\ oder\ 4}$-Alkinyl,
$R^3$ Halogen oder Cyano,
$R^4$ Wasserstoff oder Halogen,
$R^5$ Wasserstoff, Fluor oder $C_{1-4}$-Alkyl

und

$R^6$ $C_{1-4}$-Alkyl oder $C_{1-4}$-Halogenalkyl bedeuten,

und von deren Salzen, dadurch gekennzeichnet, dass man eine Verbindung der allgemeinen Formel

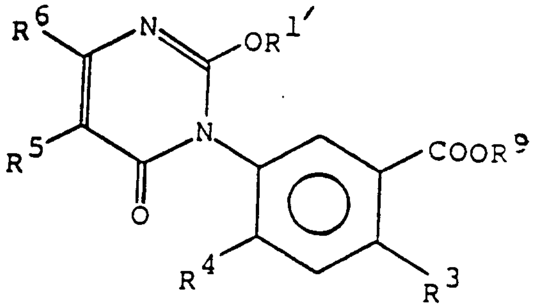

XV

worin $R^{1'}$, $R^3$, $R^4$, $R^5$ und $R^6$ die oben angegebenen Bedeutungen besitzen und $R^9$ $C_{1-6}$-Alkyl, $C_{2-4}$-Alkenyl, $C_{2-4}$-Alkinyl oder $C_{2-6}$-Alkoxyalkyl bedeutet,
einer Hydrolyse unterwirft,
und gewünschtenfalls eine so erhaltene Verbindung der Formel IIIa in ein Salz überführt.

**22.** Verfahren nach Anspruch 20 oder 21, worin $R^6$ jeweils $C_{1-4}$-Alkyl, insbesondere Methyl, bedeutet.

**23.** Verfahren zur Bekämpfung von Unkräutern, dadurch gekennzeichnet, dass man das gegen Unkräuter zu schützende Gut und/oder die Unkräuter mit einer wirksamen Menge einer Verbindung gemäss einem der Ansprüche 1 bis 14 bzw. eines Mittels gemäss einem der Ansprüche 15 bis 19 behandelt.

**24.** Verwendung einer Verbindung gemäss einem der Ansprüche 1 bis 14 bzw. eines Mittels gemäss einem der Ansprüche 15 bis 19 zur Bekämpfung von Unkräutern.

## Claims

**1.** Compounds of the general formula

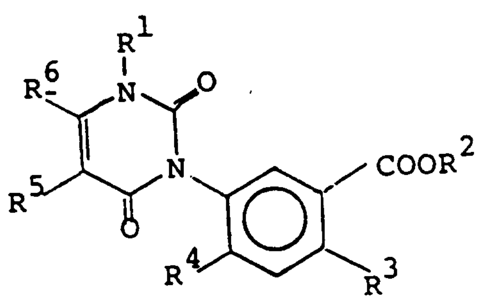

I

in which
$R^1$ signifies hydrogen, $C_{1-4}$alkyl, $C_{3\ or\ 4}$alkenyl, $C_{3\ or\ 4}$alkynyl or $C_{1-4}$haloalkyl,
$R^2$ signifies a group

$$\begin{array}{c} R^7 \\ | \\ -(C)_n-Q \\ | \\ R^7 \end{array}$$

or, where $R^1$ signifies haloalkyl, also hydrogen, $C_{1-8}$alkyl, $C_{2-8}$alkenyl, $C_{2-8}$alkynyl or $C_{2-8}$alkoxyalkyl,
$R^3$ signifies halogen or cyano,
$R^4$ signifies hydrogen or halogen,
$R^5$ signifies hydrogen, fluorine or $C_{1-4}$alkyl,
$R^6$ signifies $C_{1-4}$alkyl or $C_{1-4}$haloalkyl, the symbols $R^7$ each independently of one another signify hydrogen or $C_{1-3}$alkyl and
n signifies 0, 1 or 2,
and
Q signifies a saturated three- to seven-membered carbocyclic or heterocyclic radical which is optionally substituted by one or more $C_{1-4}$alkyl radicals, where the heterocyclic radical has 1 or 2 hetero atoms selected from oxygen and sulphur and optionally a keto function in the ring,
or
Q signifies a phenyl radical which is optionally mono- or poly-substituted by halogen, $C_{1-4}$alkyl, $C_{1-4}$haloalkyl, $C_{1-4}$alkoxy, $C_{1-4}$alkylthio, nitro and/or cyano and which additionally can contain a fused, saturated, carbocyclic or heterocyclic five- to seven-membered ring, where the heterocycle has 1 or 2 oxygen atoms in the ring, and the corresponding enol ethers of those compounds of formula I in which $R^1$ is different from hydrogen or $C_{1-4}$haloalkyl as well as salts of those compounds of formula I in which $R^1$ or $R^2$ signifies hydrogen.

**2.** Compounds according to claim 1, in which $R^1$ signifies hydrogen, $C_{1-4}$alkyl, $C_{3\ or\ 4}$alkenyl or $C_{3\ or\ 4}$alkyl, and their enol ethers and salts.

**3.** Compounds according to claim 1, in which $R^1$ signifies $C_{1-4}$haloalkyl, $R^2$ signifies hydrogen, $C_{1-8}$alkyl, $C_{2-8}$alkenyl, $C_{2-8}$alkynyl, $C_{2-8}$alkoxyalkyl or a group $-(CR^7R^7)_n-Q$ and Q signifies either a saturated 3- to 7-membered carbocyclic or heterocyclic radical which is optionally substituted by one or more $C_{1-4}$alkyl radicals, where the heterocyclic radical has 1 or 2 hetero atoms selected from oxygen or sulphur in the ring, or signifies a phenyl radical which is optionally mono- or poly-substituted by halogen, $C_{1-4}$alkyl, $C_{1-4}$haloalkyl, $C_{1-4}$alkoxy, $C_{1-4}$alkylthio, nitro and/or cyano, and the salts of these compounds.

**4.** Compounds according to claim 2, in which $R^1$ signifies methyl.

**5.** Compounds according to claim 3, in which $R^1$ signifies difluoromethyl.

**6.** Compounds according to any one of claims 1 to 5, in which $R^3$ signifies chlorine or bromine and $R^4$ signifies fluorine.

**7.** Compounds according to any one of claims 1 to 6, in which $R^3$ signifies chlorine or bromine and $R^4$ signifies hydrogen.

**8.** Compounds according to any one of claims 1 to 7, in which $R^5$ signifies hydrogen, fluorine or methyl.

**9.** Compounds according to any one of claims 1 to 8, in which $R^6$ signifies $C_{1-4}$alkyl.

**10.** Compounds according to any one of claims 1 to 9, in which $R^2$ signifies a group $-(CR^7R^7)_n-Q$ in which each $R^7$ signifies hydrogen and n signifies 0 or 1.

11. A compound according to claim 2, selected from
cycloheptyl 2-chloro-5-[3,6-dihydro-2,6-dioxo-3-methyl-4-trifluoromethyl-1-(2H)-pyrimidinyl]-4-fluorobenzoate,
tetrahydrofuran-3-yl 2-chloro-5-[3,6-dihydro-2,6-dioxo-3-methyl-4-trifluoromethyl-1(2H)-pyrimidinyl]-4-fluorobenzoate,
1,3-dioxan-5-yl 2-chloro-5-[3,6-dihydro-2,6-dioxo-3-methyl-4-trifluoromethyl-1(2H)-pyrimidinyl]-4-fluorobenzoate,
cyclopropylmethyl 2-chloro-5-[3,6-dihydro-2,6-dioxo-3-methyl-4-trifluoromethyl-1(2H)-pyrimidinyl]-4-fluorobenzoate,
tetrahydrofurfuryl 2-chloro-5-[3,6-dihydro-2,6-dioxo-3-methyl-4-trifluoromethyl-1(2H)-pyrimidinyl]-4-fluorobenzoate,
1,3-dioxolan-4-ylmethyl 2-chloro-5-[3,6-dihydro-2,6-dioxo-3-methyl-4-trifluoromethyl-1(2H)-pyrimidinyl]-4-fluorobenzoate,
benzyl 2-chloro-5-[3,6-dihydro-2,6-dioxo-3-methyl-4-trifluoromethyl-1(2H)-pyrimidinyl]-4-fluorobenzoate,
4,4-dimethyl-2-oxo-tetrahydrofuran-3-yl 2-chloro-5-[3,6-dihydro-2,6-dioxo-3-methyl-4-trifluoromethyl-1-(2H)-pyrimidinyl]-4-fluorobenzoate,
cyclopentyl 2-chloro-5-[3,6-dihydro-2,6-dioxo-3-methyl-4-trifluoromethyl-1(2H)-pyrimidinyl]-4-fluorobenzoate,
1-cyclopropylethyl 2-chloro-5-[3,6-dihydro-2,6-dioxo-3-methyl-4-trifluoromethyl-1(2H)-pyrimidinyl]-4-fluorobenzoate and
cyclohexyl 2-chloro-5-[3,6-dihydro-2,6-dioxo-3-methyl-4-trifluoromethyl-1(2H)-pyrimidinyl]-4-fluorobenzoate.

12. A compound according to claim 3, selected from
isopropyl 2-chloro-5-[3-difluoromethyl-3,6-dihydro-4-methyl-2,6-dioxo-1(2H)-pyrimidinyl]-4-fluorobenzoate,
2-chloro-5-[3-difluoromethyl-3,6-dihydro-4-methyl-2,6-dioxo-1(2H)-pyrimidinyl]-4-fluorobenzoic acid,
isopropyl 5-[4-ethyl-3-difluoromethyl-3,6-dihydro-2,6-dioxo-1(2H)-pyrimidinyl]-2-chloro-4-fluorobenzoate,
isopropyl 5-[4-ethyl-3-difluoromethyl-3,6-dihydro-2,6-dioxo-1(2H)-pyrimidinyl]-2-bromo-4-fluorobenzoate,
isopropyl 2-bromo-5-[3-difluoromethyl-3,6-dihydro-4-methyl-2,6-dioxo-1(2H)-pyrimidinyl]-4-fluorobenzoate,
2-propynyl 2-chloro-5-[3-difluoromethyl-3,6-dihydro-4-methyl-2,6-dioxo-1(2H)-pyrimidinyl]-4-fluorobenzoate and
methyl 2-chloro-5-[3-difluoromethyl-3,6-dihydro-4-methyl-2,6-dioxo-1(2H)-pyrimidinyl]-4-fluorobenzoate.

13. A compound according to claim 1, selected from
cyclopropylmethyl 2-chloro-5-[3,6-dihydro-3,4-dimethyl-2,6-dioxo-1(2H)-pyrimidinyl]-4-fluorobenzoate,
1-cyclopropylethyl 2-chloro-5-[3,6-dihydro-3,4-dimethyl-2,6-dioxo-1(2H)-pyrimidinyl]-4-fluorobenzoate,
cyclopentyl 2-chloro-5-[3,6-dihydro-3,4-dimethyl-2,6-dioxo-1(2H)-pyrimidinyl]-4-fluorobenzoate,
cyclohexyl 2-chloro-5-[3,6-dihydro-3,4-dimethyl-2,6-dioxo-1(2H)-pyrimidinyl]-4-fluorobenzoate,
cycloheptyl 2-chloro-5-[3,6-dihydro-3,4-dimethyl-2,6-dioxo-1(2H)-pyrimidinyl]-4-fluorobenzoate,
tetrahydrofuran-3-yl 2-chloro-5-[3,6-dihydro-3,4-dimethyl-2,6-dioxo-1(2H)-pyrimidinyl]-4-fluorobenzoate,
1,3-dioxan-5-yl 2-chloro-5-[3,6-dihydro-3,4-dimethyl-2,6-dioxo-1(2H)-pyrimidinyl]-4-fluorobenzoate,
(1,3-dioxolan-4-yl)methyl 2-chloro-5-[3,6-dihydro-3,4-dimethyl-2,6-dioxo-1(2H)-pyrimidinyl]-4-fluorobenzoate,
benzyl 2-chloro-5-[3,6-dihydro-3,4-dimethyl-2,6-dioxo-1(2H)-pyrimidinyl]-4-fluorobenzoate and
tetrahydrofurfuryl 2-chloro-5-[3,6-dihydro-3,4-dimethyl-2,6-dioxo-1(2H)-pyrimidinyl]-4-fluorobenzoate.

**14.** Compounds of the general formula

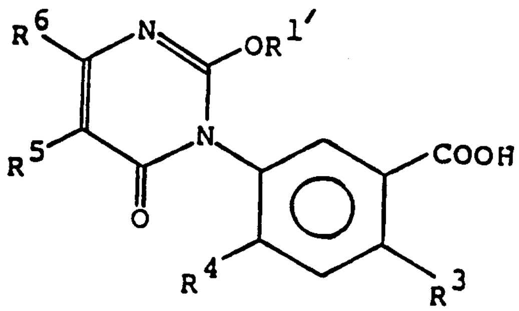

IIIa

in which
$R^{1'}$ signifies $C_{1-4}$alkyl, $C_{3\text{ or }4}$alkenyl or $C_{3\text{ or }4}$alkynyl,
$R^3$ signifies halogen or cyano,
$R^4$ signifies hydrogen or halogen,
$R^5$ signifies hydrogen, fluorine or $C_{1-4}$alkyl and
$R^6$ signifies $C_{1-4}$alkyl or $C_{1-4}$haloalkyl, as well as the salts thereof.

**15.** A weed control composition, characterized in that it contains an effective amount of at least one compound of the general formula

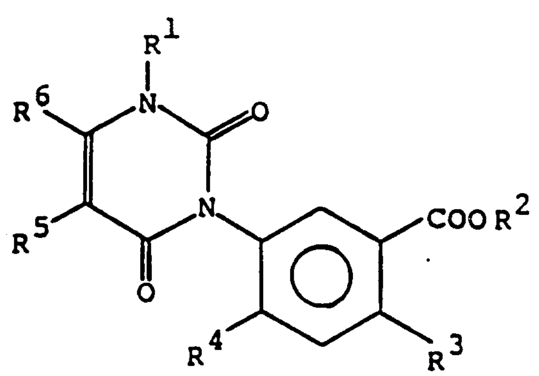

I

in which
$R^1$ signifies hydrogen, $C_{1-4}$alkyl, $C_{3\text{ or }4}$alkenyl, $C_{3\text{ or }4}$alkynyl or $C_{1-4}$haloalkyl,
$R^2$ signifies a group

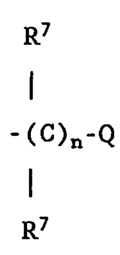

or, where $R^1$ signifies haloalkyl, also hydrogen, $C_{1-8}$alkyl, $C_{2-8}$alkenyl, $C_{2-8}$alkynyl or $C_{2-8}$alkoxyalkyl,
$R^3$ signifies halogen or cyano,
$R^4$ signifies hydrogen or halogen,
$R^5$ signifies hydrogen, fluorine or $C_{1-4}$alkyl,
$R^6$ signifies $C_{1-4}$alkyl or $C_{1-4}$haloalkyl, the symbols $R^7$ each independently of one another signify hydrogen or $C_{1-3}$alkyl and

n signifies 0, 1 or 2,
and
Q signifies a saturated three- to seven-membered carbocyclic or heterocyclic radical which is optionally substituted by one or more $C_{1-4}$alkyl radicals, where the heterocyclic radical has 1 or 2 hetero atoms selected from oxygen and sulphur and optionally a keto function in the ring,
or
Q signifies a phenyl radical which is optionally mono- or polysubstituted by halogen, $C_{1-4}$alkyl, $C_{1-4}$haloalkyl, $C_{1-4}$alkoxy, $C_{1-4}$alkylthio, nitro and/or cyano and which additionally can contain a fused, saturated, carbocyclic or heterocyclic five- to seven-membered ring, where the heterocycle has 1 or 2 oxygen atoms in the ring,
or of an enol ether of such a compound I in which $R^1$ is different from hydrogen or $C_{1-4}$haloalkyl or of a salt of such a compound I in which $R^1$ or $R^2$ is hydrogen, as well as formulation adjuvants.

**16.** A weed control composition according to claim 15, characterized in that it contains an effective amount of at least one compound selected from the group
cycloheptyl 2-chloro-5-[3,6-dihydro-2,6-dioxo-3-methyl-4-trifluoromethyl-1-(2H)-pyrimidinyl]-4-fluorobenzoate,
tetrahydrofuran-3-yl 2-chloro-5-[3,6-dihydro-2,6-dioxo-3-methyl-4-trifluoromethyl-1(2H)-pyrimidinyl]-4-fluorobenzoate,
1,3-dioxan-5-yl 2-chloro-5-[3,6-dihydro-2,6-dioxo-3-methyl-4-trifluoromethyl-1(2H)-pyrimidinyl]-4-fluorobenzoate,
cyclopropylmethyl 2-chloro-5-[3,6-dihydro-2,6-dioxo-3-methyl-4-trifluoromethyl-1(2H)-pyrimidinyl]-4-fluorobenzoate,
tetrahydrofurfuryl 2-chloro-5-[3,6-dihydro-2,6-dioxo-3-methyl-4-trifluoromethyl-1(2H)-pyrimidinyl]-4-fluorobenzoate,
1,3-dioxolan-4-ylmethyl 2-chloro-5-[3,6-dihydro-2,6-dioxo-3-methyl-4-trifluoromethyl-1(2H)-pyrimidinyl]-4-fluorobenzoate,
benzyl 2-chloro-5-[3,6-dihydro-2,6-dioxo-3-methyl-4-trifluoromethyl-1(2H)-pyrimidinyl]-4-fluorobenzoate,
4,4-dimethyl-2-oxo-tetrahydrofuran-3-yl 2-chloro-5-[3,6-dihydro-2,6-dioxo-3-methyl-4-trifluoromethyl-1-(2H)-pyrimidinyl]-4-fluorobenzoate,
cyclopentyl 2-chloro-5-[3,6-dihydro-2,6-dioxo-3-methyl-4-trifluoromethyl-1(2H)-pyrimidinyl]-4-fluorobenzoate,
1-cyclopropylethyl 2-chloro-5-[3,6-dihydro-2,6-dioxo-3-methyl-4-trifluoromethyl-1(2H)-pyrimidinyl]-4-fluorobenzoate and
cyclohexyl 2-chloro-5-[3,6-dihydro-2,6-dioxo-3-methyl-4-trifluoromethyl1(2H)-pyrimidinyl]-4-fluorobenzoate
as well as formulation adjuvants.

**17.** A weed control composition according to claim 15, characterized in that it contains an effective amount of at least one compound selected from the group
isopropyl 2-chloro-5-[3-difluoromethyl-3,6-dihydro-4-methyl-2,6-dioxo-1(2H)-pyrimidinyl]-4-fluorobenzoate,
2-chloro-5-[3-difluoromethyl-3,6-dihydro-4-methyl-2,6-dioxo-1(2H)-pyrimidinyl]-4-fluorobenzoic acid,
isopropyl 5-[4-ethyl-3-difluoromethyl-3,6-dihydro-2,6-dioxo-1(2H)-pyrimidinyl]-2-chloro-4-fluorobenzoate,
isopropyl 5-[4-ethyl-3-difluoromethyl-3,6-dihydro-2,6-dioxo-1(2H)-pyrimidinyl]-2-bromo-4-fluorobenzoate,
isopropyl 2-bromo-5-[3-difluoromethyl-3,6-dihydro-4-methyl-2,6-dioxo-1(2H)-pyrimidinyl]-4-fluorobenzoate,
2-propynyl 2-chloro-5-[3-difluoromethyl-3,6-dihydro-4-methyl-2,6-dioxo-1(2H)-pyrimidinyl]-4-fluorobenzoate and
methyl 2-chloro-5-[3-difluoromethyl-3,6-dihydro-4-methyl-2,6-dioxo-1(2H)-pyrimidinyl]-4-fluorobenzoate
as well as formulation adjuvants.

**18.** A weed control composition according to claim 15, characterized in that it contains an effective amount of at least one compound selected from the group
cyclopropylmethyl 2-chloro-5-[3,6-dihydro-3,4-dimethyl-2,6-dioxo-1(2H)-pyrimidinyl]-4-fluorobenzoate,
1-cyclopropylethyl 2-chloro-5-[3,6-dihydro-3,4-dimethyl-2,6-dioxo-1(2H)-pyrimidinyl]-4-fluorobenzoate,
cyclopentyl 2-chloro-5-[3,6-dihydro-3,4-dimethyl-2,6-dioxo-1(2H)-pyrimidinyl]-4-fluorobenzoate,
cyclohexyl 2-chloro-5-[3,6-dihydro-3,4-dimethyl-2,6-dioxo-1(2H)-pyrimidinyl]-4-fluorobenzoate,

cycloheptyl 2-chloro-5-[3,6-dihydro-3,4-dimethyl-2,6-dioxo-1(2H)-pyrimidinyl]-4-fluorobenzoate,
tetrahydrofuran-3-yl 2-chloro-5-[3,6-dihydro-3,4-dimethyl-2,6-dioxo-1(2H)-pyrimidinyl]-4-fluorobenzoate,
1,3-dioxan-5-yl 2-chloro-5-[3,6-dihydro-3,4-dimethyl-2,6-dioxo-1(2H)-pyrimidinyl]-4-fluorobenzoate,
(1,3-dioxolan-4-yl)methyl 2-chloro-5-[3,6-dihydro-3,4-dimethyl-2,6-dioxo-1(2H)-pyrimidinyl]-4-fluorobenzoate,
benzyl 2-chloro-5-[3,6-dihydro-3,4-dimethyl-2,6-dioxo-1(2H)-pyrimidinyl]-4-fluorobenzoate and
tetrahydrofurfuryl 2-chloro-5-[3,6-dihydro-3,4-dimethyl-2,6-dioxo-1(2H)-pyrimidinyl]-4-fluorobenzoate
as well as formulation adjuvants.

**19.** A weed control composition, characterized in that it contains an effective amount of at least one compound of the general formula

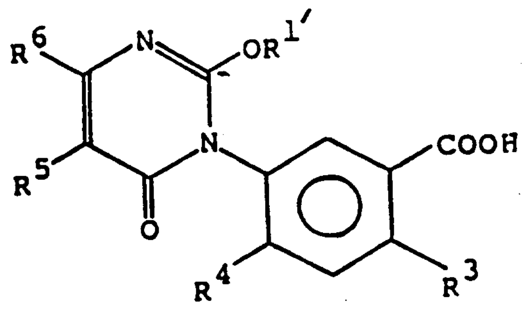

IIIa

in which
$R^{1'}$ signifies $C_{1-4}$alkyl, $C_{3\ or\ 4}$alkenyl or $C_{3\ or\ 4}$alkynyl,
$R^3$ signifies halogen or cyano,
$R^4$ signifies hydrogen or halogen,
$R^5$ signifies hydrogen, fluorine or $C_{1-4}$alkyl and
$R^6$ signifies $C_{1-4}$alkyl or $C_{1-4}$halolkyl or of a salt thereof, as well as formulation adjuvants.

**20.** A process for the preparation of compounds of the general formula

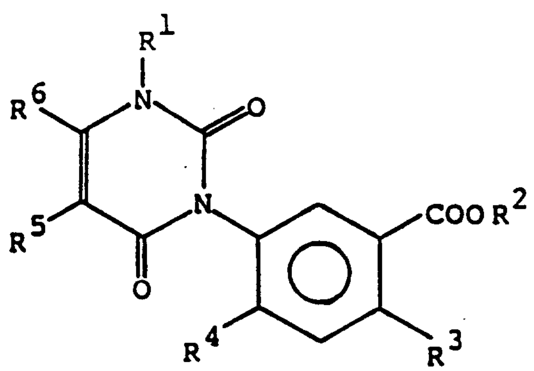

I

in which
$R^1$ signifies hydrogen, $C_{1-4}$alkyl, $C_{3\ or\ 4}$alkenyl, $C_{3\ or\ 4}$alkynyl or $C_{1-4}$haloalkyl,
$R^2$ signifies a group

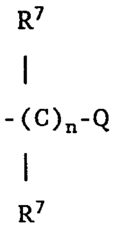

or, where $R^1$ signifies haloalkyl, also hydrogen, $C_{1-8}$alkyl, $C_{2-8}$alkenyl, $C_{2-8}$alkynyl or $c_{2-8}$alkoxyalkyl,
$R^3$ signifies halogen or cyano,
$R^4$ signifies hydrogen or halogen,
$R^5$ signifies hydrogen, fluorine or $C_{1-4}$alkyl,
$R^6$ signifies $C_{1-4}$alkyl or $C_{1-4}$haloalkyl, the symbols $R^7$ each independently of one another signify hydrogen or $C_{1-3}$alkyl and
n signifies 0, 1 or 2,
and
Q signifies a saturated three- to seven-membered carbocyclic or heterocyclic radical which is optionally substituted by one or more $C_{1-4}$alkyl radicals, where the heterocyclic radical has 1 or 2 hetero atoms selected from oxygen and sulphur and optionally a keto function in the ring,
or
Q signifies a phenyl radical which is optionally mono- or poly-substituted by halogen, $C_{1-4}$alkyl, $C_{1-4}$haloalkyl, $C_{1-4}$alkoxy, $C_{1-4}$alkylthio, nitro and/or cyano and which additionally can contain a fused, saturated, carbocyclic or heterocyclic five- to seven-membered ring, where the heterocycle has 1 or 2 oxygen atoms in the ring, and of the corresponding enol ethers of those compounds of formula I in which $R^1$ is different from hydrogen or $C_{1-4}$haloalkyl as well as of salts of those compounds of formula I in which $R^1$ or $R^2$ signifies hydrogen, characterized by

a) for the preparation of those compounds of formula I in which $R^1$ signifies hydrogen as well as, if desired, of metal salts of these compounds, subjecting a compound of the general formula

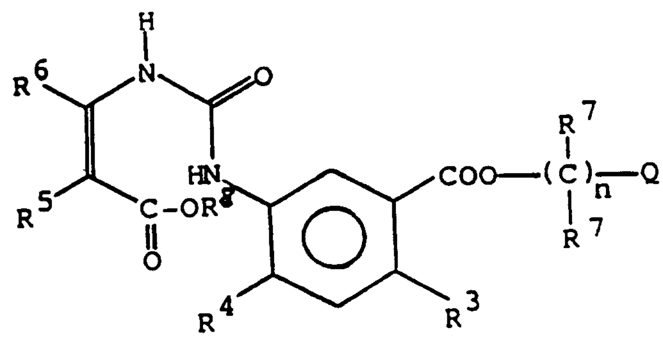

II

in which $R^3$, $R^4$, $R^5$, $R^6$, $R^7$, n and Q have the significances given above and $R^8$ signifies lower alkyl, to a cyclization under basic conditions and, if desired, converting a metal salt of the uracil derivative of formula I which may be obtained into the acid form ($R^1$ = hydrogen) by treatment with an acid,

b) for the preparation of those compounds of formula I in which $R^1$ signifies $C_{1-4}$alkyl, $C_{3 \text{ or } 4}$alkenyl, $C_{3 \text{ or } 4}$alkynyl or $C_{1-4}$haloalkyl, where $R^2$ is different from hydrogen when $R^1$ signifies $C_{1-4}$haloalkyl, subjecting a uracil derivative of the general formula

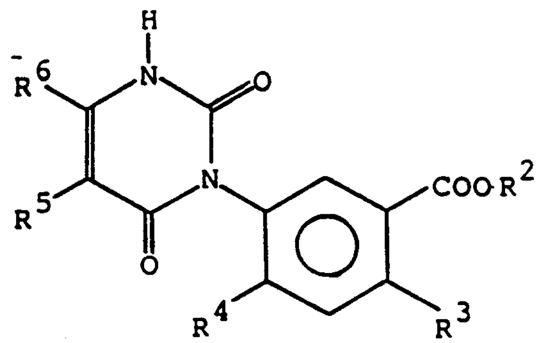

I'

in which $R^2$, $R^3$, $R^4$, $R^5$ and $R^6$ have the significances given above, to an alkylation with a corresponding alkylating agent containing a $C_{1-4}$alkyl, $C_{3\ or\ 4}$alkenyl or $C_{3\ or\ 4}$alkynyl group, with the proviso that in the preparation of a compound of formula I in which $R^1$ signifies $C_{1-4}$haloalkyl $R^2$ in formula I' is different from hydrogen,

c) for the preparation of those compounds of formula I in which $R^1$ is different from hydrogen and of the corresponding enol ethers, esterifying a benzoic acid of the general formula

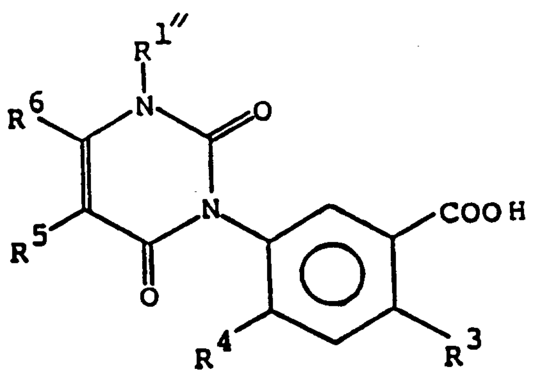

III

in which $R^3$, $R^4$, $R^5$ and $R^6$ have the significances given above and $R^{1''}$ signifies $C_{1-4}$alkyl, $C_{3\ or\ 4}$alkenyl, $C_{3\ or\ 4}$alkynyl or $C_{1-4}$haloalkyl, or the corresponding enol ether, where the benzoic acid or its enol ether can be present in the form of a reactive derivative, with a hydroxy compound of the general formula

$R^2OH$ IV

in which $R^2$ has the significance given above with the proviso that in the preparation of a compound of formula I in which $R^1$ signifies $C_{1-4}$haloalkyl $R^2$ in formula IV is different from hydrogen, or with a reactive derivative of this hydroxy compound,

d) for the preparation of those compounds of formula I in which $R^1$ is different from hydrogen, subjecting a benzoic acid ester of the general formula

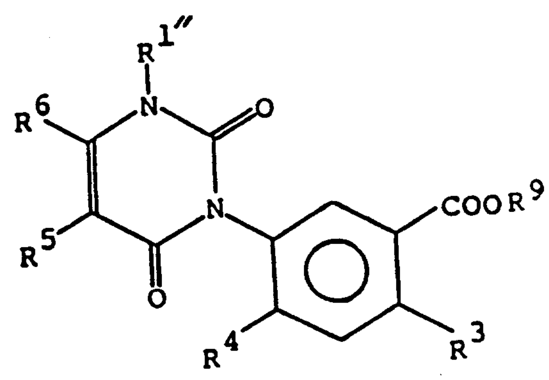

V

in which $R^{1''}$, $R^3$, $R^4$, $R^5$ and $R^6$ have the significances given above and $R^9$ signifies $C_{1-6}$alkyl, $C_{2-4}$alkenyl, $C_{2-4}$alkynyl or $C_{2-6}$alkoxyalkyl, to a trans-esterification reaction with a hydroxy compound of formula IV given above, where the reagent IV has a higher boiling point than the alkanol, alkenol or alkynol $R^9OH$, or

e) for the preparation of those compounds of formula I in which $R^1$ signifies $C_{1-4}$haloalkyl and $R^2$ signifies hydrogen, hydrolyzing a benzoic acid ester of the general formula

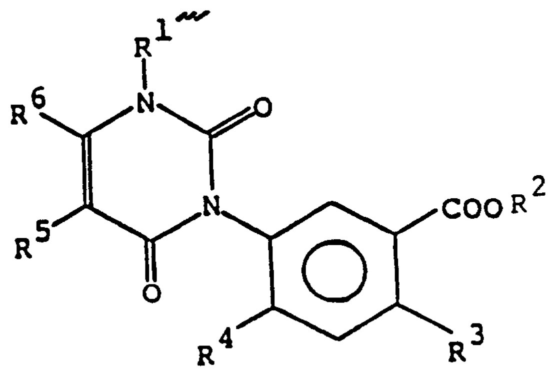

I"

in which $R^3$, $R^4$, $R^5$ and $R^6$ have the significances given above and $R^{1'''}$ signifies $C_{1-4}$haloalkyl and $R^{2'}$ has the significance of $R^2$ with the exception of hydrogen,

to the corresponding benzoic acid,

f) for the preparation of the enol ethers of the compounds of formula I, treating a uracil derivative of the general formula

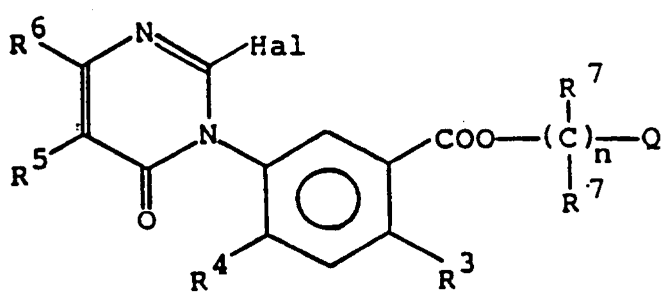

VI

in which $R^3$, $R^4$, $R^5$, $R^6$, $R^7$, n and Q have the significances given above and Hal signifies chlorine or bromine,

with an alkanol, alkenol or alkynol $R^{1'}OH$ in the presence of an organic base or with the corresponding alcoholate, alkenolate or alkynolate of the general formula

$R^{1'}O^{\ominus}M^{\oplus}$ VII

in which $R^{1'}$ has the significance given above and $M^{\oplus}$ signifies an equivalent of a metal ion, and, if desired, converting a thus-obtained compound of formula I in which $R^1$ or $R^2$ signifies hydrogen into a salt.

**21.** A process for the preparation of compounds of the general formula

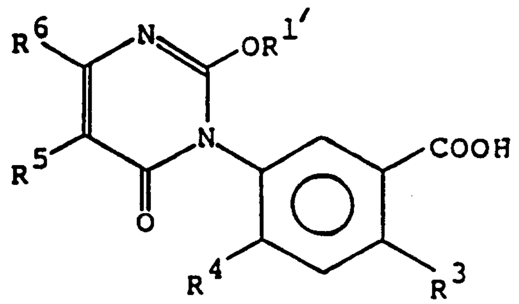

IIIa

in which
$R^{1'}$ signifies $C_{1-4}$alkyl, $C_{3\ or\ 4}$alkenyl or $C_{3\ or\ 4}$alkynyl,
$R^3$ signifies halogen or cyano,
$R^4$ signifies hydrogen or halogen,
$R^5$ signifies hydrogen, fluorine or $C_{1-4}$alkyl and
$R^6$ signifies $C_{1-4}$alkyl or $C_{1-4}$haloalkyl,
and of their salts, characterized by subjecting a compound of the general formula

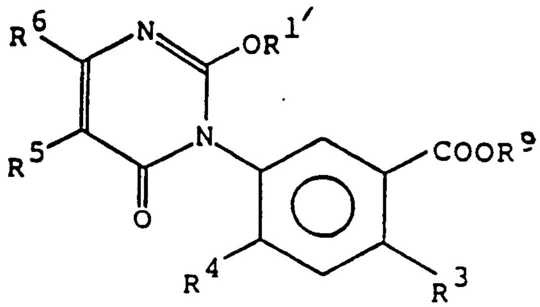

XV

in which $R^{1'}$, $R^3$, $R^4$, $R^5$ and $R^6$ have the significances given above and $R^9$ signifies $C_{1-6}$alkyl, $C_{2-4}$alkenyl, $C_{2-4}$alkynyl or $C_{2-6}$alkoxyalkyl, to a hydrolysis,
and, if desired, converting a thus-obtained compound of formula IIIa into a salt.

**22.** A process according to claim 20 or 21, in which $R^6$ signifies in each case $C_{1-4}$alkyl, especially methyl.

**23.** A method for the control of weeds, characterized by treating the locus to be protected against weeds and/or the weeds with an effective amount of a compound according to any one of claims 1 to 14 or of a composition according to any one of claims 15 to 19.

**24.** Use of a compound according to any one of claims 1 to 14 or of a composition according to any one of claims 15 to 19 for the control of weeds.

## Revendications

**1.** composés de formule générale

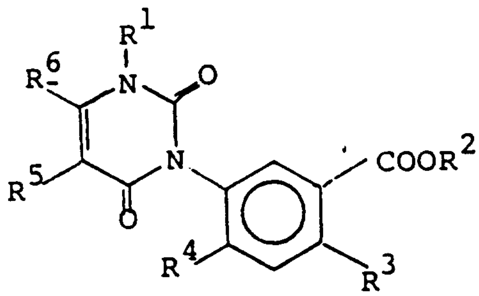

I

dans laquelle

$R^1$ représente l'hydrogène, un groupe alkyle en C1-C4, alcényle en C3 ou C4, alcynyle en C3 ou C4 ou halogénoalkyle en C1-C4,

$R^2$ représente un groupe

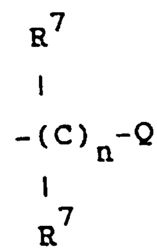

ou bien encore, dans le cas où $R^1$ représente un groupe halogénoalkyle, l'hydrogène, un groupe alkyle en C1-C8, alcényle en C2-C8, alcynyle en C2-C8 ou alcoxyalkyle en C2-C8,

$R^3$ représente un halogène ou un groupe cyano,

$R^4$ représente l'hydrogène ou un halogène,

$R^5$ représente l'hydrogène, le fluor ou un groupe alkyle en C1-C4,

$R^6$ représente un groupe alkyle on C1-C4 ou halogénoalkyle en C1-C4,

les symboles $R^7$ représentent chacun, indépendamment l'un de l'autre, l'hydrogène ou un groupe alkyle en C1-C3 et

n est égal à 0, 1 ou 2, et

Q représente un radical carbocyclique ou hétérocyclique saturé de 3 à 7 chaînons éventuellement substitué par un ou plusieurs groupes alkyle en C1-C4, le radical hétérocyclique contenant un ou deux hétéroatomes choisis parmi l'oxygène et le soufre et le cas échéant une fonction céto dans le cycle,

ou bien

Q représente un groupe phényle portant éventuellement un ou plusieurs substituants choisis parmi les halogènes, les groupes alkyle en C1-C4, halogénoalkyle en C1-C4, alcoxy en C1-C4, alkylthio en C1-C4, nitro et/ou cyano et qui peut en outre être condensé avec un noyau carbocyclique ou hétérocyclique saturé de 5 à 7 chaînons, l'hétérocycle contenant un ou deux atomes d'oxygène dans le cycle,

et les éthers d'énols correspondant aux composés de formule I dans laquelle $R^1$ a une signification autre que l'hydrogène ou un groupe halogénoalkyle en C1-C4, et les sels des composés de formule I dans laquelle $R^1$ ou $R^2$ représente l'hydrogène.

**2.** Composés selon revendication 1, pour lesquels $R^1$ représente l'hydrogène, un groupe alkyle en C1-C4, alcényle en C3 ou C4 ou alcynyle en C3 ou C4 et leurs éthers d'énols et sels.

**3.** Composés selon revendication 1, pour lesquels $R^1$ représente un groupe halogénoalkyle en C1-C4, $R^2$ représente l'hydrogène, un groupe alkyle en C1-C8, alcényle en C2-C8, alcynyle en C2-C8, alcoxyalkyle en C2-C8 ou un groupe -$(CR^7R^7)_n$-Q et Q représente un radical carbocyclique ou hétérocyclique saturé de 5 à 7 chaînons éventuellement substitué par un ou plusieurs groupes alkyle en C1-C4, le radical hétérocyclique contenant dans le cycle un ou deux hétéroatomes choisis parmi l'oxygène ou le soufre, ou bien un groupe phényle portant éventuellement un ou plusieurs substituants choisis parmi les halogènes, les groupes alkyle en C1-C4, halogénoalkyle en C1-C4, alcoxy en C1-C4, alkylthio en C1-C4, nitro et/ou cyano, et les sels de ces composes.

**4.** Composés selon revendication 2, pour lesquels $R^1$ représente un groupe méthyle.

**5.** Composés selon revendication 3, pour lesquels $R^1$ représente un groupe difluorométhyle.

**6.** Composés selon une des revendications 1 à 5, pour lesquels $R^3$ représente le chlore ou le brome et $R^4$ le fluor.

**7.** Composés selon une des revendications 1 à 6, pour lesquels $R^3$ représente le chlore ou le brome et $R^4$ l'hydrogène.

**8.** Composés selon une des revendications 1 à 7, pour lesquels $R^5$ représente l'hydrogène, le fluor ou un groupe méthyle.

**9.** Composés selon une des revendications 1 à 8, pour lesquels $R^6$ représente un groupe alkyle en C1-C4.

**10.** Composés selon une des revendications 1 à 9, pour lesquels $R^2$ représente un groupe -$(CR^7R^7)_n$-Q dans lequel chacun des symboles $R^7$ représente l'hydrogène et n est égal à 0 ou 1.

**11.** Un composé selon revendication 2, choisi parmi les suivants :
2-chloro-5-[3,6-dihydro-2,6-dioxo-3-méthyl-4-trifluorométhyl-1(2H)-pyrimidinyl]-4-fluorobenzoate de cycloheptyle,
2-chloro-5-[3,6-dihydro-2,6-dioxo-3-méthyl-4-trifluorométhyl-1(2H)-pyrimidinyl]-4-fluorobenzoate de tétrahydrofuranne-3-yle,
2-chloro-5-[3,6-dihydro-2,6-dioxo-3-méthyl-4-trifluorométhyl-1(2H)-pyrimidinyl]-4-fluorobenzoate de 1,3-dioxanne-5-yle,
2-chloro-5-[3,6-dihydro-2,6-dioxo-3-méthyl-4-trifluorométhyl-1(2H)-pyrimidinyl]-4-fluorobenzoate de cyclopropylméthyle,
2-chloro-5-[3,6-dihydro-2,6-dioxo-3-méthyl-4-trifluorométhyl-1(2H)-pyrimidinyl]-4-fluorobenzoate de tétrahydrofurfuryle,
2-chloro-5-[3,6-dihydro-2,6-dioxo-3-méthyl-4-trifluorométhyl-1(2H)-pyrimidinyl]-4-fluorobenzoate de 1,3-dioxolanne-4-ylméthyle,
2-chloro-5-[3,6-dihydro-2,6-dioxo-3-méthyl-4-trifluorométhyl-1(2H)-pyrimidinyl]-4-fluorobenzoate de benzyle,
2-chloro-5-[3,6-dihydro-2,6-dioxo-3-méthyl-4-trifluorométhyl-1(2H)-pyrimidinyl]-4-fluorobenzoate de 4,4-diméthyl-3-oxo-tétrahydrofuranne-3-yle,
2-chloro-5-[3,6-dihydro-2,6-dioxo-3-méthyl-4-trifluorométhyl-1(2H)-pyrimidinyl]-4-fluorobenzoate de cyclopentyle,
2-chloro-5-[3,6-dihydro-2,6-dioxo-3-méthyl-4-trifluorométhyl-1(2H)-pyrimidinyl]-4-fluorobenzoate de 1-cyclopropyléthyle et
2-chloro-5-[3,6-dihydro-2,6-dioxo-3-méthyl-4-trifluorométhyl-1(2H)-pyrimidinyl]-4-fluorobenzoate de cyclohexyle.

**12.** Un composé selon revendication 3, choisi parmi les suivants :
2-chloro-5-[3-difluorométhyl-3,6-dihydro-4-méthyl-2,6-dioxo-1(2H)-pyrimidinyl]-4-fluorobenzoate d'isopropyle,
acide 2-chloro-5-[3-difluorométhyl-3,6-dihydro-4-méthyl-2,6-dioxo-1(2H)-pyrimidinyl]-4-fluorobenzoïque,
5-[4-éthyl-3-difluorométhyl-3,6-dihydro-2,6-dioxo-1(2H)-pyrimidinyl]-2-chloro-4-fluorobenzoate d'isopropyle,
5-[4-éthyl-3-difluorométhyl-3,6-dihydro-2,6-dioxo-1(2H)-pyrimidinyl]-2-bromo-4-fluorobenzoate d'isopropyle,

2-bromo-5-[3-difluorométhyl-3,6-dihydro-4-méthyl-2,6-dioxo-1(2H)-pyrimidinyl]-4-fluorobenzoate d'isopropyle,

2-chloro-5-[3-difluorométhyl-3,6-dihydro-4-méthyl-2,6-dioxo-1(2H)-pyrimidinyl]-4-fluorobenzoate de 2-propynyle et

2-chloro-5-[3-difluorométhyl-3,6-dihydro-4-méthyl-2,6-dioxo-1(2H)-pyrimidinyl]-4-fluorobenzoate de méthyle.

**13.** Un composé selon revendication 1, choisi parmi les suivants :

2-chloro-5-[3,6-dihydro-3,4-diméthyl-2,6-dioxo-1(2H)-pyrimidinyl]-4-fluorobenzoate de cyclopropylméthyle,

2-chloro-5-[3,6-dihydro-3,4-diméthyl-2,6-dioxo-1(2H)-pyrimidinyl]-4-fluorobenzoate de 1-cyclopropyléthyle,

2-chloro-5-[3,6-dihydro-3,4-diméthyl-2,6-dioxo-1(2H)-pyrimidinyl]-4-fluorobenzoate de cyclopentyle,

2-chloro-5-[3,6-dihydro-3,4-diméthyl-2,6-dioxo-1(2H)-pyrimidinyl]-4-fluorobenzoate de cyclohexyle,

2-chloro-5-[3,6-dihydro-3,4-diméthyl-2,6-dioxo-1(2H)-pyrimidinyl]-4-fluorobenzoate de cycloheptyle,

2-chloro-5-[3,6-dihydro-3,4-diméthyl-2,6-dioxo-1(2H)-pyrimidinyl]-4-fluorobenzoate de tétrahydrofuranne-3-yle,

2-chloro-5-[3,6-dihydro-3,4-diméthyl-2,6-dioxo-1(2H)-pyrimidinyl]-4-fluorobenzoate de 1,3-dioxanne-5-yle,

2-chloro-5-[3,6-dihydro-3,4-diméthyl-2,6-dioxo-1(2H)-pyrimidinyl]-4-fluorobenzoate de(1,3-dioxolanne-4-yle)méthyle,

2-chloro-5-[3,6-dihydro-3,4-diméthyl-2,6-dioxo-1(2H)-pyrimidinyl]-4-fluorobenzoate de benzyle et

2-chloro-5-[3,6-dihydro-3,4-diméthyl-2,6-dioxo-1(2H)-pyrimidinyl]-4-fluorobenzoate de tétrahydrofurfuryle.

**14.** Composés de formule générale

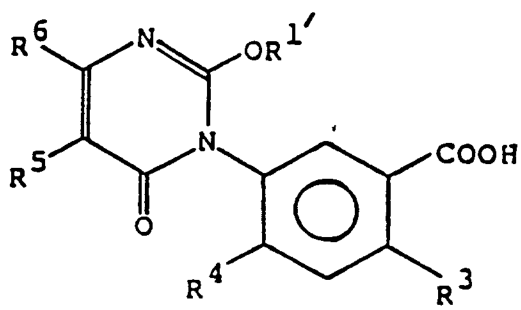

IIIa

dans laquelle

$R^{1'}$ représente un groupe alkyle en C1-C4, alcényle en C3 ou C4 ou alcynyle en C3 ou C4,

$R^3$ représente un halogène ou un groupe cyano,

$R^4$ représente l'hydrogène ou un halogène,

$R^5$ représente l'hydrogène, le fluor ou un groupe alkyle en C1-C4 et

$R^6$ représente un groupe alkyle en C1-C4 ou halogénoalkyle en C1-C4,

et leurs sels.

**15.** Produit herbicide, caractérisé en ce qu'il contient une quantité efficace d'au moins un composé de formule générale

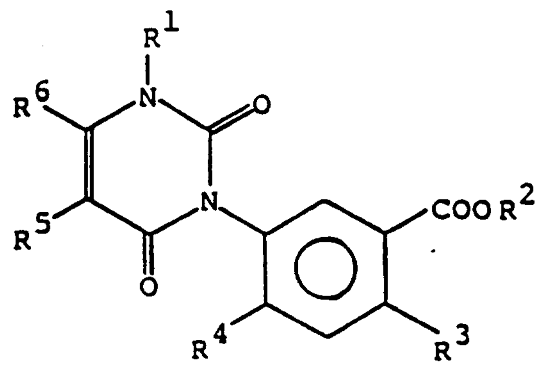

I

dans laquelle

$R^1$ représente l'hydrogène, un groupe alkyle en C1-C4, alcényle en C3 ou C4, alcynyle en C3 ou C4 ou halogénoalkyle en C1-C4,

$R^2$ représente un groupe

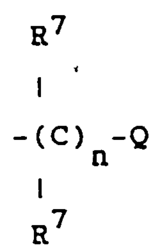

ou bien encore, dans le cas où $R^1$ représente un groupe halogénoalkyle, l'hydrogène, un groupe alkyle en C1-C8, alcényle en C2-C8, alcynyle en C2-C8 ou alcoxyalkyle en C2-C8,

$R^3$ représente un halogène ou un groupe cyano,

$R^4$ représente l'hydrogène ou un halogène,

$R^5$ représente l'hydrogène, le fluor ou un groupe alkyle en C1-C4,

$R^6$ représente un groupe alkyle en C1-C4 ou halogénoalkyle en C1-C4,

les symboles $R^7$ représentent chacun, indépendamment l'un de l'autre, l'hydrogène ou un groupe alkyle en C1-C3 et

n est égal à 0, 1 ou 2,

et

Q représente un radical carbocyclique ou hétérocyclique saturé de 3 à 7 chaînons portant éventuellement un ou plusieurs substituants alkyle en C1-C4, le radical hétérocyclique contenant un ou deux atomes hétéroatomes choisis parmi l'oxygène et le soufre et le cas échéant une fonction céto dans le cycle,

ou bien

Q représente un groupe phényle portant éventuellement un ou plusieurs substituants choisis parmi les halogènes, les groupes alkyle en C1-C4, halogénoalkyle en C1-C4, alcoxy en C1-C4, alkylthio en C1-C4, nitro et/ou cyano et qui peut en outre être condensé avec un noyau carbocyclique ou hétérocyclique saturé de 5 à 7 chaînons, le noyau hétérocyclique contenant un ou deux atomes d'oxygène dans le cycle,

ou bien d'un éther d'énol d'un tel composé I pour lequel $R^1$ a une signification autre que l'hydrogène ou un groupe halogénoalkyle en C1-C4, ou bien d'un sel d'un tel composé I pour lequel $R^1$ ou $R^2$ représente l'hydrogène, avec des produits auxiliaires de formulation.

**16.** Produit herbicide selon revendication 15, caractérisé en ce qu'il contient une quantité efficace d'au moins un composé du groupe suivant :

2-chloro-5-[3,6-dihydro-2,6-dioxo-3-méthyl-4-trifluorométhyl-1(2H)-pyrimidinyl]-4-fluorobenzoate de cycloheptyle,

2-chloro-5-[3,6-dihydro-2,6-dioxo-3-méthyl-4-trifluorométhyl-1(2H)-pyrimidinyl]-4-fluorobenzoate de

tétrahydrofuranne-3-yle,
2-chloro-5-[3,6-dihydro-2,6-dioxo-3-méthyl-4-trifluorométhyl-1(2H)-pyrimidinyl]-4-fluorobenzoate de 1,3-dioxanne-5-yle,
2-chloro-5-[3,6-dihydro-2,6-dioxo-3-méthyl-4-trifluorométhyl-1(2H)-pyrimidinyl]-4-fluorobenzoate de cyclopropylméthyle,
2-chloro-5-[3,6-dihydro-2,6-dioxo-3-méthyl-4-trifiuorométhyl-1(2H)-pyrimidinyl]-4-fluorobenzoate de tétrahydrofurfuryle,
2-chloro-5-[3,6-dihydro-2,6-dioxo-3-méthyl-4-trifluorométhyl-1(2H)-pyrimidinyl]-4-fluorobenzoate de 1,3-dioxolanne-4-ylméthyle,
2-chloro-5-[3,6-dihydro-2,6-dioxo-3-méthyl-4-trifluorométhyl-1(2H)-pyrimidinyl]-4-fluorobenzoate de benzyle,
2-chloro-5-[3,6-dihydro-2,6-dioxo-3-méthyl-4-trifluorométhyl-1(2H)-pyrimidinyl]-4-fluorobenzoate de 4,4-diméthyl-2-oxo-tétrahydrofuranne-3-yle,
2-chloro-5-[3,6-dihydro-2,6-dioxo-3-méthyl-4-trifluorométhyl-1(2H)-pyrimidinyl]-4-fluorobenzoate de cyclopentyle,
2-chloro-5-[3,6-dihydro-2,6-dioxo-3-méthyl-4-trifluorométhyl-1(2H)-pyrimidinyl]-4-fluorobenzoate de 1-cyclopropyléthyle et
2-chloro-5-[3,6-dihydro-2,6-dioxo-3-méthyl-4-trifluorométhyl-1(2H)-pyrimidinyl]-4-fluorobenzoate de cyclohexyle,
avec des produits auxiliaires de formulation.

**17.** Produit herbicide selon revendication 15, caractérisé en ce qu'il contient une quantité efficace d'au moins un composé du groupe suivant :
2-chloro-5-[3-difluorométhyl-3,6-dihydro-4-méthyl-2,6-dioxo-1(2H)-pyrimidinyl]-4-fluorobenzoate d'isopropyle,
2-chloro-5-[3-difluorométhy1-3,6-dihydro-4-méthyl-2,6-dioxo-1(2H)-pyrimidinyl]-4-fluorobenzoate,
5-[4-éthyl-3-difluorométhyl-3,6-dihydro-2,6-dioxo-1(2H)-pyrimidinyl]-2-chloro-4-fluorobenzoate d'isopropyle,
5-[4-éthyl-3-difluorométhyl-3,6-dihydro-2,6-dioxo-1(2H)-pyrimidinyl]-2-bromo-4-fluorobenzoate d'isopropyle,
2-bromo-5-[3-difluorométhyl-3,6-dihydro-4-méthyl-2,6-dioxo-1(2H)-pyrimidinyl]-4-fluorobenzoate d'isopropyle,
2-chloro-5-[3-difluorométhyl-3,6-dihydro-4-méthyl-2,6-dioxo-1(2H)-pyrimidinyl]-4-fluorobenzoate de 2-propinyle et
2-chloro-5-[3-difluorométhyl-3,6-dihydro-4-méthy1-2,6-dioxo-1(2H)-pyrimidinyl]-4-fluorobenzoate de méthyle,
avec des produits auxiliaires de formulation.

**18.** Produit herbicide selon revendication 15, caractérisé en ce qu'il contient une quantité efficace d'au moins un composé du groupe suivant :
2-chloro-5-[3,6-dihydro-3,4-diméthyl-2,6-dioxo-1(2H)-pyrimidinyl]-4-fluorobenzoate de cyclopropylméthyle,
2-chloro-5-[3,6-dihydro-3,4-diméthyl-2,6-dioxo-1(2H)-pyrimidinyl]-4-fluorobenzoate de 1-cyclopropyléthyle,
2-chloro-5-[3,6-dihydro-3,4-diméthyl-2,6-dioxo-1(2H)-pyrimidinyl]-4-fluorobenzoate de cyclopentyle,
2-chloro-5-[3,6-dihydro-3,4-diméthyl-2,6-dioxo-1(2H)-pyrimidinyl]-4-fluorobenzoate de cyclohexyle,
2-chloro-5-[3,6-dihydro-3,4-diméthyl-2,6-dioxo-1(2H)-pyrimidinyl]-4-fluorobenzoate de cycloheptyle,
2-chloro-5-[3,6-dihydro-3,4-diméthyl-2,6-dioxo-1(2H)-pyrimidinyl]-4-fluorobenzoate de tétrahydrofuranne-3-yle,
2-chloro-5-[3,6-dihydro-3,4-diméthyl-2,6-dioxo-1(2H)-pyrimidinyl]-4-fluorobenzoate de 1,3-dioxanne-5-yle,
2-chloro-5-[3,6-dihydro-3,4-diméthyl-2,6-dioxo-1(2H)-pyrimidinyl]-4-fluorobenzoate de (1,3-dioxolanne-4-yle)méthyle,
2-chloro-5-[3,6-dihydro-3,4-diméthyl-2,6-dioxo-1(2H)-pyrimidinyl]-4-fluorobenzoate de benzyle et
2-chloro-5-[3,6-dihydro-3,4-diméthyl-2,6-dioxo-1(2H)-pyrimidinyl]-4-fluorobenzoate de tétrahydrofurfuryle,
avec des produits auxiliaires de formulation.

**19.** Produit herbicide caractérisé en ce qu'il contient une quantité efficace d'au moins un composé de formule générale

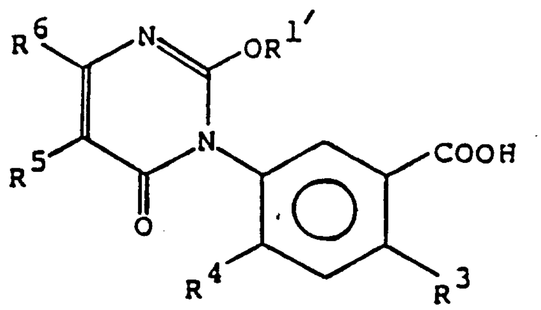

IIIa

dans laquelle

$R^{1'}$ représente un groupe alkyle en C1-C4, alcényle en C3 ou C4 ou alcynyle en C3 ou C4,

$R^3$ représente un halogène ou un groupe cyano,

$R^4$ représente l'hydrogène ou un halogène,

$R^5$ représente l'hydrogène, le fluor ou un groupe alkyle en C1-C4 et

$R^6$ représente un groupe alkyle en C1-C4 ou halogénoalkyle en C1-C4,

ou d'un sel d'un tel composé, avec des produits auxiliaires de formulation.

**20.** Procédé de préparation des composés de formule générale

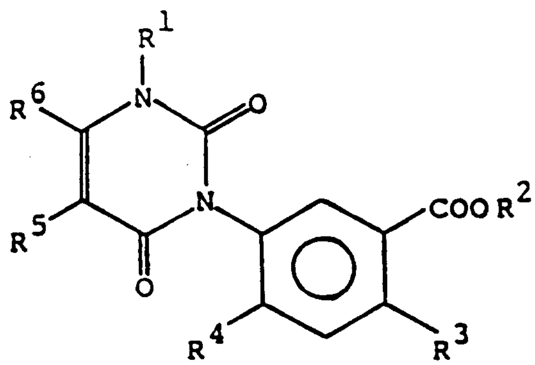

I

dans laquelle

$R^1$ représente l'hydrogène, un groupe alkyle en C1-C4, alcényle en C3 ou C4, alcynyle en C3 ou C4 ou halogénoalkyle en C1-C4,

$R^2$ représente un groupe

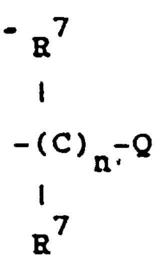

ou bien encore, dans le cas où $R^1$ représente un groupe halogénoalkyle, l'hydrogène, un groupe alkyle en C1-C8, alcényle en C2-C8, alcynyle en C2-C8 ou alcoxyalkyle en C2-C8,

$R^3$ représente un halogène ou un groupe cyano,

$R^4$ représente l'hydrogène ou un halogène,

$R^5$ représente l'hydrogène, le fluor ou un groupe alkyle en C1-C4,

$R^6$ représente un groupe alkyle en C1-C4 ou halogénoalkyle en C1-C4,

les symboles $R^7$ représentent chacun , indépendamment l'un de l'autre, l'hydrogène ou un groupe alkyle en C1-C3 et

n est égal à 0, 1 ou 2,

et

Q représente un radical carbocyclique ou hétérocyclique saturé de 3 à 7 chaînons, portant éventuellement un ou plusieurs substituants alkyle en C1-C4, le radical hétérocyclique contenant un ou deux hétéroatomes choisis parmi l'oxygène et le soufre et le cas échéant une fonction céto dans le cycle,

ou bien

Q représente un groupe phényle portant éventuellement un ou plusieurs substituants choisis parmi les halogènes, les groupes alkyle en C1-C4, halogénoalkyle en C1-C4, alcoxy en C1-C4, alkylthio en C1-C4, nitro et/ou cyano et qui peut en outre être condensé avec un noyau carbocyclique ou hétérocyclique saturé de 5 à 7 chaînons, le noyau hétérocyclique contenant un ou deux atomes d'oxygène dans le cycle,

et des éthers d'énols correspondant aux composés de formule I dans laquelle $R^1$ a une signification autre que l'hydrogène ou un groupe halogénoalkyle en C1-C4, et des sels des composés de formule I dans laquelle $R^1$ ou $R^2$ représente l'hydrogène, caractérisé en ce que

a) pour préparer les composés de formule I dans laquelle $R^1$ représente l'hydrogène et, si on le désire, les sels métalliques de ces composés, on soumet un composé de formule générale

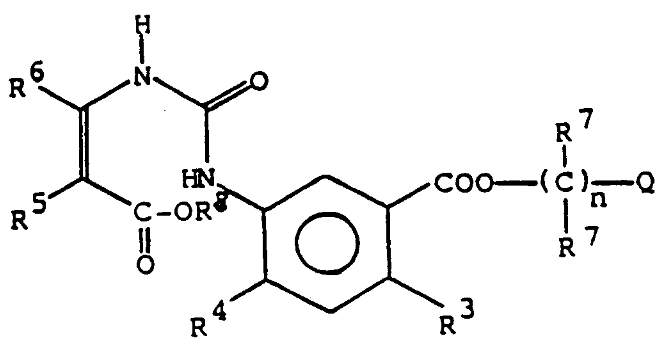

II

dans laquelle $R^3$, $R^4$, $R^5$, $R^6$, $R^7$, n et Q ont les significations indiquées ci-dessus et $R^8$ représente un groupe alkyle inférieur,

à une cyclisation en milieu basique et, si on le désire, on convertit un sel métallique du dérivé d'uracile de formule I ainsi obtenu en la forme acide ($R^1$ = hydrogène) par traitement à l'aide d'un acide,

b) pour préparer les composés de formule I dans laquelle $R^1$ représente un groupe alkyle en C1-C4, alcényle en C3 ou C4, alcynyle en C3 ou C4 ou halogénoalkyle en C1-C4, sous réserve que, lorsque $R^1$ représente un groupe halogénoalkyle en C1-C4, $R^2$ a une signification autre que l'hydrogène, on soumet un dérivé d'uracile de formule générale

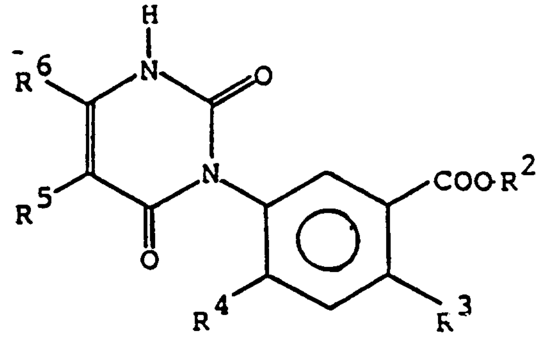

I′

dans laquelle $R^2$, $R^3$, $R^4$, $R^5$ et $R^6$ ont les significations indiquées ci-dessus,
à une alkylation à l'aide d'un agent alkylant approprié, contenant un groupe alkyle en C1-C4, alcényle en C3 ou C4 ou alcynyle en C3 ou C4, sous réserve que dans le cas où l'on prépare un composé de formule I dans laquelle $R^1$ représente un groupe halogénoalkyle en C1-C4, le symbole $R^2$ de la formule I' a une signification autre que l'hydrogène,
c) pour préparer les composés de formule I dans laquelle $R^1$ a une signification autre que l'hydrogène, et les éthers d'énols correspondants, on estérifie un acide benzoïque de formule générale

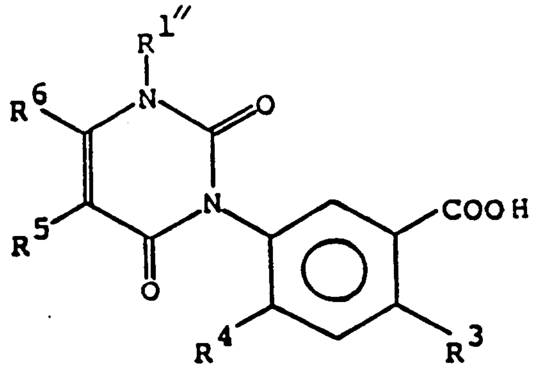

III

dans laquelle $R^3$, $R^4$, $R^5$ et $R^6$ ont les significations indiquées ci-dessus et $R^{1''}$ représente un groupe alkyle en C1-C4, alcényle en C3 ou C4, alcynyle en C3 ou C4 ou halogénoalkyle en C1-C4,
ou l'éther d'énol correspondant, l'acide benzoïque ou l'éther d'énol pouvant être à l'état de dérivé réactif, à l'aide d'un dérivé hydroxylé de formule générale

$R^2OH$ IV

dans laquelle $R^2$ a les significations indiquées ci-dessus, sous réserve que dans le cas où l'on prépare un composé de formule I dans laquelle $R^1$ représente un groupe halogénoalkyle en C1-C4, le symbole $R^2$ de la formule IV doit avoir une signification autre que l'hydrogène, ou à l'aide d'un dérivé réactif de ce dérivé hydroxylé,
d) pour préparer les composés de formule I dans laquelle $R^1$ a une signification autre que l'hydrogène, on soumet un ester benzoïque de formule générale

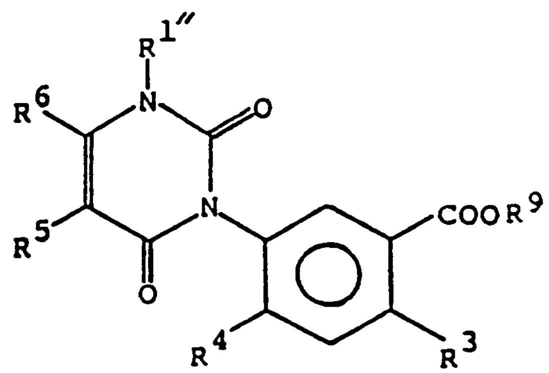

V

dans laquelle
$R^{1''}$, $R^3$, $R^4$, $R^5$ et $R^6$ ont les significations indiquées ci-dessus et $R^9$ représente un groupe alkyle en C1-C6, alcényle en C2-C4, alcynyle en C2-C4 ou alcoxyalkyle en C2-C6,
à une réaction de transestérification avec un dérivé hydroxylé de formule IV ci-dessus, lequel doit bouillir plus haut que l'alcanol, l'alcénol ou l'alcynol $R^9OH$,
e) pour préparer les composés de formule I dans laquelle $R^1$ représente un groupe halogénoalkyle en C1-C4 et $R^2$ l'hydrogène, on hydrolyse un ester benzoïque de formule générale

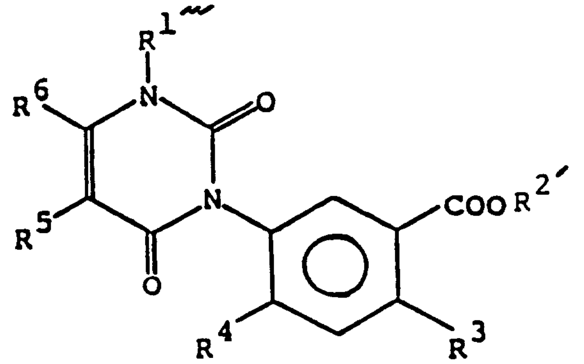

I'''

dans laquelle $R^3$, $R^4$, $R^5$ et $R^6$ ont les significations indiquées ci-dessus et $R^{1'''}$ représente un groupe halogénoalkyle en C1-C4, et $R^{2'}$ a les significations de $R^2$ à l'exception de l'hydrogène,
en l'acide benzoïque correspondant,
f) pour préparer les éthers d'énols des composés de formule I, on traite un dérivé d'uracile de formule générale

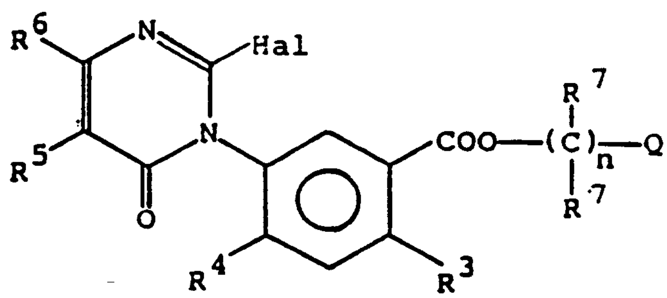

VI

dans laquelle $R^3$, $R^4$, $R^5$, $R^6$, $R^7$, n et Q ont les significations indiquées ci-dessus, et Hal représente le chlore ou le brome,
par un alcanol, un alcénol ou un alcynol $R^{1'}OH$ en présence d'une base organique ou à l'aide d'un alcoolate, alcénolate ou alcynolate correspondant de formule générale

$R1'\,O^{\ominus}M^{\oplus}$ VII

dans laquelle $R^{1'}$ a les significations indiquées ci-dessus et $M^{\oplus}$ représente un équivalent d'un ton métallique, et si on le désire, on convertit un composé ainsi obtenu, répondant à la formule I dans laquelle $R^1$ ou $R^2$ représente l'hydrogène, en un sel.

**21.** Procédé de préparation des composés de formule générale

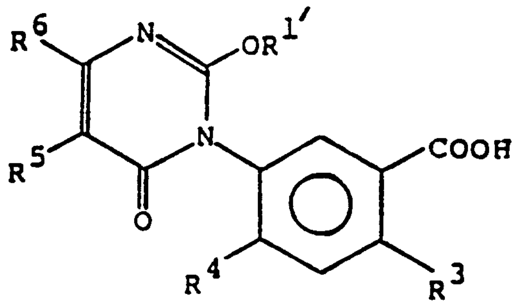

IIIa

dans laquelle

$R^{1'}$ représente un groupe alkyle en C1-C4, alcényle en C3 ou C4 ou alcynyle en C3 ou C4,
$R^3$ représente un halogène ou un groupe cyano,
$R^4$ représente l'hydrogène ou un halogène,
$R^5$ représente l'hydrogène, le fluor ou un groupe alkyle en C1-C4 et
$R^6$ représente un groupe alkyle en C1-C4 ou halogénoalkyle en C1-C4,

et de leurs sels, caractérisé en ce que l'on soumet à une hydrolyse un composé de formule générale

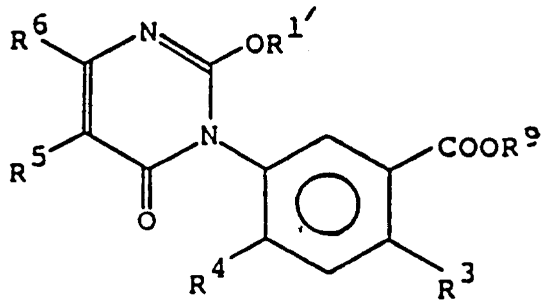

XV

dans laquelle $R^{1'}$, $R^3$, $R^4$, $R^5$ et $R^6$ ont les significations indiquées ci-dessus et $R^9$ représente un groupe alkyle en C1-C6, alcényle en C2-C4, alcynyle en C2-C4 ou alcoxyalkyle en C2-C6,
et si on le désire, on convertit un composé ainsi obtenu, répondant à la formule IIIa, en un sel.

**22.** Procédé selon la revendication 20 ou 21, dans lequel $R^6$ représente un groupe alkyle en C1-C4, plus spécialement un groupe méthyle.

**23.** Procédé pour combattre les végétaux adventices, caractérisé en ce que l'on traite l'endroit à protéger contre les végétaux adventices et/ou les végétaux adventices eux-mêmes par une quantité efficace d'un composé selon une des revendications 1 à 14 ou d'un produit selon une des revendications 15 à 19.

**24.** Utilisation d'un composé selon une des revendications 1 à 14 ou d'un produit selon une des revendications 15 à 19 pour la lutte contre les végétaux adventices.